# EUROPEAN PATENT SPECIFICATION

(11) **EP 4 025 556 B1**
(45) Date of publication and mention of the grant of the patent: **26.08.2026**
(21) Application number: 21824268.3
(22) Date of filing: 27.05.2021
(51) Int. Cl.: C07C 229/12, C07C 229/16, C07D 249/04, C07D 295/088, A61K 9/51, A61K 47/28, A61P 9/00, C07C 237/10

(54) **LIPID COMPOUND AND THE COMPOSITION THEREOF**
LIPIDVERBINDUNG UND IHRE ZUSAMMENSETZUNG
COMPOSÉ LIPIDIQUE ET SA COMPOSITION

(30) Priority: 27.11.2020 CN 202011355845; 31.03.2021 WO PCT/IB2021/052705
(43) Date of publication of application: 13.07.2022
(73) Proprietor: Guangzhou Ribobio Co., Ltd, Huangpu District Guangzhou, Guangdong 510530 (CN)
(72) Inventor: ZHANG, Bill Biliang, Guangzhou, Guangdong 510530 (CN); ZHAO, Haoting, Guangzhou, Guangdong 510530 (CN); WEN, Jian, Guangzhou, Guangdong 510530 (CN)
(74) Representative: Maiwald GmbH
(86) International application number: PCT/IB2021/054662
(87) International publication number: WO 2022/112855

(56) References cited:
- WO-A1-2010/144740
- WO-A1-2013/016058
- WO-A1-2013/016058
- WO-A1-2016/176330
- WO-A1-2016/176330
- WO-A1-2017/075531
- WO-A1-2018/232357
- WO-A1-2021/155274
- WO-A2-2017/049245
- WO-A2-2017/049245
- WO-A2-2017/201340
- CN-A- 106 795 096
- CN-A- 108 368 028
- CN-A- 109 640 962
- US-A1- 2005 208 015
- US-A1- 2015 376 115

## Description

### Technical field

The invention belongs to the field of biomedicine and biotechnology, and relates to a series of lipid compounds and therapeutic pharmaceutical delivery systems thereof.

### Background

Exogenous biomolecules and some pharmaceutical molecules are hard to reach the cytoplasm though the cell membrane for curing effect. mRNA is one kind of biomolecule with negative charge, which has to overcome the barrier of cell membrane, for translating to protein and playing the biological function. Thus, delivering the biomolecules efficiently in vivo is an important challenge.

Lipid Nanoparticle (LNP) is one kind of new nucleic molecule delivery technology, which typically includes four components: (1) ionizable lipid, which combines with mRNA into a particle as large as bacteria, and releases mRNA from endosome to cytoplasm; (2) PEG-lipid, which improves the half-life of LNPs in blood; (3) cholesterol, which improves the stability of nanoparticles; (4), phospholipid, which is beneficial to form the double lipid structure (lipid bilayer). LNPs function to not only protect the mRNA from being decomposed by RNA enzyme (RNAses) or recognized by TLRs, but to also avoid the over-reaction of the innate immune system. The ionizable lipid can accelerate the cell uptake, and help the pharmaceutical molecules to release from endosome, achieving therapeutic effect.

The first LNP-siRNA medicine encapsulated by MC3 cationic lipid has been approved for marketing, proving that LNP can deliver the nucleic acid pharmaceuticals effectively in vivo, with an acceptable safety profile to some extent. In recent years, the study found that LNP also showed great application potential in the field of mRNA pharmaceutical and vaccine. The development direction of LNP delivery system is mainly focused on the ionizable lipid, the formula thereof and how to overcome the toxicity of some lipid preparations.

PCT/US2016/052352, published as WO2017/049245, discloses compounds and compositions and their use for intracellular delivery of therapeutic agents, including several novel lipid structures which can deliver the mRNA molecule to the target cell. PCT/US2010/038224, published as WO2010/144740 discloses the chemical structure of MC3 which can encapsulate the siRNA pharmaceuticals with high efficiency, and avoid decomposition and removal during the delivery. Currently, the LNP delivery system is considered as a key technology for promoting nucleic acid pharmaceuticals into therapeutic application.

### SUMMARY OF THE DISCLOSURE

For the current technical question, it was necessary to discover novel ionizable lipid compounds to improve the delivery efficiency and lower the toxicity of nucleic acid pharmaceuticals, such as mRNA and siRNA. The disclosure provides a series of novel ionizable lipid compounds which form the aliphatic chain by ester group of glycerol and ether group. The delivery effects of such lipids are better than the ionizable lipid of aliphatic chain. The novel ionizable lipids are combined with other lipid ingredients and formed into lipid nanoparticles which deliver mRNA or other pharmaceutical agents effectively in vivo where the intended biological function occurs. For example, delivering siRNA into a cell plays a role in gene silencing therapy; delivering mRNA into a cell can translate to protein or antigen efficiently for vaccine or pharmaceutical therapy; delivering antibody in vivo plays a role in therapy; and delivering Cas 9 mRNA in vivo plays a role in gene editing.

### BRIEF DESCRIPTION OF THE DRAWINGS

Figure 1 is a graph depicting the weight change of male rat in LNP safety evaluation.
Figure 2 depicts the weight change of female rat in LNP safety evaluation.
Figure 3 depicts the food uptake change of male rat in LNP safety evaluation.
Figure 4 depicts the food uptake change of female rat in LNP safety evaluation.
Figure 5 depicts the IgG antibody titer of LNP-mRNA in an immunogenicity study.

### DETAILED DESCRIPTION

The disclosure provides a series of novel ionizable lipids, synthesis methods thereof, and pharmaceutical molecules mixed and encapsulated by a mixture comprising ionizable lipid, PEG lipid, structural lipid (such as, cholesterol) and phospholipid, thereby forming a nanoparticle delivery system which can be used for in vitro cell delivery and in vivo organ targeted cell delivery. The invention is defined by the appended claims. The more generic description is provided for illustrative purposes only. Embodiments not falling under these claims are for reference purposes only.

In one embodiment, the disclosure relates to a compound of the following formula (I): 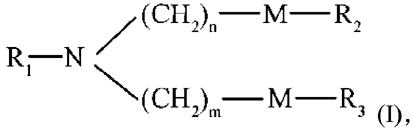
wherein R₁ is selected from -R₁'-X,
R₁' is -(CH₂)₀₋₆-, and X is amino, hydroxyl, ethynyl, cyano, -C(O)(CH₂)₁₋₃NRₐR_{b}, -C(O)O(CH₂)₁₋₃NRₐR_{b}, -OC(O)(CH₂)₁₋₃NRₐR_{b}, -C(O)NH(CH₂)₁₋₃NRₐR_{b}, -NHC(O)(CH₂)₁₋₃NRₐR_{b}, -NHC(O)CH(NRₐR_{b})(CH₂)₁₋₃NRₐR_{b}, C₃₋₇ cycloalkyl, 4-7 membered heterocyclic group, C₆₋₁₀ aryl or 5-10 membered heteroaryl, the said cycloalkyl, heterocyclic group, aryl or heteroaryl are optionally substituted by the following groups: -(CH₂)₁₋₃OH, -(CH₂)₁₋₃NRₐR_{b}, -(CH₂)₁₋₃C(O)NRₐR_{b}; or X can also be: 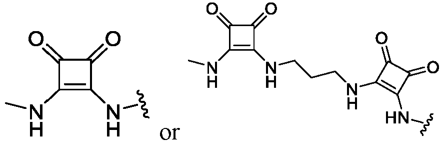
Rₐ, and R_{b} are independently selected from H, C₁₋₃ alkyl, -(CH₂)₁₋₃NH₂, -(CH₂)₁₋₃NH(CH₂)₁₋₃NH₂; or Ra and Rb together with the nitrogen to which they are connected form a 5-10 membered heterocycle that includes 1-3 heteroatoms selected from N, O or S, said heterocycle is optionally substituted by the following groups: C₁₋₆ alkyl, C₁₋₆ alkyl halide, C₁₋₆ alkyl hydroxyl group and C₁₋₆ alkyl amino group;
R₂, and R₃ are independently selected from H, C₂₋₁₈ alkyl, C₄₋₁₈ alkenyl or 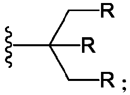
each M is independently selected from -CH₂-, -CH=CH-, -NH-, -C(O)-, -O-, -C(O)O-, -OC(O)-, -C(O)NH-, or -NHC(O)-;
each R is independently selected from H, R', -OR* or -R"OR*;
each R' is independently selected from C₁₋₁₀ alkyl or C₃₋₁₂ alkenyl;
each R" is independently selected from C₁₋₁₀ alkyl or C₃₋₁₂ alkenyl;
each R* is independently selected from C₁₋₁₀ alkyl or C₃₋₁₂ alkenyl;
n, and m are independently an integer independently selected from from 1 through 9;
or a salt or an isomer thereof.

In one embodiment, the ionizable lipid is a compound of formula (I), wherein:
R' is -(CH₂)₂₋₃-, and X is hydroxyl, -C(O)(CH₂)₂₋₃NRₐR_{b}, -C(O)O(CH₂)₂₋₃NRₐR_{b}, -C(O)NH(CH₂)₂₋₃NRₐR_{b}, or 5-10 heteroaryl which is optionally substituted by one or more of the following groups: -(CH₂)₂₋₃OH, -(CH₂)₂₋₃NRₐR_{b}, -(CH₂)₂₋₃C(O)NRₐR_{b} ; or X can also be: 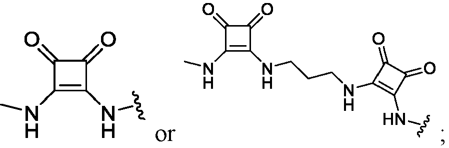
Rₐ, and R_{b} are independently selected from H, C₁₋₃ alkyl, -(CH₂)₂₋₃NH₂, -(CH₂)₂₋₃NH(CH₂)₂₋₃NH₂; or the 5-10 membered heterocycle including 1-3 heteroatoms selected from N or O , which is formed together by Rₐ, R_{b} and their connected nitrogen, the said heterocycle is optionally substituted by the one or more of the following groups: C₁₋₆ alkyl, C₁₋₆ alkyl halide, C₁₋₆ alkyl hydroxyl group and C₁₋₆ alkyl amino group.

In one embodiment, the ionizable lipid is a compound of formula (I), wherein:
each M is independently selected from -CH₂-, -CH=CH-, -C(O)O-, -OC(O)-, -C(O)NH-, or -NHC(O)-.

In one embodiment, the compound of formula (I) is a compound of formula (II): 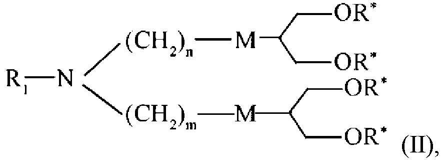 wherein:
each R* is independently selected from C₂₋₁₀ alkyl, preferably C₆₋₁₀ alkyl, preferably C₆ alkyl.

In one embodiment, the ionizable lipid is a compound of formula (II), wherein:
each M is independently selected from -C(O)O- or -OC(O)-, preferably -C(O)O-.

In one embodiment, the ionizable lipid is acompound of formula (II), wherein:
R₁ is selected from -R₁'-X, R₁' is -(CH₂)₁₋₆-, and X is hydroxyl.

In one embodiment, the ionizable lipid is acompound of formula (II), wherein:
R₁ is selected from -R₁'-X, R₁' is -(CH₂)₁₋₆-, and X is -C(O)(CH₂)₂₋₃NRₐR_{b}, - C(O)O(CH₂)₂₋₃NRₐR_{b}, -C(O)NH(CH₂)₂₋₃NRₐR_{b},
Rₐ. and R_{b} are independently selected from H, C₁₋₃ alkyl, -(CH₂)₂₋₃NH₂; or 5-10 membered heterocycle containing 1-3 heteroatoms selected from N or O, which is formed together by Rₐ, and R_{b} and their connected nitrogen atom, preferably morpholinyl or piperidinyl, the said heterocycle is optionally substituted by C₁₋₆ alkyl hydroxyl.

In one embodiment, the ionizable lipid is a compound of formula (II), wherein:
R₁ is selected from -R₁'-X, R₁' is -(CH₂)₁₋₆-, X is 5-6 membered heteroaromatic group, preferably triazolyl, said heteroaromatic group is optionally substituted by one or more of the the following groups: -(CH₂)₂₋₃OH, -(CH₂)₂₋₃NRₐR_{b}, and -(CH₂)₂₋₃C(O)NRₐR_{b},
Rₐ, and R_{b} are independently selected from H, C₁₋₃ alkyl, -(CH₂)₂₋₃NH₂, -(CH₂)₂₋₃NH(CH₂)₂₋₃NH₂, or 5-10 membered heterocycle containing 1-3 heteroatoms selected from N or O, which is formed together by Rₐ, and R_{b} and their connected nitrogen atom, preferably morpholinyl, piperazinyl or piperidinyl, the said heterocycle is optionally substituted by one or more of the following groups: C₁₋₆ alkyl, and hydroxyl.

In one embodiment, the ionizable lipid is a compound of formula (II), wherein:
R₁ is selected from -R₁'-X, R₁' is -(CH₂)₁₋₆-, and X is

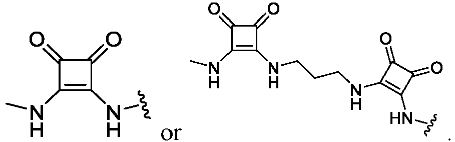

In one embodiment, the ionizable lipid is a compound of formula (II), wherein:
each n is 7, m is 7.

In one embodiment, the compound of formula (I) is a compound of formula (III):

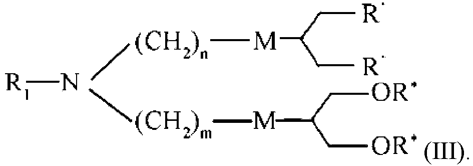

In one embodiment, the ionizable lipid is a compound of formula (III), wherein:
each R' is independently selected from C₁₋₁₀ alkyl, preferably C₂₋₈ alkyl.

In one embodiment, the ionizable lipid is acompound of formula (III), wherein:
each M is independently selected from -C(O)O- or -OC(O)-, preferably -C(O)O-.

In one embodiment, the ionizable lipid is a compound of formula (I) is compound of formula (IV):

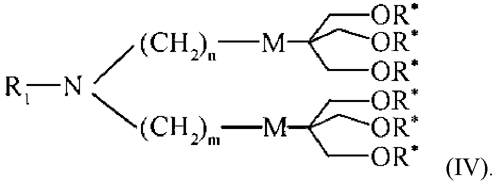

In one embodiment, the ionizable lipid is a compound of formula (IV), wherein:
each R* is independently selected from C₂₋₁₀ alkyl, preferably C₆₋₁₀ alkyl, preferably C₆ alkyl.

In one embodiment, the ionizable lipid is a compound of formula (IV), wherein:
each M is independently selected from -C(O)O- or -OC(O)-, preferably -C(O)O-.

In one embodiment, the ionizable lipid is a compound of formula (I) is a compound of formula (V):

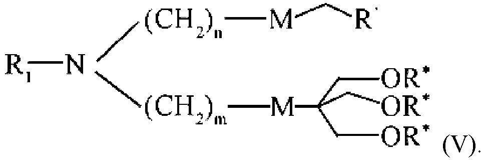

In one embodiment, the ionizable lipid is a compound of formula (V), wherein:
each R* is independently selected from C₂₋₁₀ alkyl, preferably C₆₋₁₀ alkyl, preferably C₆ alkyl.

In one embodiment, the ionizable lipid is a compound of formula (V), wherein:
each M is independently selected from -CH=CH-, -C(O)O- or -OC(O)-, preferably -CH=CH- or -C(O)O-.

In one embodiment, the ionizable lipid is a compound of formula (V), wherein:
each R' is independently selected from C₁₋₁₀ alkyl or C₃₋₁₂ alkenyl, preferably C₁₀ alkyl or C₈ alkenyl.

In another embodiment, the compound is a salt of any of the prior embodiments.

In another embodiment, the compound is a stereoisomer of any of the prior embodiments.

In one embodiment, the said compound is selected from the following compounds, salts or stereoisomers thereof: A1, A5, A6, A7, A9, A10, A11, A12, A13, A15, A16, A17, A18, A19, A20, A21, A22, A23, A24, A25, A26, A27, A28,A29, A30, A31, A32, A34, A35, A36, A37, A38, A39, A40, A41, A42, A43, A44, A45, A46, A47, and 48.

In one embodiment, the disclosure related to a composition comprising a ionizable lipid compound according to the claim 1, in admixture with a PEG lipid, a structural lipid and a phospholipid.

In one embodiment, the phospholipids are selected from at least any one of the following groups: 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), l,2-dipalmitoyl-sn-glycero-3-phosphocholine (DPPC), l,2-distearoyl-sn-glycero-3 - phosphocholine (DSPC), 1 ,2-diundecanoyl-sn-glycero-phosphocholine (DUPC), 1-palmitoyl-2-oleoyl-sn-glycero-3 -phosphocholine (POPC), l,2-di-0-octadecenyl-5«-glycero-3 -phosphocholine (18:0 Diether PC), l-oleoyl-2-cholesterylhemisuccinoyl-5«-glycero-3-phosphocholine (OChemsPC), l-hexadecyl-sn-glycero-3 -phosphocholine (C16 Lyso PC), 1 ,2-dilinolenoyl-sn-glycero-3 -phosphocholine, l,2-diarachidonoyl-sn-glycero-3 -phosphocholine, l,2-didocosahexaenoyl-sn-glycero-3-phosphocholine, l,2-dioleoyl-sn-glycero-3-phosphoethanol amine (DOPE), l,2-diphytanoyl-sn-glycero-3-phosphoethanolamine (ME 16.0 PE), l,2-distearoyl-sn-glycero-3-phosphoethanolamine, 1 ,2-dilinoleoyl-sn-glycero-3 -phosphoethanolamine, l,2-dilinolenoyl-sn-glycero-3-phosphoethanolamine, l,2-diarachidonoyl-sn-glycero-3 -phosphoethanolamine, 1,2-didocosahexaenoyl-sn-glycero-3-phosphoethanolamin, l,2-dioleoyl-sn-glycero-3-phospho-rac-(l -glycerol) sodium salt (DOPG), dipalmitoylphosphatidylglycerol (DPPG), palmitoyloleoylphosphatidylethanolamine (POPE), distearoyl-phosphatidyl-ethanolamine (DSPE), dipalmitoyl phosphatidyl ethanolamine (DPPE), dimyristoylphosphoethanolamine (DMPE), 1 -stearoyl-2-oleoyl-phosphatidyethanolamine (SOPE), l-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine (LPE). One or more of the recited phospholipids can be used in the mixture.

In one embodiment, the PEG lipid is selected from at least any one of the following groups: PEG-modified phosphatidylethanolamine, PEG- modified phosphatidic acid, PEG-modified ceramide, PEG-modified dialkylamine, PEG- modified diacylglycerol, PEG-modified dialkylglycerol.One or more PEG lipids can be used in the mixture.

In one embodiment, the structural lipid is selected from at least any one of the following groups: cholesterol, fecosterol, sitosterol, ergosterol, campesterol, stigmasterol, brassicasterol, tomatidine, ursolic acid, alpha-tocopherol. One or more structural lipids can be used in the mixture.

In one embodiment, in the composition, ionizable lipid compound is from 20% to 80%, PEG lipid is from 1% to 10%, structural lipid is from 10% to 50% and phospholipid is from 5% to 30%, each of these percentages being calculated based on mole percentage of all lipids in the composition. In another embodiment, ionizable lipid compound is 20%, 25%, 30%, 35%, 40%, 45%, 50%, 55%, 60%, 65%, 70%, 75%, or 80%, calculated based on mole percentage of all lipids in the composition. In another embodiment, PEG lipid is 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10%, calculated based on mole percentage of all lipids in the composition. In another embodiment, structural lipid is 10%, 15%, 20%, 25%, 30%, 35%, 40%, 45%, or 50%, calculated based on mole percentage of all lipids in the composition. In another embodiment, phospholipid is 5%, 10%, 15%, 20%, 25%, or 30%, calculated based on mole percentage of all lipids in the composition..

In one embodiment, the composition is in the form of a lipid nanoparticle.

In another embodiment, the lipid nanoparticle also comprises active ingredient. The active ingredient can be selected from at least any one of: DNA, RNA, protein, or an active pharmaceutical molecule.

In one embodiment, the RNA is selected from at least any one of: mRNA, siRNA, aiRNA, miRNA, dsRNA, aRNA, lncRNA, antisense nucleotide (ASO) or oligonucleotide.

In one embodiment, the protein is selected from at least any one of: antibody, enzyme, recombinant protein, polypeptide and short chain polypeptide.

The disclosure also relates to a method of producing lipid nanoparticles, comprising step (1): mixing the ionizable lipid compound, a PEG lipid, a structural lipid and a phospholipid in ethanol to form a lipid mixture.

The method may further comprise step (2): mixing the lipid mixture with an active ingredient to form lipid nanoparticle by mixer.

In one embodiment, the ionizable lipid compound, PEG lipid or PEG modified lipid, structural lipid and phospholipid are dissolved and mixed in ethanol, then mixed with an active ingredient by mixer to form lipid nanoparticle.

In one embodiment, the disclosure relates to an ionizable compound for use in the production of lipid nanoparticle.

In one embodiment, the lipid nanoparticle is neutral and uncharged in a neutral medium, and is positively charged after being protonated in an acidic medium.

In one embodiment, the lipid nanoparticle is as defined in the specification.

In one embodiment, disclosed is a pharmaceutical composition comprising the lipid nanoparticle and a pharmaceutically acceptable carrier.

In one embodiment, the disclosure relates to the lipid nanoparticle or a pharmaceutical composition thereof for use in the production of medicine.

In one embodiment, the medicine also comprises an active ingredient, wherein the active ingredient comprises at least any one of DNA, RNA, protein, and an active pharmaceutical molecule.

In one embodiment, the RNA is selected from at least any one of: mRNA, siRNA, aiRNA, miRNA, dsRNA, aRNA, and lncRNA.

In one embodiment, the invention relates to the lipid nanoparticle for use in the production of medicine, encapsulating an active ingredient into the said lipid nanoparticle.

In one embodiment, the invention relates to the use of medicine, the medicine can be applied to a human by intravenous injection, intramuscular injection, subcutaneous injection, microneedle patch, oral administration, oral and nasal spray, or painting.

The structures of representative ionizable lipid compounds of the disclosure are shown as follows:

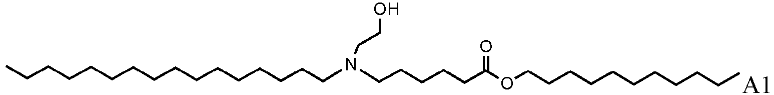

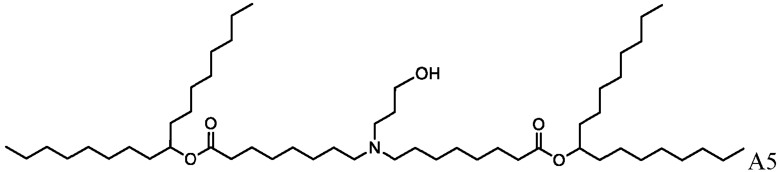

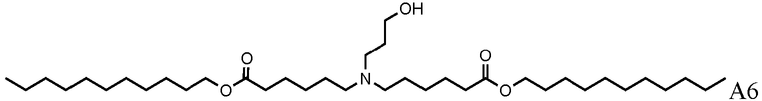

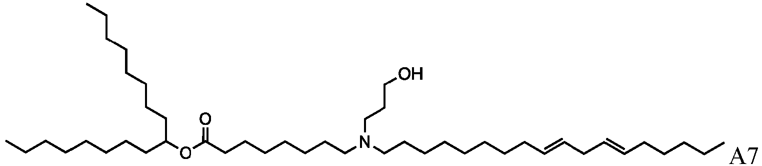

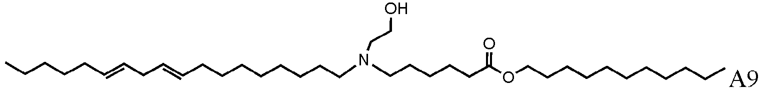

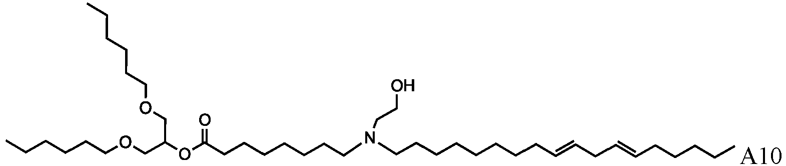

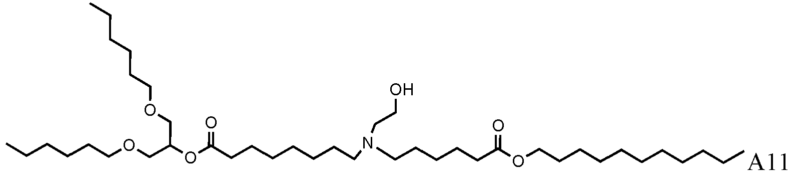

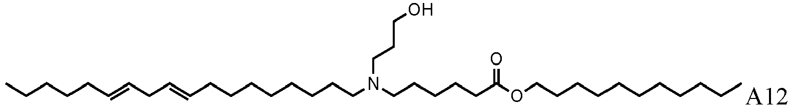

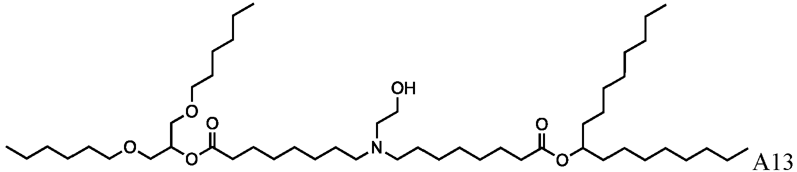

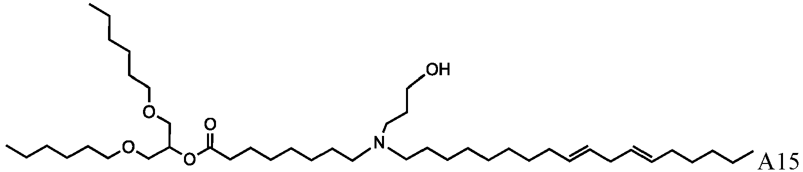

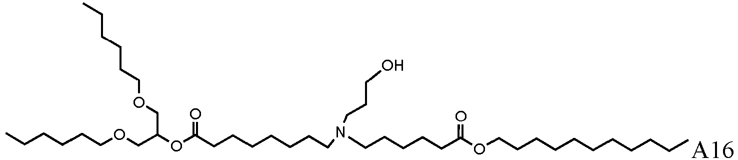

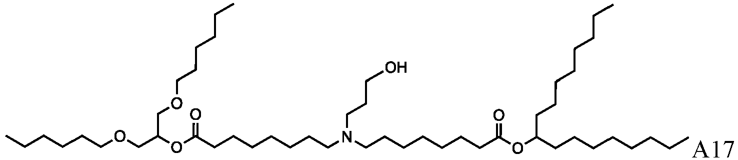

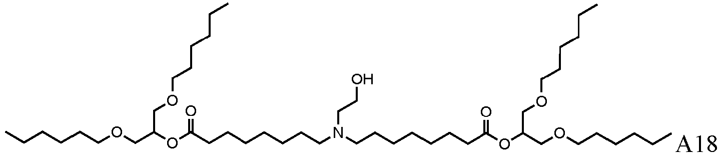

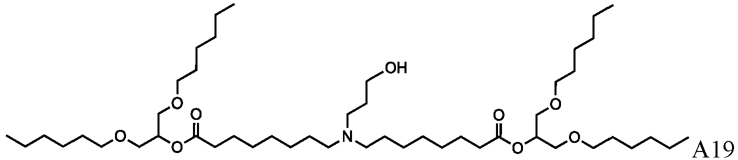

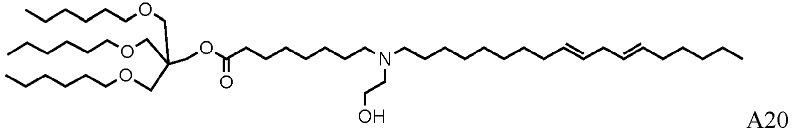

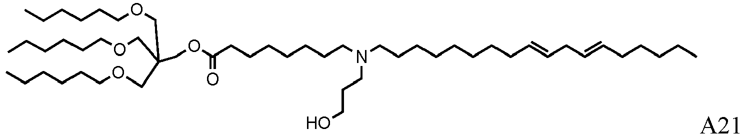

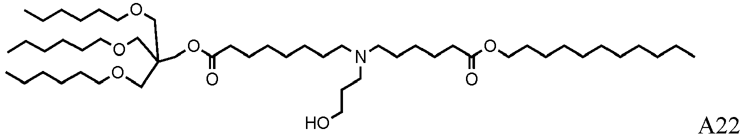

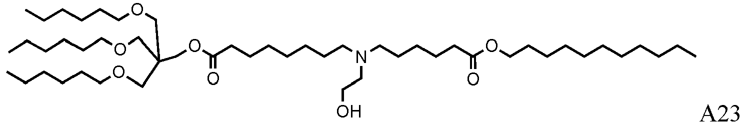

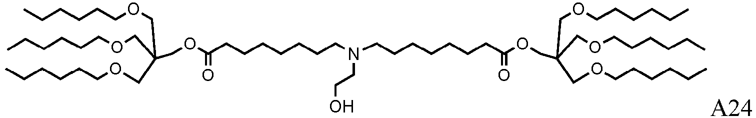

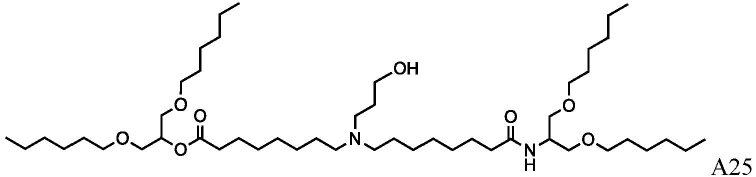

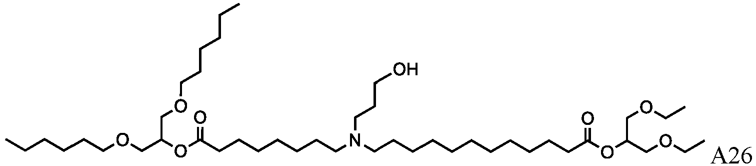

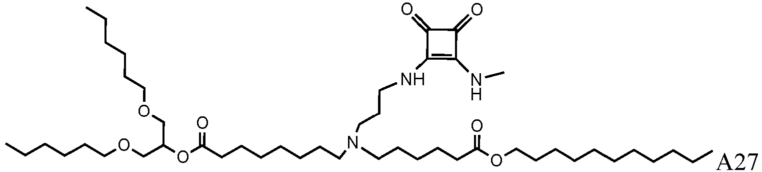

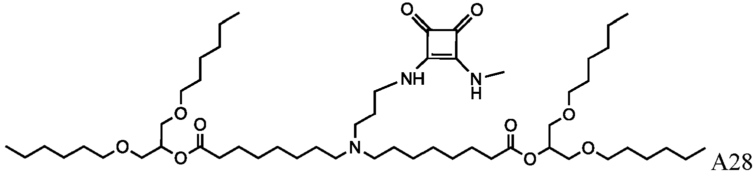

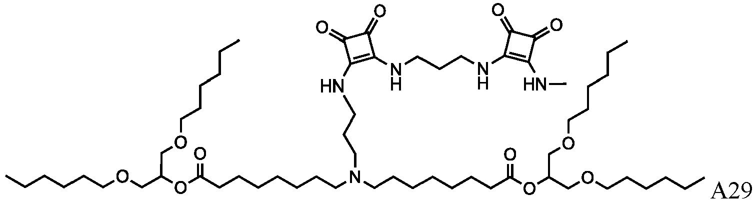

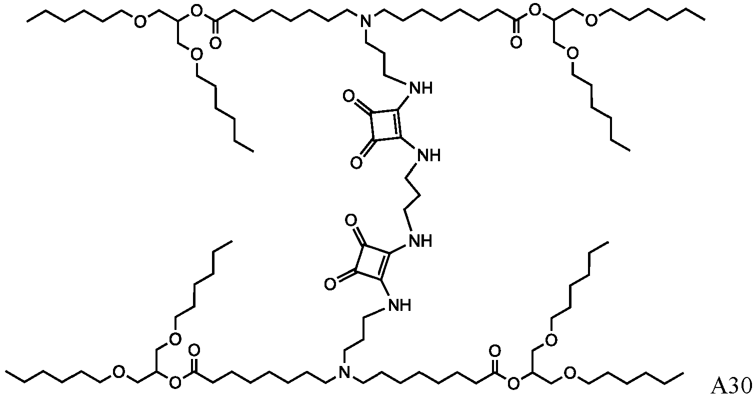

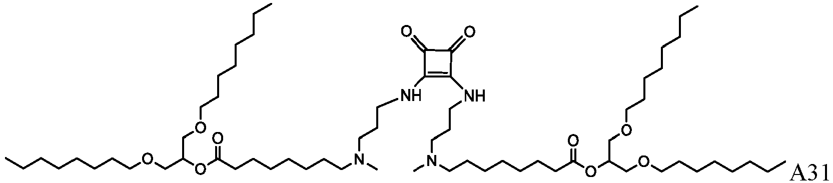

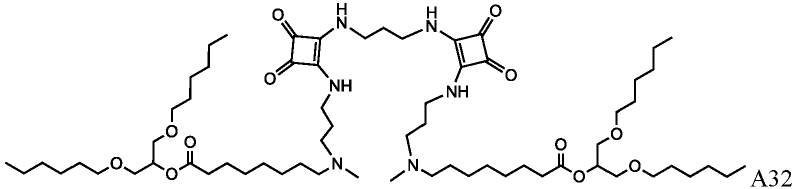

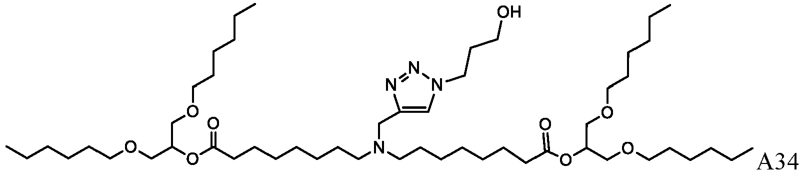

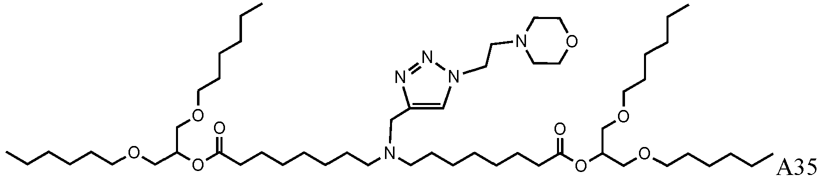

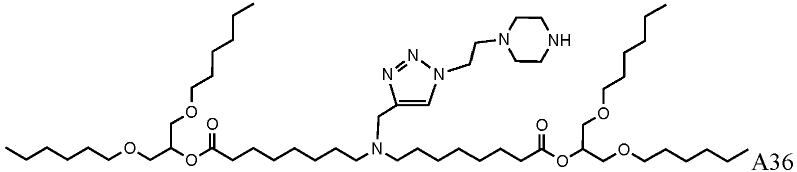

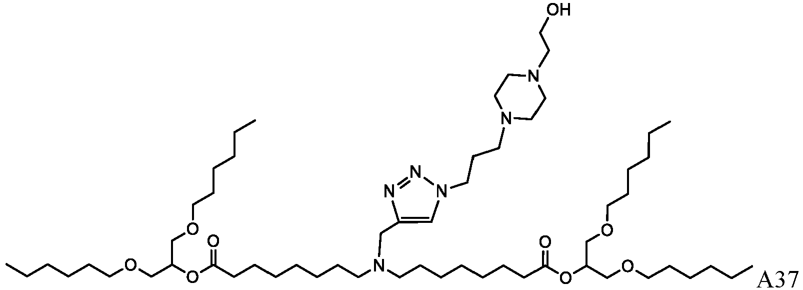

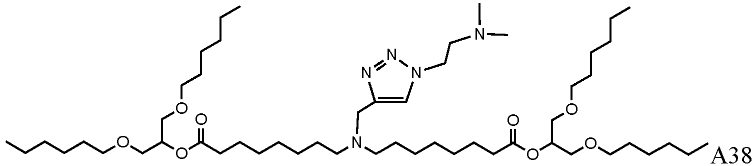

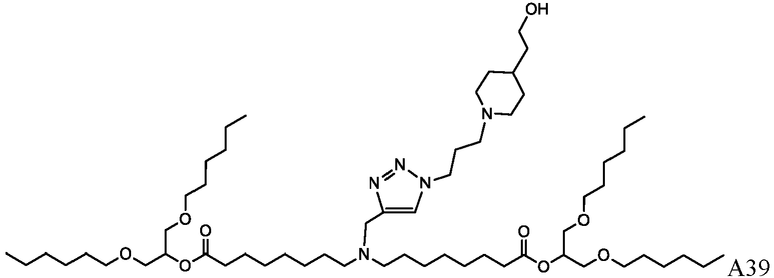

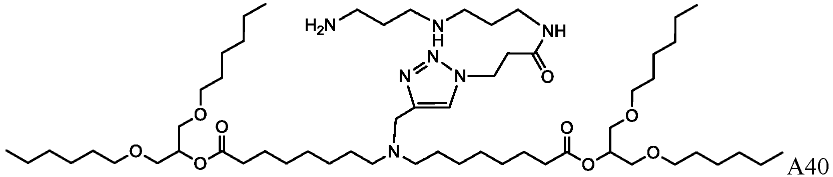

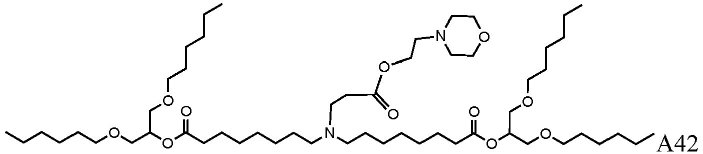

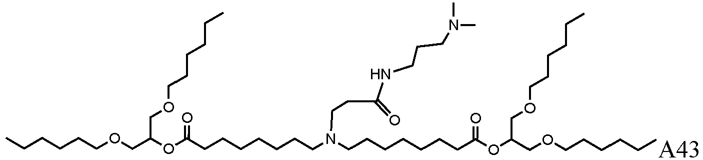

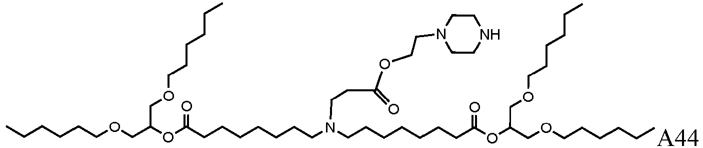

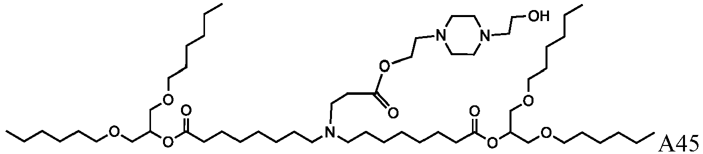

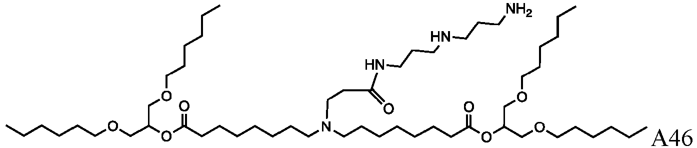

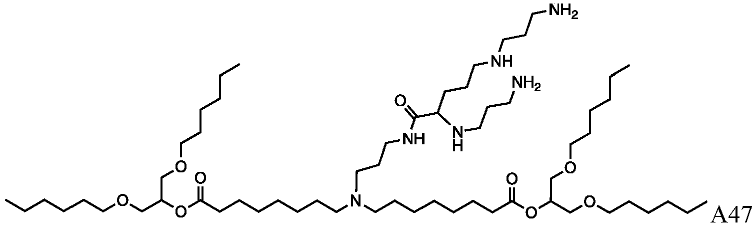

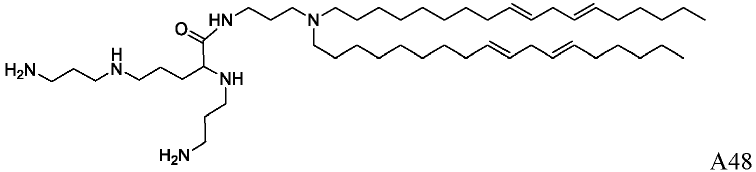

Compared with the prior art, such as PCT/US2016/052352 (published as WO2017/049245), and PCT/US2010/038224 (published as WO2010/144740), the ionizable lipids of the present disclosure differ from ionizable lipids disclosed in these documents in one or more of the following ways:
1. Different chemical structure: the 1 or 2 aliphatic chains that are connected to nitrogen (N) of tertiary amine contain an ester group formed with another saturated or unsaturated aliphatic chain having glycerol structure to become novel aliphatic chain with ether groups, the outcome shows that this transfection efficiency is higher than ionizable lipid having aliphatic chains lacking ether groups;
2. Different metabolites: the aliphatic chain of ionizable lipids of the disclosure consists of ester group, glycerol and short aliphatic chains. The metabolites of such molecules are small molecule compounds, such as short fatty acids, fatty alcohols or ethers, which are capable of being metabolized and extracted more easily and hard to accumulate in vivo, as well as lower toxicity.
3. Novel alkynyl intermediate structure: the alkynyl intermediate is formed by propargylamine and brominated aliphatic chain, which can click react with many azido compounds to generate a series of novel ionizable aliphatic compounds.
4. Ionizable lipids of the disclosure are easy to synthesize and easy to obtain the raw materials: the original raw materials are glycerol, short fatty alcohols and fatty acids, which are relatively inexpensive and easy to synthesize.

### DEFINITION

When the numeric range is listed, it includes each value and the subrange within the said range. For example, "C₁₋₆ alkyl" includes C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅ and C₅₋₆ alkyl.

The term "alkyl" refers to straight or branched saturated alkyl containing one or several carbon atoms (such as, 1, 2, 3, 4, 5, 6, 7, 8, 9, 10, 11, 12, 13, 14, 15, 16, 17, 18, 19, 20 or more carbon atoms). Specifically, "C₁₋₁₀ alkyl" refers to straight or branched saturated alkyl containing 1-10 carbon atoms. "C₂₋₁₈ alkyl" refers to randomly substituted straight or branched saturated alkyl containing 2-18 carbon atoms. Unless otherwise specified, the said alkyl in this specification refers to unsubstituted and substituted alkyl.

The term "alkenyl" refers to straight or branched alkyl containing two or more carbon atoms and at least one carbon-carbon double bond (such as, 2, 3, 4, 5, 6, 7, 8, 9, 10, 11, 12, 13, 14, 15, 16, 17, 18, 19, 20 or more carbon atoms). The alkenyl can include one, two, three, four or more carbon-carbon double bond. Specifically, "C₃₋₁₂ alkenyl" refers to straight or branched saturated alkenyl containing 3-12 carbon atoms and at least one carbon-carbon double bond. Specifically, "C₄₋₁₈ alkenyl" refers to straight or branched saturated alkenyl containing 4-18 carbon atoms and at least one carbon-carbon double bond. Unless otherwise specified, the said alkenyl in this specification refers to unsubstituted and substituted alkenyl.

The term "halogen" refers to fluorine (F), chlorine (Cl), bromine (Br) and iodine (I).

The term "C₁₋₆ alkyl halide" refers to the above mentioned "C₁₋₆ alkyl" is substituted by one or more halogen groups. Examplary said alkyl halide includes but not limited to -CF₃, -CH₂F, -CHF₂, -CHFCH₂F, -CH₂CHF₂, -CF₂CF₃, -CCl₃, -CH₂Cl, -CHCl₂, and 2,2,2-trifluoro-1, 1-dimethyl-ethyl.

The term " C₃₋₇cycloalkyl" refers to non-aromatic cyclic hydrocarbon group containing 3-7 cyclocarbon atoms and 0 heteroatom. Examplary cycloalkyl groups include but are not limited to: cyclopropyl (C3), cyclopropenyl (C3), cyclobutyl (C4), cyclobutenyl (C4), cyclopentyl (C5), cyclopentenyl (C5), cyclohexyl (C6), cyclohexenyl (C6), cyclohexadienyl (C6), cycloheptyl (C7), cycloheptenyl (C7), cycloheptadienyl (C7), cycloheptatrienyl (C7), etc. The cycloalkyl group can be optionally substituted by one or more substituents, for example, it is substituted by 1-5 substituents, 1-3 substituents or 1 substituent.

The term "4-10 membered heterocyclyl" refers to 4-10 membered non-aromatic ring system containing ring carbon atom and 1-3 ring heteroatoms, wherein each heteroatom is independently selected from nitrogen, oxygen, sulfur, boron, phosphorus and silicon. Likewise, the term "4-7 membered heterocyclyl" and "5-10 membered heterocyclyl" are also have the same definition except that the total number of carbon atoms and heteroatoms varies for each grouping. In the heterocyclyl containing one or several nitrogen atoms, the point of attachment can be carbon or nitrogen atom as long as the valence allows. Examplary 3 membered heterocyclyl groups containing one heteroatom include but are not limied to: aziridinyl, oxiranyl and thiorenyl. Examplary 4 membered heterocyclyl groups containing one heteroatom include but are not limied to: azetidinyl, oxetanyl, and thietanyl. Examplary 5 membered heterocyclyl groups containing one heteroatom include but are not limied to: tetrahydrofuranyl, dihydrofuryl, tetrahydrothienyl, dihydrothienyl, pyrrolidinyl, dihydropyrrolyl and pyrrolyl-2, 5-dione. Examplary 5 membered heterocyclyl grouops containing two heteroatoms include but are not limited to: dioxolanyl, oxasulfuranyl, disulfuranyl, and oxazolidin-2-ketone. Examplary 5 membered heterocyclyl groups containing three heteroatoms include but are not limited to: triazolinyl, oxadiazolinyl and thiadiazolinyl. Examplary 6 membered heterocyclyl groups containing one heteroatom include but are not limited to: piperidinyl, tetrahydropyranyl, dihydropyridyl and thianyl. Examplary 6 membered heterocyclyl groups containing two heteroatoms include but are not limited to: piperazinyl, morpholinyl, dithianyl, dioxanyl. Examplary 6 membered heterocyclyl groups containing three heteroatoms include but are not limited to: hexahydrotriazinyl. Examplary 7 membered heterocyclyl groups containing one heteroatom include but are not limited to: azepanyl, oxepanyl and thiepanyl.

The term "C₆₋₁₀ aryl" refers to monocyclic or polycyclic (e.g., bicyclic) 4n+2 aromatic ring system (e.g., including 6 or 10 π electrons shared in a cyclic array) containing 6-10 ring carbon atoms and 0 heteroatom. In some embodiments, the aryl has six ring carbon atoms ("C₆ aryl"; e.g., phenyl). In some embodiments, the aryl has ten ring carbon atoms ("C₁₀ aryl"; e.g., naphthyl, e.g., 1-naphthyl and 2- naphthyl).

The term "5-10 membered heteroaryl" refers to 5-10 membered monocyclic or bicyclic 4n+2 aromatic ring system (e.g., including 6 or 10 π electrons shared in a cyclic array) containing ring carbon atom and 1-4 heteroatoms, wherein each heteroatom is independently selected from nitrogen, oxygen and sulfur. In the heteroaryl containing one or more nitrogen atoms, the point of attachment could be carbon or nitrogen atom, as valence permits. The heteroaryl bicyclic system in one or two rings can include one or more heteroatoms. The heteroaryl also includes ring system fused by the above mentioned heteroaryl ring and one or more cycloalkyl or heterocyclyl wherein the point of attachment located on the said heteroaryl ring, and the number of carbon atoms still continue to represent the number of carbon atoms in the heteroaryl ring system. Exemplary 5 membered heteroaryl groups containing one heteroatom include but are not limited to: pyrrolyl, furanyl and thiophenyl. Exemplary 5 membered heteroaryl groups containing two heteroatoms include but are not limited to: imidazolyl, pyrazolyl, oxazolyl, isoxazolyl, thiazolyl and isothiazolyl. Exemplary 5 membered heteroaryl groups containing three heteroatoms include but are not limited to: triazolyl, oxadiazolyl (e.g., 1,2,4-oxadiazolyl) and thiadiazolyl. Exemplary 5 membered heteroaryl groups containing four heteroatoms include but are not limited to: tetrazolyl. Exemplary 6 membered heteroaryl groups containing one heteroatom include but are not limited to: pyridinyl. Exemplary 6 membered heteroaryl groups containing two heteroatoms include but are not limited to: pyridazinyl, pyrimidinyl and pyrazinyl. Exemplary 6 membered heteroaryl groups containing three or four heteroatoms include but are not limited to: triazinyl and tetrazinyl. Exemplary 7 membered heteroaryl groups containing one heteroatom include but are not limited to: azepinyl, oxepinyl and thiepinyl. Exemplary 5, 6-bicyclic heteroaryl groups include but are not limited to: indolyl, isoindolyl, indazolyl, benzotriazolyl, benzothienyl, isobenzothienyl, benzofuranyl, benzoisofuranyl, benzimidazolyl, benzoxazolyl, benzisoxazolyl, benzoxadiazolyl, benzothiazolyl, benzisothiazolyl, benzothiadiazolyl, indolizinyl and purinyl. Exemplary 6, 6 bicyclic heteroaryl groups include but are not limited to: naphthyridinyl, pterridinyl, quinolinyl, isoquinolinyl, cinnolinyl, quinoxalinyl, phthalazinyl and quinazolinyl.

The term "isomer" refers to different compounds with the same molecular formula. The disclosure especially relates to stereoisomers, the term "stereoisomer" refers to isomers that are only different in the atom space arrangement.

In some situations, the invention's compounds can form into salts, which are also in the scope of the invention. The term "salt (one or more)" refers to acidic and/or basic salts formed by inorganic and/or organic acids and bases. The salts of compounds of the invention are preferably the pharmaceutically acceptable salts.

The term "pharmaceutically acceptable salts" refers to those carboxylate salts, amino acid addition salts of the compounds of the present invention which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention.

Pharmaceutically acceptable base addition salts are formed with metals or amines, such as alkali and alkaline earth metal hydroxides, or of organic amines. Examples of metals used as cations are sodium, potassium, magnesium, calcium, and the like. Examples of suitable amines are N, N'-dibenzylethylenediamine, chloroprocaine, choline, diethanolamine, ethylenediamine, N-methylglucamine, and procaine.

The base addition salts of acidic compounds are prepared by contacting the free acid form with a sufficient amount of the desired base to produce the salt in the conventional manner. The free acid form may be regenerated by contacting the salt form with an acid and isolating the free acid in a conventional manner. The free acid forms differ from their respective salt forms somewhat in certain physical properties such as solubility in polar solvents, but otherwise the salts are equivalent to their respective free acid for purposes of the present invention.

Salts may be prepared from inorganic acids sulfate, pyrosulfate, bisulfate, sulfite, bisulfite, nitrate, phosphate, monohydrogenphosphate, dihydrogenphosphate, metaphosphate, pyrophosphate, chloride, bromide, iodide such as hydrochloric, nitric, sulfuric, hydrobromic, hydriodic, phosphorus, and the like. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactobionate, laurylsulphonate and isethionate salts, and the like. Salts may also be prepared from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. and the like. Representative salts include acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Pharmaceutically acceptable salts may include cations based on the alkali and alkaline earth metals, such as sodium, lithium, potassium, calcium, magnesium and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like. Also contemplated are the salts of amino acids such as arginate, gluconate, galacturonate, and the like. (See, for example, Berge S. M. et al., "Pharmaceutical Salts," J. Pharm. Sci., 1977; 66:1-19)

Examples of pharmaceutically acceptable, non-toxic esters of the compounds of this invention include C₁-C₆ alkyl esters wherein the alkyl group is a straight or branched chain. Acceptable esters also include C₅-C₇ cycloalkyl esters as well as arylalkyl esters such as, but not limited to benzyl. C₁-C₄ alkyl esters are preferred. Esters of the compounds of the present invention may be prepared according to conventional methods "March's Advanced Organic Chemistry, 5th Edition". M. B. Smith & J. March, John Wiley & Sons, 2001.

Examples of pharmaceutically acceptable, non-toxic amides of the compounds of this invention include amides derived from ammonia, primary C₁-C₆ alkyl amines and secondary C₁-C₆ dialkyl amines wherein the alkyl groups are straight or branched chain. In the case of secondary amines the amine may also be in the form of a 5- or 6-membered heterocycle containing one nitrogen atom. Amides derived from ammonia, C₁-C₃ alkyl primary amines and C₁-C₂ dialkyl secondary amines are preferred. Amides of the compounds of the invention may be prepared according to conventional methods such as "March's Advanced Organic Chemistry, 5th Edition". M. B. Smith & J. March, John Wiley & Sons, 2001.

The term "acceptable carrier" refers to suitable carrier using current materials for the purpose of the invention, without undue toxicity, irritation, allergic response, and the like, commensurate with a reasonable benefit/risk ratio.

### EXAMPLES

To make the purpose, technological protocol and benefit of the invention clearer, the present invention is illustrated in further details by the following examples, as well as drawings.

### Example 1 - Synthesis of A1

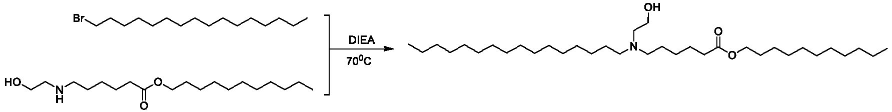

Hexadecane bromide (2.22 g, 7.28 mmol) was soluted in 50mL absolute ethanol, N,N-diisopropylethylamine (DIEA, 1.17 g, 9.10 mmol) and alkanolamine compound (2 g, 6.07 mmol) were added, reacted at 80 °C for 18 h. After the reaction was completed, the solvent was removed by concentrating, the reaction was diluted with 200 mL ethyl acetate (EA), washed once with 200 mL water, extracted, the organic layer was dried over, concentrated, and passed through a silica gel column for purification (DCM:MeOH=3%-5%), 1.2 g oily product was obtained. MS(ES): m/z (M+H)⁺ 553.54_{∘} ¹HNMR (CDCl3) δ:ppm : 4.06(t, 2H), 3.57(t, 2H), 2.62 (bs, 2H), 2.50(br, 4H), 2.29(m, 2H), 1.68-1.25(m, 52H), 0.88(m, 6H).

The synthetic routes for the following examples and synthesis of A5-A7, A9-A13, A15-A32 were designed based on this example, most were as follows: a substitution reaction occurs through a brominated compound or a ketene compound and a primary/secondary amine compound (e.g., alkanolamine compound).

### Example 2 - Synthesis of A5

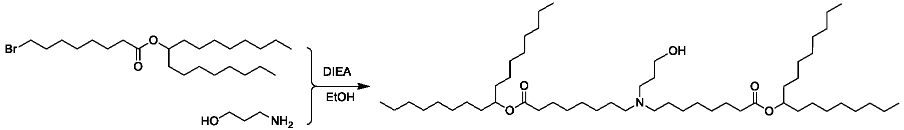

A5 was synthesized employing reaction steps similar to Example 1, 0.75 g oily product was obtained. MS(ES): m/z (M+H)⁺ 835.80_{∘} ¹HNMR (CDCl₃) δ:ppm : 4.87(m,2H), 3.79(t,2H), 2.67(br,2H), 2.45(br,4H), 2.27(t,4H), 1.70-1.25(m,78H), 0.90(m,12H).

### Example 3 - Synthesis of A6

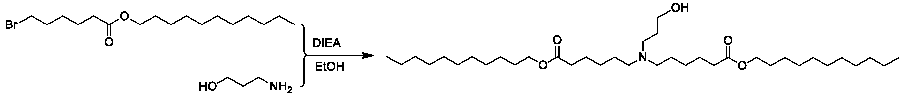

A6 was synthesized employing reaction steps similar to Example 1, 0.51 g oily product was obtained. MS(ES): m/z (M+H)⁺ 611.55_{∘} ¹HNMR (CDCl₃) δ:ppm : 4.05(t,4H), 3.78(m,2H), 2.65(t,2H), 2.43(br,4H), 2.29(m,4H), 1.69-1.31(m,50H), 0.90(m,6H).

### Example 4 - Synthesis of A7

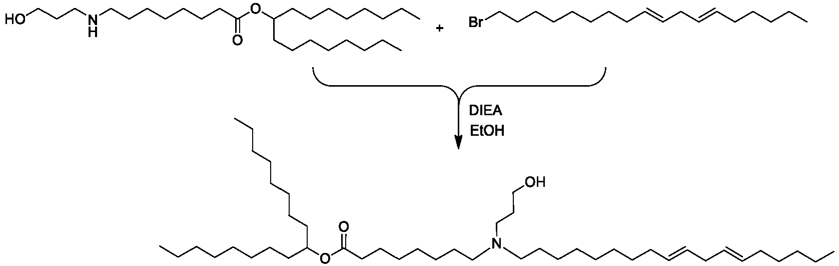

A7 was synthesized employing reaction steps similar to Example 1, 2.45 g oily product was obtained. MS(ES): m/z (M+H)⁺703.68_{∘} ¹HNMR (CDCl₃) δ:ppm : 5.38-5.31(m,4H), 4.86 (m, 1H), 3.79(t,2H), 2.77(m,2H), 2.67(br,2H), 2.45(br,4H), 2.27(m,2H), 2.04(m,4H), 1.70-1.25(m,58H), 0.90(m,9H).

### Example 5 - Synthesis of A9

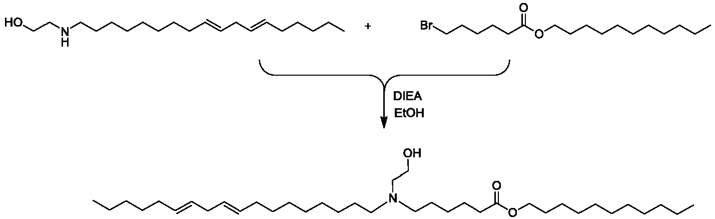

A9 was synthesized employing reaction steps similar to Example 1, 1.6 g oily product was obtained. MS(ES): m/z (M+H)⁺ 577.54_{∘} ¹HNMR (CDCl₃) δ:ppm : 5.38-5.33(m,4H), 4.06(t,2H), 3.60(t,2H), 2.77(t,2H), 2.66(m,2H), 2.54 (bs,4H), 2.30(m,2H), 2.05(m,4H), 1.68-1.25(m,42H), 0.88(m,6H).

### Example 6 - Synthesis of A10

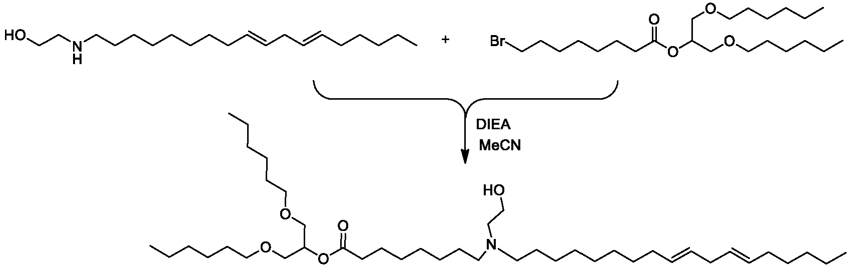

A10 was synthesized employing reaction steps similar to Example 1, 0.4 g oily product was obtained. MS(ES): m/z (M+H)⁺ 693.63_{∘} ¹HNMR (CDCl₃) δ:ppm : 5.36(m,4H), 5.10(m,1H), 3.56(t,4H), 3.46-3.40(br,4H), 2.76(t,2H), 2.64(br,4H), 2.51(bs,4H), 2.32(m,2H), 2.05(m,6H), 1.67-1.25(m,44H), 0.88(m,9H).

### Example 7 - Synthesis of A11

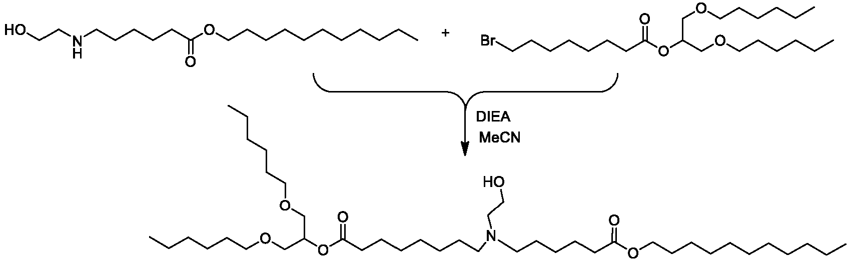

A11 was synthesized employing reaction steps similar to Example 1, 0.5 g oily product was obtained. MS(ES): m/z (M+H)⁺ 713.62_{∘} ¹HNMR (CDCl₃) δ:ppm : 5.10(m, 1H), 4.05(d,4H), 4.03(t,4H), 3.54(m,2H), 3.43(s,2H), 3.16(t,2H), 3.10(br,4H), 2.32(t,4H), 1.66-1.27(m,50H), 0.88(m,9H).

### Example 8 - Synthesis of A12

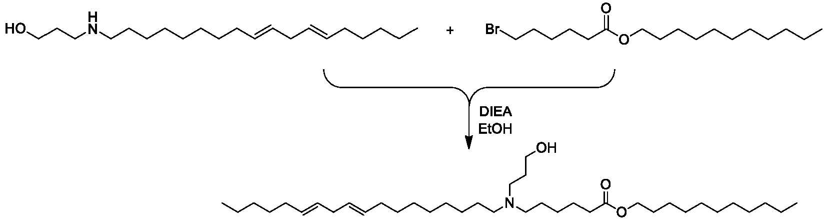

A12 was synthesized employing reaction steps similar to Example 1, 2.68 g oily product was obtained. MS(ES): m/z (M+H)⁺ 591.56, ¹HNMR (CDCl₃) δ:ppm : 5.37-5.35(m,4H), 4.05(t,2H), 3. 78(t,2H), 2.77(t,2H), 2.64(m,2H), 2.41(bs,4H), 2.31(m,2H), 2.03(m,4H), 1.68-1.25(m,44H), 0.88(m,6H).

### Example 9 - Synthesis of A13

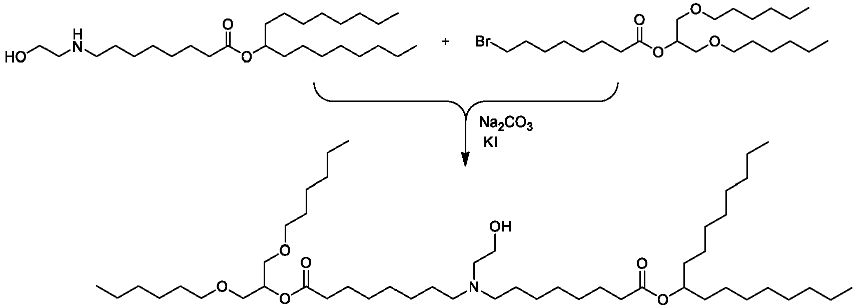

A13 was synthesized employing reaction steps similar to Example 1, 1.2 g oily product was obtained. MS(ES): m/z (M+H)⁺ 825.74 ; ¹HNMR (CDCl₃) δ:ppm : 5.12(m,1H), 4.86(m,1H), 3.65-3.40(m, 10H), 2.72(br,2H), 2.60(br,4H), 2.34-2.26(m,4H), 1.62-1.25(m,64H), 0.88(m, 12H).

### Example 10 - Synthesis of A15

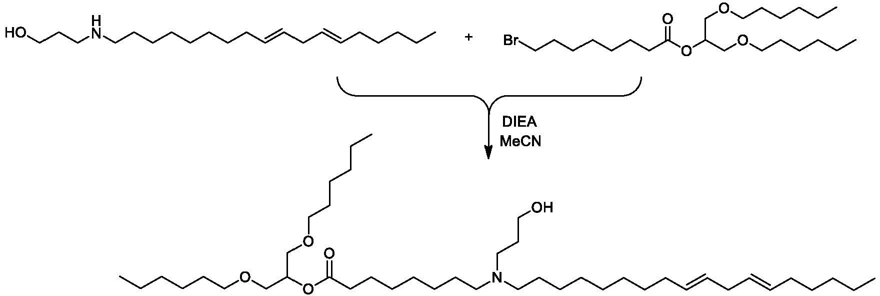

A15 was synthesized employing reaction steps similar to Example 1, 0.4 g oily product was obtained. MS(ES): m/z (M+H)⁺ 707.64_{∘} ¹HNMR (CDCl₃) δ:ppm : 5.34(m,4H), 5.10(m,1H), 3.79(t,2H), 3.56-3.41(m,8H), 2.80(t,2H), 2.77(t,2H), 2.46(br,4H), 2.32(m,2H), 2.05(m,4H), 1.67-1.25(m,46H), 0.88(m,9H).

### Example 11 - Synthesis of A16

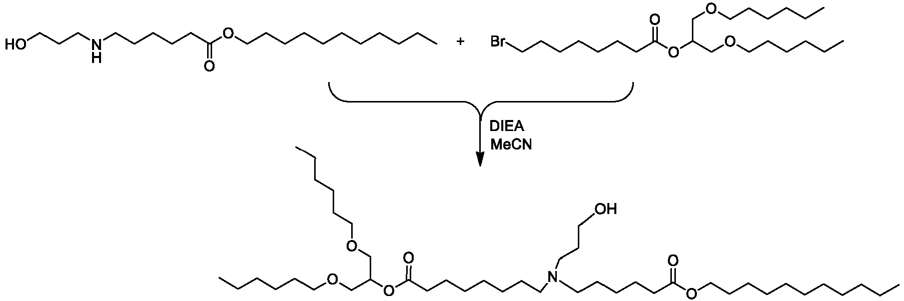

A16 was synthesized employing reaction steps similar to Example 1, 0.59 g oily product was obtained. MS(ES): m/z (M+H)⁺ 727.63_{∘} ¹HNMR (CDCl₃) δ:ppm : 5.10(m, 1H), 4.05(dd,2H), 3.77(t,2H), 3.54(dd,4H), 3.46-3.38(m,4H), 3.19(t,2H), 3.01(br,4H), 2.32(t,4H), 1.66-1.27(m,52H), 0.88(m,9H).

### Example 12 - Synthesis of A17

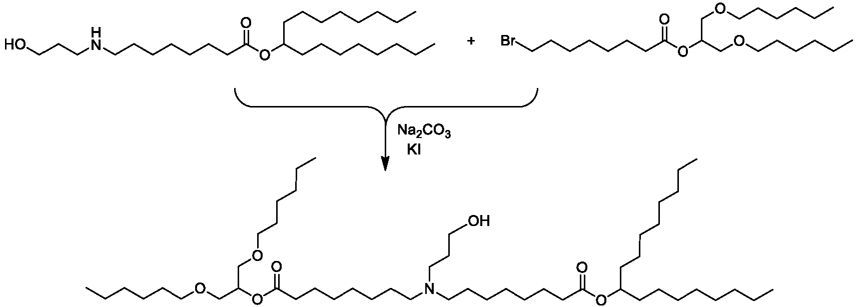

A17 was synthesized employing reaction steps similar to Example 1, 1.1 g oily product was obtained. MS(ES): m/z (M+H)⁺ 839.76 ; ¹HNMR (CDCl₃) δ:ppm : 5.12(m, 1H), 4.86(m,1H), 3.79(t,2H),3.55(t,4H), 3.41(m,4H), 2.67(br,2H), 2.44(br,4H), 2.32-2.26(t,4H), 1.62-1.25(m,66H), 0.88(m,12H).

### Example 13 - Synthesis of A18

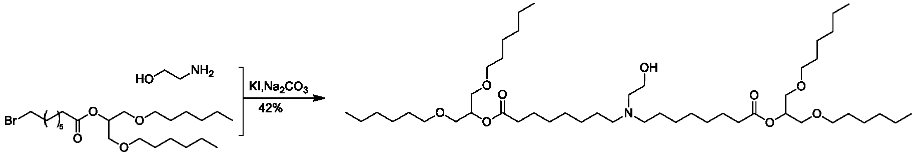

A18 was synthesized employing reaction steps similar to Example 1, 1.44 g oily product was obtained. ¹HNMR (CDCl₃) δ: ppm.5.11(t, 2H), 3.57-3.37(m, 18H), 2.57(t, 2H), 2.44(t,4H), 2.32(m, 4H), 1.62-1.27(m, 52H),0.88(m, 12H), MS(ES): m/z (M+H)⁺ 829.70.

### Example 14 - Synthesis of A19

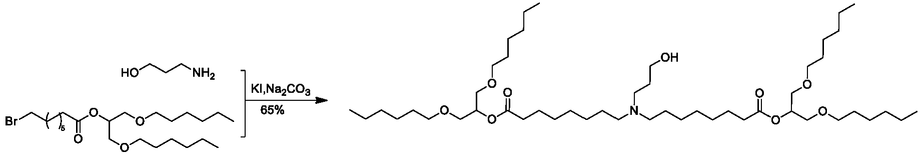

A19 was synthesized employing reaction steps similar to Example 1, 2.3 g oily product was obtained. ¹HNMR (CDCl₃) δ: ppm.5.06(t, 2H), 3.72(t, 2H), 3.50-3.33(m, 16H), 2.27(t, 2H), 2.25-2.24(m, 8H), 1.56-1.19(m, 52H), 0.88(m, 12H), MS(ES): m/z (M+H)⁺ 843.72.

### Example 15 - Synthesis of A20

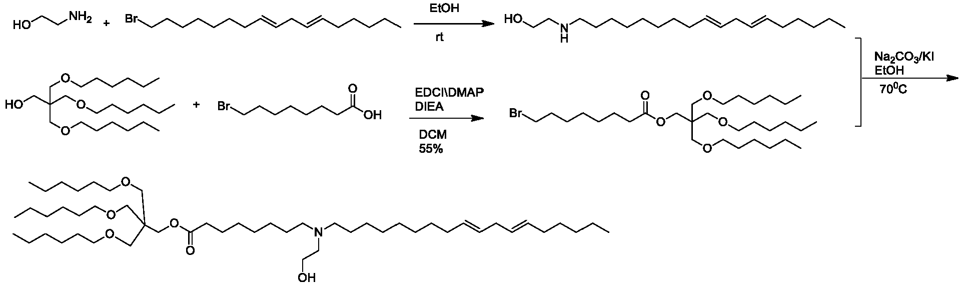

A20 was synthesized employing reaction steps similar to Example 1, 0.95 g oily product was obtained. MS (ES): m/z (MH⁺) 821.75; ¹H-NMR (400 MHz, CDCl₃) δ: ppm 5.29 (m, 4H), 4.03 (s, 2H), 3.51(t, 2H), 3.30-3.27(m, 12H), 2.70 (m, 2H), 2.57(t, 2H), 2.46 (br, 4H), 2.21(m, 2H), 1.30 -1.19(br. m, 52H), 0.89 (m, 12H).

### Example 16 - Synthesis of A21

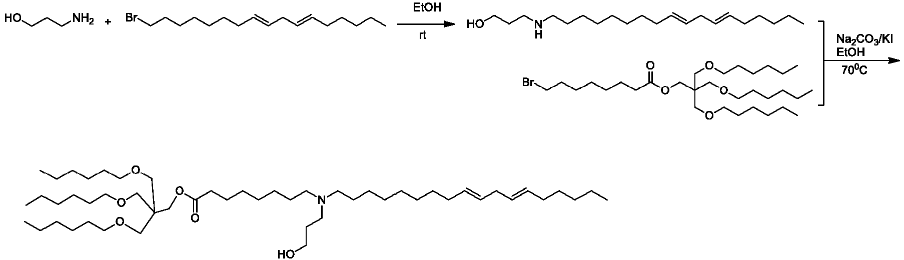

A21 was synthesized employing reaction steps similar to Example 1, 0.68 g oily product was obtained. MS (ES): m/z (MH⁺) 835.76; ¹H-NMR (400 MHz, CDC1₃) δ: ppm 5.35 (m, 4H), 4.10 (s, 2H), 3.79 (t, 2H), 3.30 (m, 12H), 2.76
(br, m, 4H), 2.50(br, 4H), 2.28 (m, 2H), 2.05(m, 4H), 1.61(br, 4H), 1.57(m, 4H), 1.54 - 1.24(br. m, 54H), 0.88(m, 12H).

### Example 17 - Synthesis of A22

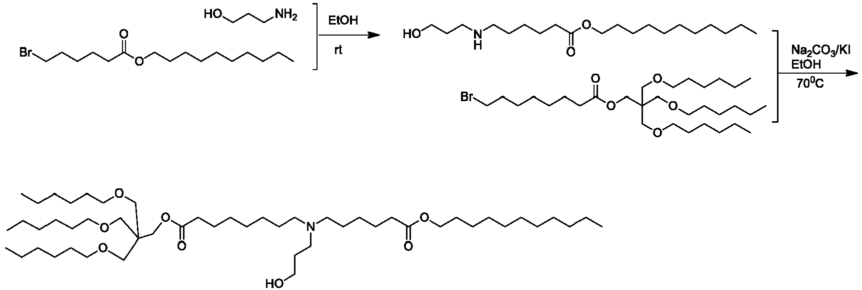

A22 was synthesized employing reaction steps similar to Example 1, 0.43 g oily product was obtained. ¹HNMR (400 MHz, CDCl₃)δ : ppm.4.03(s, 2H), 3.98(t, 2H), 3.72(t, 2H), 3.28(m, 12H), 2.65(t, 2H), 2.45(t, 4H), 2.25-2.20(m, 4H), 1.66-1.19(m, 60H), 0.83(m, 12H), MS(ES): m/z (M+H)⁺ 855.75.

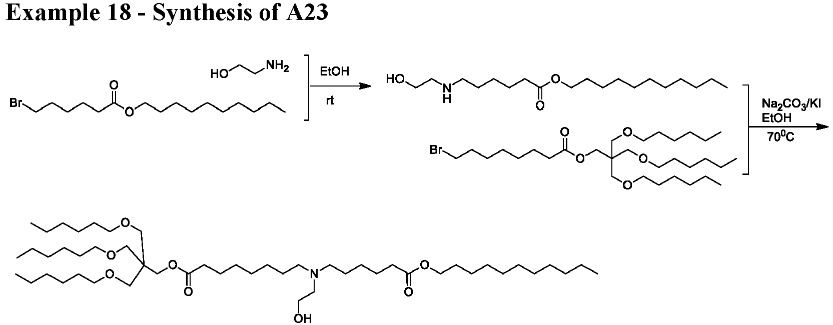

A23 was synthesized employing reaction steps similar to Example 1, 1.8 g oily product was obtained. ¹HNMR (400 MHz, CDCl₃) δ: ppm.4.10(s, 2H), 4.06(t, 2H), 3.56(t, 2H), 3.36-3.34(m, 12H), 2.61(t, 2H), 2.49(t, 4H), 2.30(m, 4H), 1.67-1.26(m, 58H), 0.88(m, 12H), MS(ES): m/z (M+H)⁺841.74.

### Example 19 - Synthesis of A24

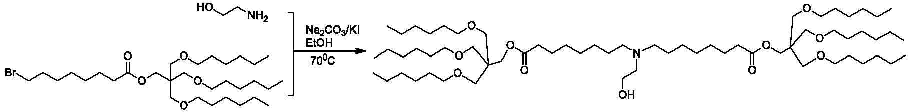

A24 was synthesized employing reaction steps similar to Example 1, 0.72 g oily product was obtained. ¹HNMR (400 MHz, CDCl₃) δ: ppm.4.10(s, 4H), 3.67(br, 2H), 3.35(m, 24H), 2.80-2.50(br, 6H), 2.28(m, 4H), 1.67-1.23(m, 68H), 0.89(m, 18H), MS(ES) : m/z (M+H)⁺ 1085.94.

### Example 20 - Synthesis of A25

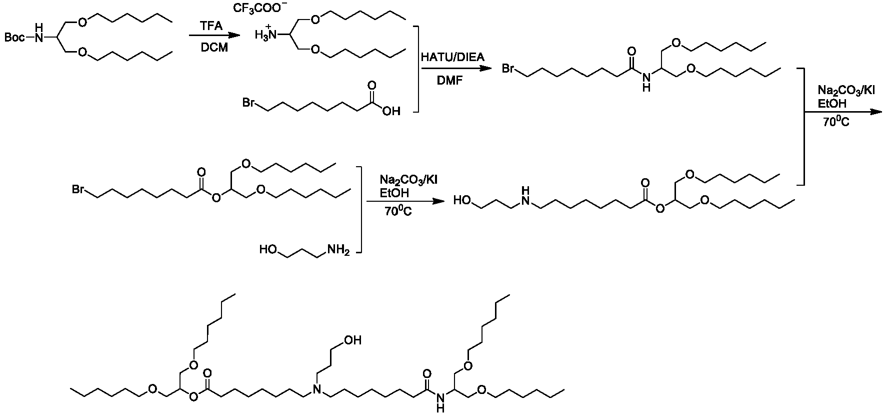

A25 was synthesized employing reaction steps similar to Example 1, 0.3 g oily product was obtained. ¹HNMR (400 MHz, CDCl₃) δ: ppm. 5.23(m, 1H), 5.05(t, 1H), 4.11(br, 4H), 3.78(t, 2H), 3.49-3.34(br, m, 16H), 3.15(t, 2H), 3.01(t, 4H), 2.26(m, 2H), 2.10(m, 2H), 2.01(m, 2H), 1.79-1.18(br, m, 52H), 0.83(br, m, 12H), MS(ES): m/z (M+H)⁺ 842.73.

### Example 21 - Synthesis of A26

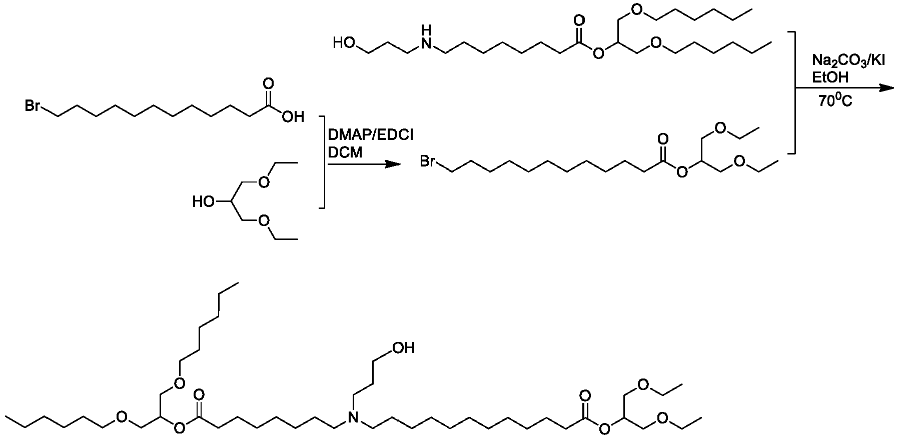

A26 was synthesized employing reaction steps similar to Example 1, 0.46 g oily product was obtained. ¹HNMR (400 MHz, CDCl₃) δ:ppm. 5.03(m, 2H), 3.75(t, 2H), 3.48-3.34(br, m, 16H), 2.91(br, 2H), 2.72(br, 4H), 2.27(t, 4H), 1.85(m, 2H), 1.58-1.10(br, m, 48H), 0.81(br, m, 6H), MS(ES): m/z (M+H)⁺ 787.65.

### Example 22 - Synthesis of A27

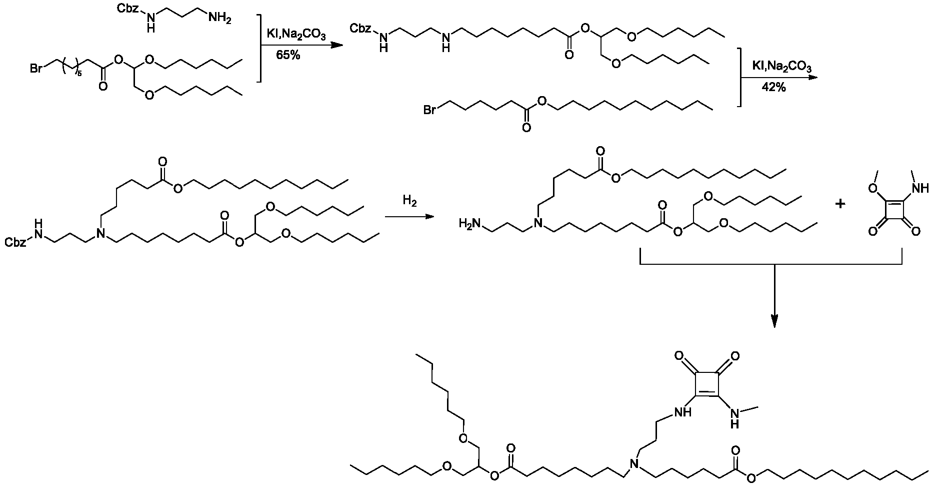

Cbz-1, 3- propylenediamineoctanoate was synthesized employing reaction steps similar to Example 1.Cbz-1, 3- propylenediamineoctanoate (3.5g, 5.9mmol), sodium carbonate anhydrous (0.94g, 8.8mmol), KI (0.19g, 1.18mmol) were soluted in 30 mL absolute ethanol and 30 mL absolute acetonitrile, then bromide was added and reacted together at 75 °C for 24 h. After the reaction was completed, the solvent was removed by concentrating, the reaction was diluted with 200 mL dichloromethane, washed with 200 mL water, extracted, the organic layer was dried over, concentrated, and passed through a silica gel column for purification (DCM:MeOH=3%--10%), oily Cbz-amine product was obtained. Cbz-amine (2.1g, 2.43mmol) was soluted in 20 mL absolute methanol and 20 mL ethyl acetate, then palladium (0.35g, 10%) was added, after hydrogen was replaced for three times, hydrogenation occurred at room temperature for 20 h. After the reaction was completed, the palladium was removed by filtration, the solvent was concentrated and removed, extracted and the amine product was obtained. The obtained amine product (1.1g, 1.51mmol) was soluted in 20 mL absolute ethanol, Ketone-methylamine (0.22g, 1.51mmol) was added, solution was stirred and reacted at room temperature for 20 h. After the reaction was completed, the solvent was concentrated and removed, the filtrate was dried over, concentrated and passed through a silica gel column for purification(DCM:MeOH=3%--10%), A27 was obtained(0.6 g oily product). ¹HNMR (d-DMSO) δ:ppm. 4.99(p, 1H), 3.98(t, 2H), 3.50-3.31(m, 8H), 3.10(d, 3H),
2.49(dt, 8H), 2.26(m, 4H), 1.52(dd, 6H), 1.43(m, 6H), 1.26(m, 40H), 0.84(m, 9H), MS(E S):m/z (M+H)⁺ 835.66.

### Example 23 - Synthesis of A28

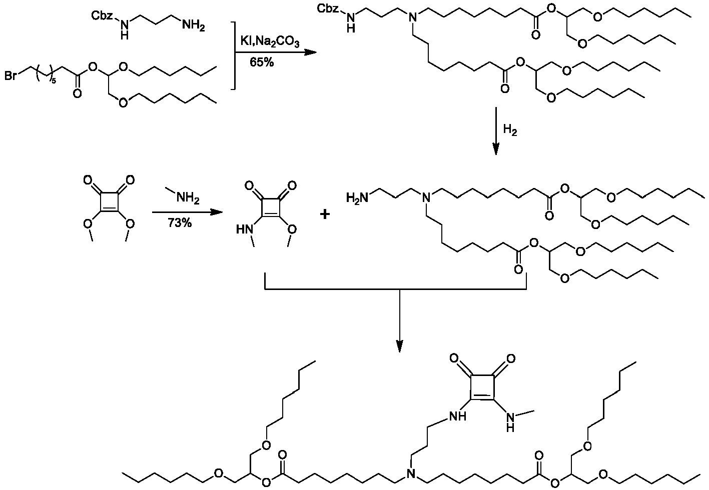

A28 was synthesized employing reaction steps similar to Example 22, 1.3 g oily product was obtained. ¹HNMR (CDCl₃) δ: ppm. 5.04(t, 2H), 3.61(t, 2H), 3.50-3.31(m, 16H), 3.21(s, 3H), 2.71(t, 2H), 2.55(t, 4H), 2.28-2.24(m, 4H), 1.86-1.19(m, 54H), 0.82(m, 12H), MS(ES): m/z (M+H)⁺951.75.

### Example 24 - Synthesis of A29

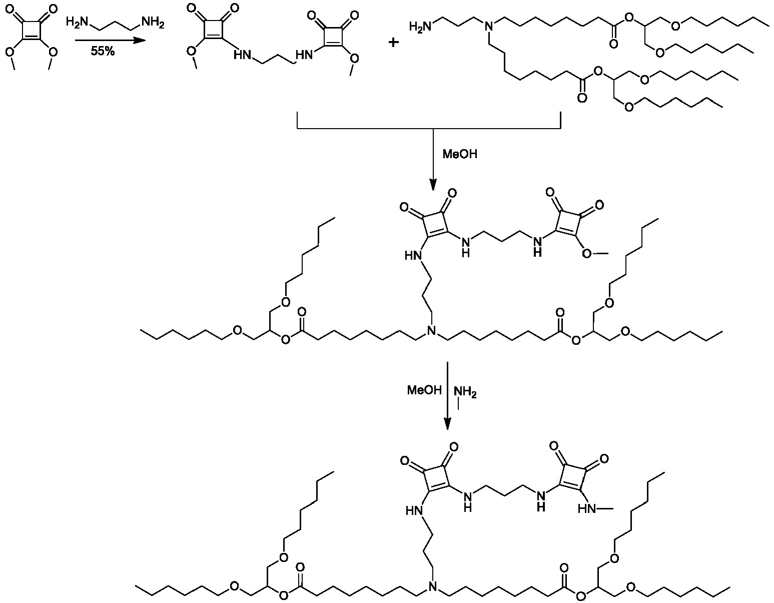

A29 was synthesized employing reaction steps similar to Example 22, 0.11 g oily product was obtained. ¹HNMR (d-DMSO) δ:ppm. 5.00(m, 2H), 3.60-3.30(m, 16H), 3.11(s, 3H), 2.63-2.49(m, 10H), 2.36(m, 2H), 2.26(m, 4H), 1.80 (m, 2H), 1.46-1.26(m, 54H), 0.85(t, 12H), MS(ES):m/z (M+H)⁺ 1103.81.

### Example 25 - Synthesis of A30

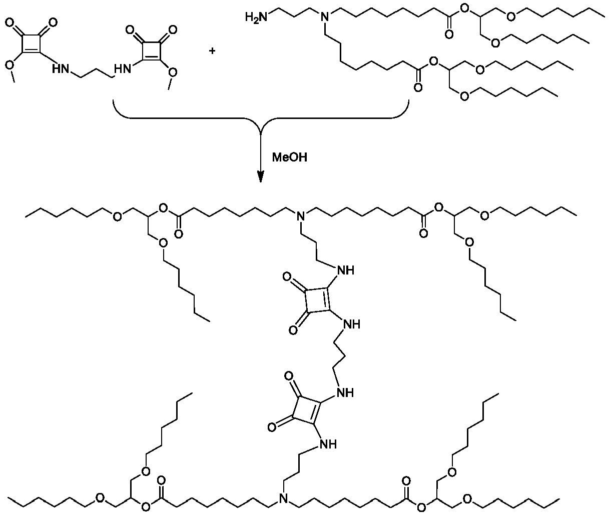

A30 was synthesized employing reaction steps similar to Example 22, 0.34 g oily product was obtained. ¹HNMR (d-DMSO) δ:ppm. 4.99(m, 4H), 3.60-3.30(m, 32H), 2.63-2.40(m, 20H), 2.25(m, 8H), 1.80-1.20(m, 110H), 0.81(m, 24H), MS(ES):m/z (M+H)⁺ 1915.5.

### Example 26 - Synthesis of A31

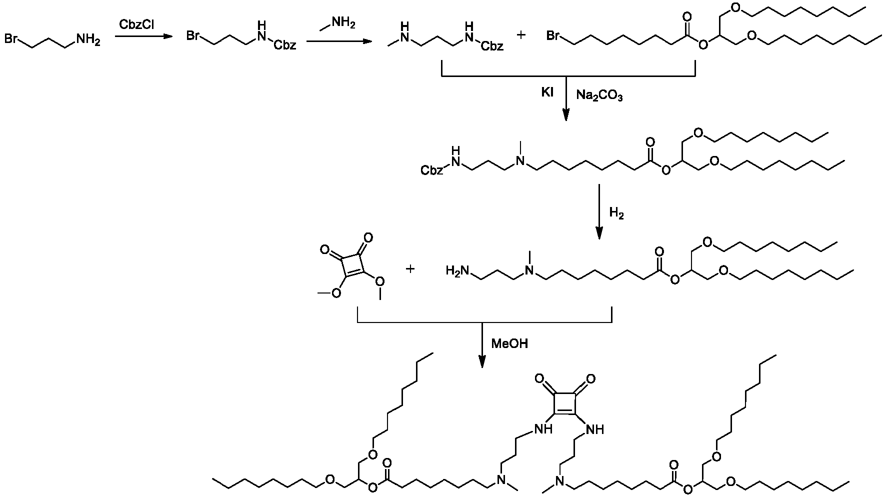

A31 was synthesized employing reaction steps similar to Example 22, 0.7 g oily product was obtained. ¹HNMR (d-DMSO) δ: ppm.5.05(m, 2H), 3.60-3.30(m, 20H), 2.63-2.40(m, 12H), 2.2(m, 6H), 1.80-1.20(m, 64H), 0.85(m, 12H), MS(ES): m/z (M+H)⁺ 1134.95.

### Example 27 - Synthesis of A32

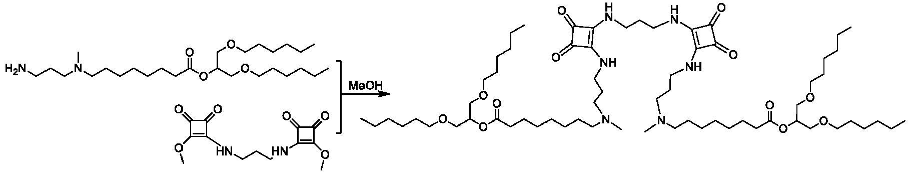

A32 was synthesized employing reaction steps similar to Example 22, 1.5 g oily product was obtained. ¹HNMR (d-DMSO) δ: ppm.5.1(m, 2H), 3.60-3.30(m, 24H), 2.5(m, 4H), 2.4(m, 4H), 2.3(m, 4H), 2.2(m, 6H), 1.95(m, 2H), 1.8(m, 2H), 1.5-1.6(m, 8H), 1.2-1.4(m, 48H), 0.9(m, 8H), MS(ES): m/z (M+H)⁺ 1174.88.

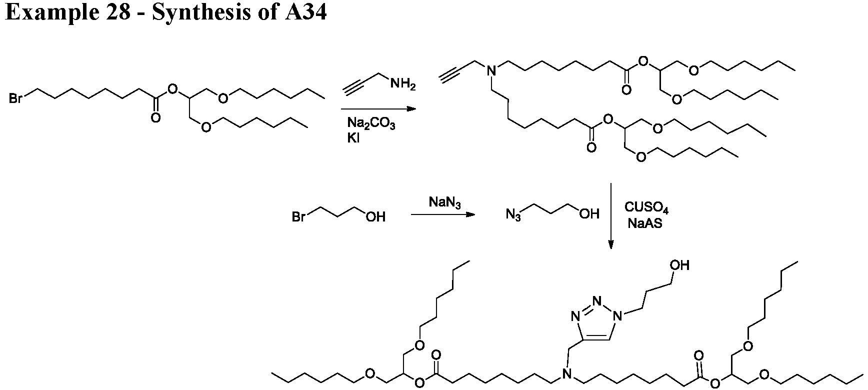

Alkynyl lipid intermediate was synthesized employing reaction steps similar to Example 1. Bromooxy ether ester (11 g), sodium carbonate (2.5 g), KI (0.4 g) were dissolved in 50 mL acetonitrile, alkynamine (0.65 g) was added. After the reaction was completed, it was concentrated to remove acetonitrile, stired and extracted with 150 mL of ethyl acetate and water, the organic layer was dried over, concentrated, and passed through a silica gel column for purification (PE:EA=10:1-5:1), for obtaining the alkynyl lipid intermediate.

Steps to prepare A34: 3-azidopropanol-1 compound (0.5 g), anhydrous copper sulfate (0.15 g), sodium ascorbate (0.24 g) and alkynyl compound (0.08 g) were dissolved in 10 mL THF and 10 mL water, after the reaction occurred at room temperature, it was concentrated to remove THF, diluted with 100 mL dichloromethane, filtered to remove unsolved material, filtrate was stired and extracted with water, the organic layer was dried over, concentrated, and passed through a silica gel column for purification (DCM: MeOH=1%-2%), 0.39 g A34 was obtained. ¹HNMR (CDCl₃) δ:ppm. 7.56(s, 1H), 5.12(p, 2H), 4.50(m, 2H), 3.77(s, 2H), 3.62-3.43(m, 18H), 2.44(s, 4H), 2.32(t, 4H), 2.13(tt, 2H), 1.70-1.50 (m, 16H), 1.26(m, 36H), 0.87(m, 12H); MS(ES): m/z (M+H)⁺925.37.

### Example 29 - Synthesis of A35

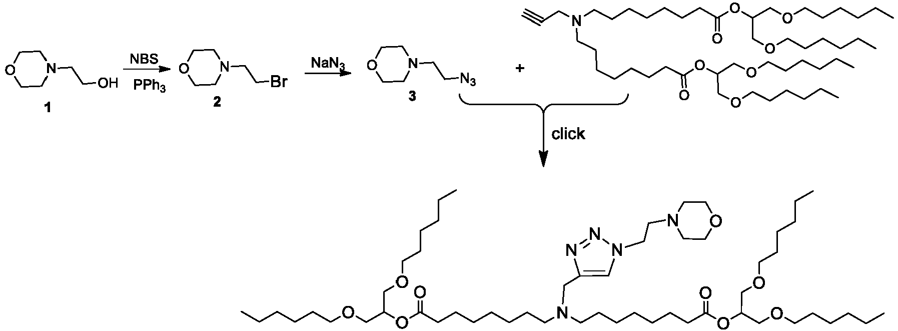

A35 was synthesized employing reaction steps similar to Example 28, 2.2 g product was obtained. ¹HNMR (CDCl₃) δ:ppm. 7.50(d, 1H), 5.11(p, 2H), 4.45(t, 2H), 3.77(s, 2H), 3.68-3.43(m, 20H), 2.82(t, 2H), 2.49(m, 4H), 2.41(s, 4H), 2.32(t, 4H), 1.70-1.50 (m, 20H), 1.26(m, 32H), 0.88(m, 12H); MS(ES): m/z (M+H)⁺980.45.

### Example 30 - Synthesis of A36

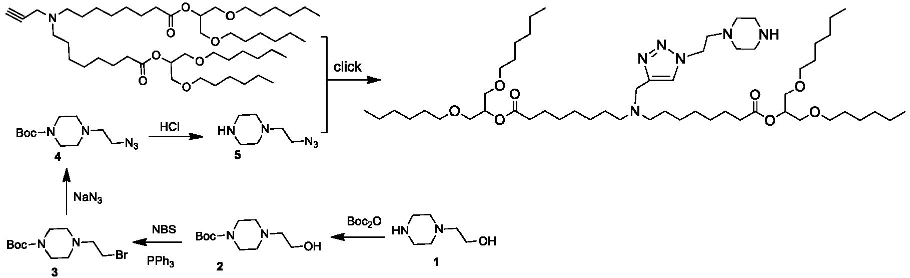

A36 was synthesized employing reaction steps similar to Example 28, 2.4 g product was obtained. ¹H NMR (500 MHz, DMSO) δ: ppm 7.86 (s, 1H), 5.00 (p, J = 5.2 Hz, 2H), 4.41 (t, J = 6.3 Hz, 2H), 3.62 (s, 2H), 3.55 - 3.41 (m, 10H), 3.42 - 3.35 (m, 8H), 2.68 (d, J = 5.9 Hz, 4H), 2.36 (s, 4H), 2.32 - 2.27 (m, 4H), 2.25 (t, J = 7.3 Hz, 4H), 1.55 - 1.48 (m, 4H), 1.46 - 1.43 (m, 6H), 1.41 - 1.36 (m, 4H), 1.25 (dd, J = 16.2, 4.5 Hz, 38H), 1.10 (s, 1H), 0.84 (t, J = 6.9 Hz, 12H)_{∘} MS(ES):m/z (M+H)⁺ 979.47.

### Example 31 - Synthesis of A37

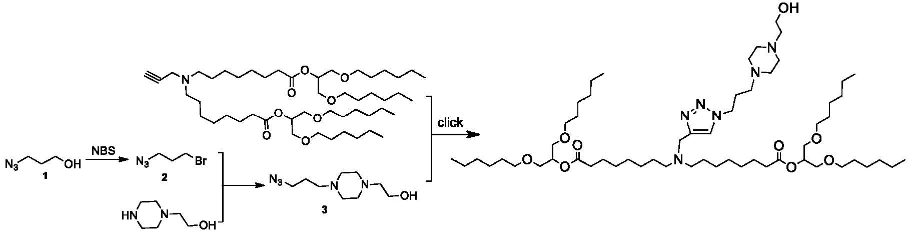

A37 was synthesized employing reaction steps similar to Example 28, 1.7 g product was obtained. ¹HNMR (CDCl₃) δ: ppm. 7.59(d, 1H), 5.10(p, 2H), 4.46(t, 2H), 3.75(s, 2H), 3.75-3.43(m, 20H), 2.94-2.87(t, 4H), 2.69-2.43(m, 4H), 2.41(s, 4H), 2.33(t, 4H), 1.70-1.50 (m, 22H), 1.26(m, 32H), 0.88(m, 12H); MS(ES): m/z (M+H)⁺ 1037.54.

### Example 32 - Synthesis of A38

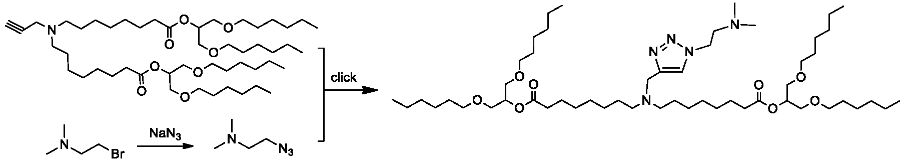

A38 was synthesized employing reaction steps similar to Example 28, 2.5 g product was obtained. ¹HNMR (CDCl₃) δ:ppm. 7.53(d, 1H), 5.11(p, 2H), 4.42(t, 2H), 3.76(s, 2H), 3.68-3.41(m, 20H), 2.83(t, 2H), 2.49(s, 6H), 2.32(t, 4H), 1.70-1.50 (m, 20H), 1.26(m, 32H), 0.87(m, 12H); MS(ES): m/z (M+H)⁺953.45.

### Example 33 - Synthesis of A39

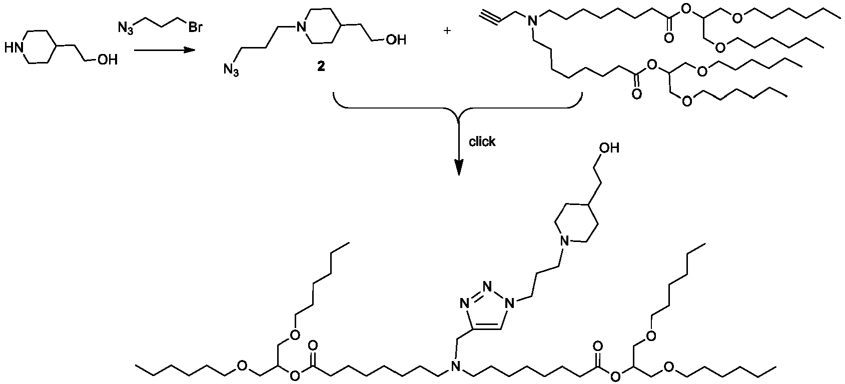

A39 was synthesized employing reaction steps similar to Example 28, 1.7 g product was obtained. ¹HNMR (CDCl₃) δ:ppm. 7.59(d, 1H), 5.11(p, 2H), 4.47(t, 2H), 3.73(s, 2H), 3.70-3.41(m, 24H), 2.78(t, 2H), 2.51(4, 4H), 2.42(m, 2H), 2.32(t, 4H), 1.59-1.25 (m, 59H), 0.87(m, 12H); MS(ES): m/z (M+H)⁺ 1036.56.

### Example 34 - Synthesis of A40

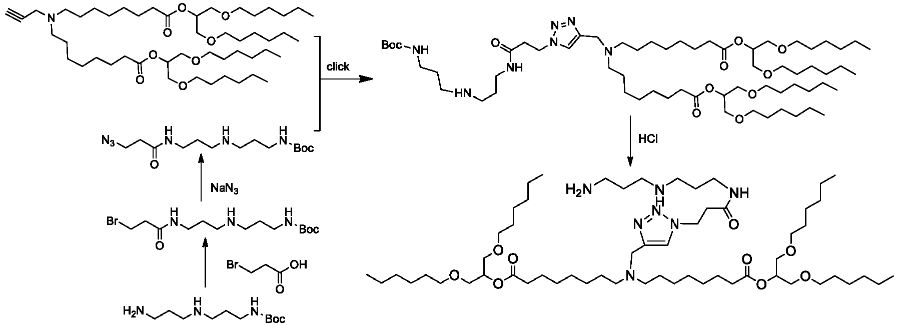

A40 was synthesized employing reaction steps similar to Example 28, 1.4 g product was obtained. ¹HNMR (CDCl₃) δ: ppm. 7.53(d, 1H), 5.11(p, 2H), 4.47(t, 2H), 3.72(s, 2H), 3.70-3.40(m, 22H), 2.66(t, 2H), 2.65-2.55(m, 6H), 2.32(t, 4H), 1.59-1.25 (m, 44H), 0.87(m, 12H); MS(ES): m/z (M+H)⁺ 1052.54.

### Example 35 - Synthesis of A42

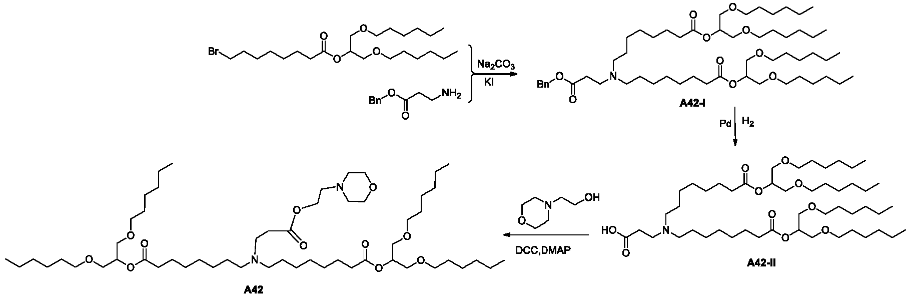

Alkynyl lipid intermediate was synthesized employing reaction steps similar to Example 1. Bromooxy ether ester (10.6 g), sodium carbonate (2.42 g), KI (0.4 g) were dissolved in 50 mL acetonitrile, benzyl-alanine (2.04 g) was added, after reflux reaction was completed, it was concentrated to remove acetonitrile, stirred and extracted with ethyl acetate and water, the organic layer was dried over, concentrated, and passed through a silica gel column for purification (DCM:MeOH=2%-3%), 7.5 g A42-I was obtained.

A42-I intermediate (2 g), palladium on carbon (0.5 g) were dissolved in 50 mL methanol, after the hydrogenation room temperature reaction was completed, it was filtered to remove the palladium on carbon, the filtrate was dried over, concentrated to obtain 1.7 g A42-II.

Steps to prepare A42: A42-II intermediate (1.5 g), DCC (0.54 g), DMAP (0.21 g) were dissolved in 50 mL dichloromethane, morpholine ethanol (0.23 g) was added, after room temperature reaction was completed, it was stirred and extracted with dichloromethane and water, the organic layer was dried over, concentrated, and passed through a silica gel column for purification (DCM:MeOH=1%-3%), 1.1 g A42 was obtained. ¹HNMR (CDCl₃) δ: ppm. 5.12(p, 2H), 4.41(t, 2H), 3.76(t, 2H), 3.55-3.40(m, 20H), 3.01-2.76(m, 10H), 2.49(t, 6H), 2.32(t, 4H), 1.59-1.25 (m, 52H), 0.87(m, 12H); MS(ES): m/z (M+H)⁺ 971.44.

### Example 36 - Synthesis of A43

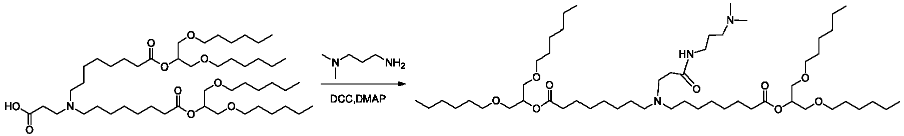

A43 was synthesized employing reaction steps similar to Example 35, 0.5 g product was obtained. ¹HNMR (CDCl₃) δ: ppm. 5.12(p, 2H), 4.41(t, 2H), 3.76(t, 2H), 3.55-3.40(m, 20H), 3.01-2.76(m, 10H), 2.49(t, 2H), 2.32(t, 4H), 1.59-1.25 (m, 54H), 0.87(m, 12H); MS(ES): m/z (M+H)⁺942.44.

### Example 37 - Synthesis of A44

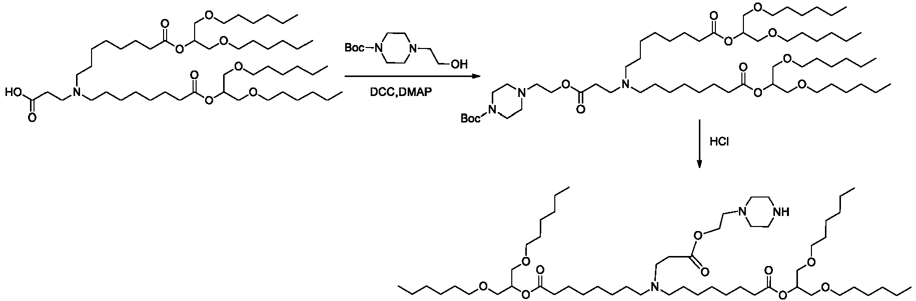

Boc protected piperazine ethanol ester intermediate was synthesized referred to Example 35.

Steps to prepare A44: Boc protected piperazine ethanol ester intermediate (1.1 g) was dissolved in 200 mL 2mol/L hydrogen ethanol, after the room temperature reaction was completed, it was dried over, concentrated to obtain 0.8 g A44. ¹HNMR (CDCl₃) δ:ppm. 5.10(p, 2H), 4.35(t, 2H), 3.60-3.40(m, 18H), 3.01-2.85(m, 6H), 2.65-2.50(t, 10H), 2.32(t, 4H), 1.59-1.25 (m, 52H), 0.87(m, 12H); MS(ES): m/z (M+H)⁺ 970.45.

### Example 38 - Synthesis of A45

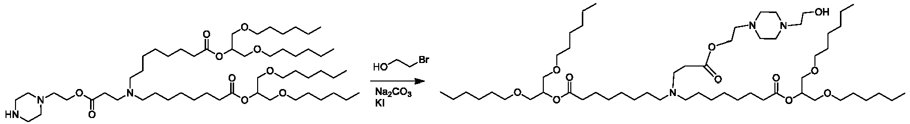

A44 was synthesized employing reaction steps similar to Example 37.

Steps to prepare A45: A44 (1.6 g), sodium carbonate (0.17 g), KI (0.054 g) were dissolved in 50 mL acetonitrile, bromoethanol (0.2 g) was added, after reflux reaction was completed, it was concentrated to remove acetonitrile, stirred and extraced with ethyl acetate and water, the organic layer was dried over, concentrated, and passed through a silica gel column for purification (DCM:MeOH=3%-5%), 1.1 g A45 was obtainted. ¹HNMR (CDCl₃) δ:ppm. 5.10(p, 2H), 4.35(t, 2H), 3.60-3.40(m, 18H), 3.32(t,2H), 3.01-2.90(m, 6H), 2.53(t, 2H), 2.49-2.35(t, 10H), 2.32(t, 4H), 1.59-1.25 (m, 52H), 0.87(m, 12H); MS(ES): m/z (M+H)⁺ 1014.50.

### Example 39 - Synthesis of A46

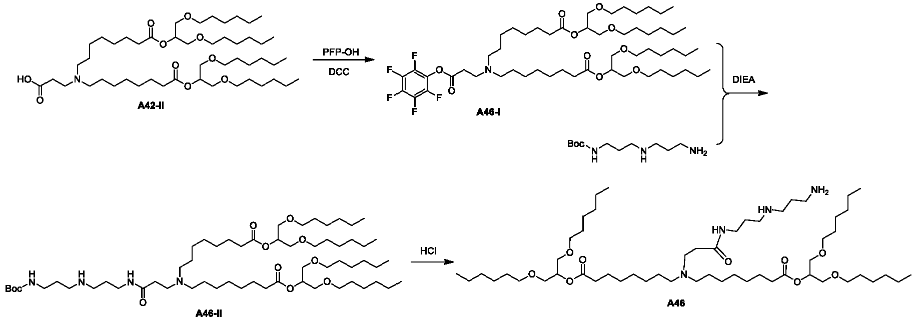

A42-II was synthesized employing reaction steps similar to Example 35.

Steps to prepare A46-I: A42-II (1.5 g), DCC (0.39 g), PFP-OH (0.35 g) were dissolved in 50 mL dichlorormethane, after the room temperature reaction was completed, it was concentrated to remove DCM, diluted with ethyl acetate, filtered to remove white undissolved material, stirred and extracted with 10% sodium carbonate solution, the organic layer was dried over, concentrated, and passed through a silica gel column for purification (DCM:MeOH=1%-3%), 1.2g A46-I was obtained.

Steps to prepare A46-II: mono Boc-diaminodipropylamine (0.23 g), DIEA (0.19 g), A46-I (1 g) were dissolved in 20 mL dichloromethane, after room temperature reaction was completed, dichloromethane and sodium carbonate solution were added to stir and extract, the organic layer was dried over, concentrated, and passed through a silica gel column for purification (DCM:MeOH=1%-3%), 0.8g A46-II was obtained.

Steps to prepare A46: A46 was synthesized referred to steps to prepare A44 in Example 37. ¹HNMR (CDCl₃) δ: ppm. 5.10(p, 2H), 3.60-3.40(m, 18H), 3.37(t, 2H), 2.70-2.55(m, 10H), .2.50(t, 2H), 2.32(t, 4H), 1.72 (m, 4H), 1.59-1.25 (m, 52H), 0.87(m, 12H); MS(ES): m/z (M+H)⁺ 971.48.

### Example 40 - Synthesis of A47

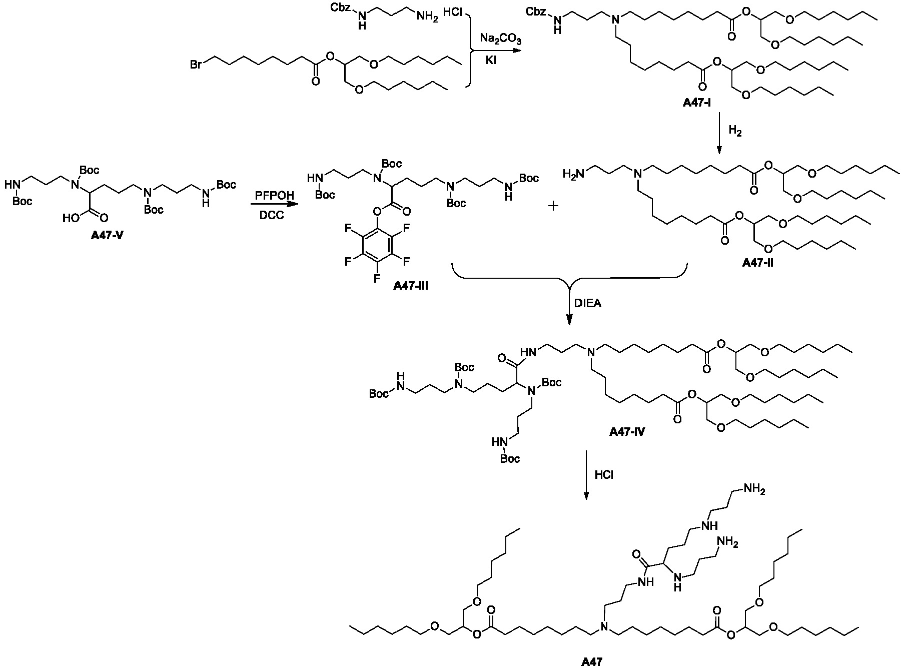

Steps to prepare A47-I: A47-I was synthesized according to steps used to prepare A42-I in Example 35.

Steps to prepare A47-II: A47- II was synthesized according to steps used to prepare A42- II in Example 35.

Steps to prepare A47-III: A47-III was synthesized according to steps used to prepare A46-I in Example 39.

Steps to prepare A47-IV: A47- IV was synthesized according to steps used to prepare A46- II in Example 39.

Steps to prepare A47: 1.1 g A47 was synthesized according to steps used to prepare A44 in Example 37. ¹HNMR (400 MHz, CD₃OD) δ: ppm.5.10(m, 2H), 3.53(m, 8H), 3.44(m, 9H), 3.20-3.0 (m, 16H), 2.34(t, 4H), 2.09(m, 4H), 1.98(dt, 4H), 1.84 (dd, 4H), 1.71(s, 4H), 1.63(dd, 4H), 1.53(m, 8H), 1.40-1.20(m, 40H), 0.88(m, 12H); MS(ES): m/z (M+H)⁺1071.65.

### Example 41 - Synthesis of A48

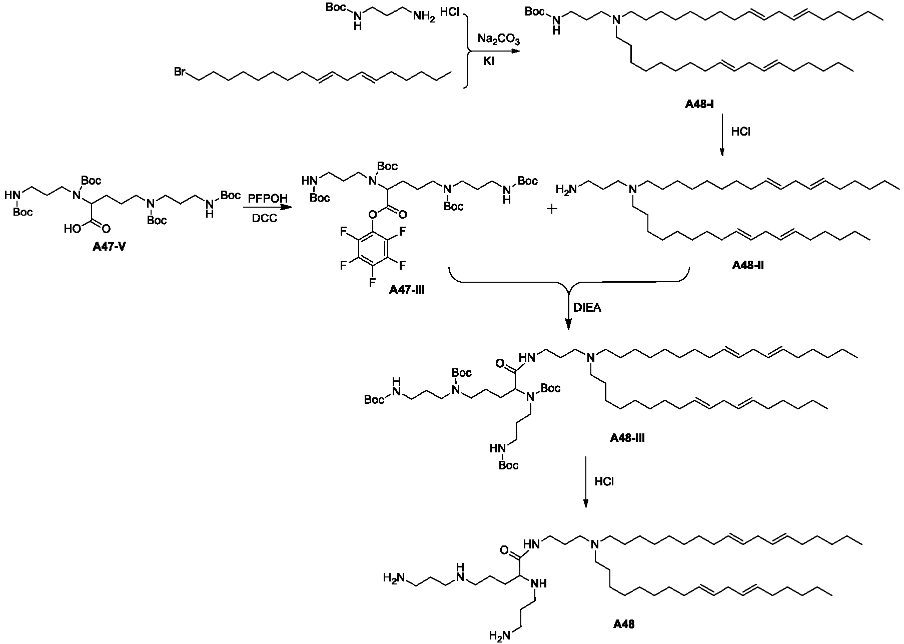

Steps to prepare A48-I: A48-I was synthesized according to steps used to prepare A42-I in Example 35.

Steps to prepare A48-II: A48- II was synthesized according to steps used to prepare A42- II in Example 35.

Steps to prepare A48-III: A48-III was synthesized according to steps used to prepare A46- II in Example 39.

Steps to prepare A48: 0.5 g A48 was synthesized according to steps used to prepare A44 in Example 37. ¹HNMR (400 MHz, CD₃OD) δ: ppm.7.78(m, 7H), 5.08(m, 8H), 3.87(m, 1H), 3.0-2.91 (m, 2H), 2.71 (m, 4H), 2.50 (m, 12H), 1.84(dd, 4H), 1.71(m, 4H), 1.63-1.53(m, 12H),1.40-1.20(m, 36H), 0.88(m, 6H); MS(ES): m/z (M+H)⁺ 799.35

### Comparative Examples - MC3, A2, A3, A4, A8 and A33

MC3, A2, A3, A4, A8 and A33 are used for comparative example. Because MC3, A2, A3, A4, A8 and A33 are known cationic liposomes, their synthesis methods are not described specifically here. MC3, A2, A3, A4, A8 and A33 are shown as follows:

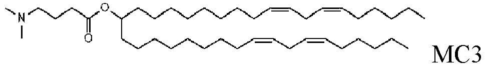

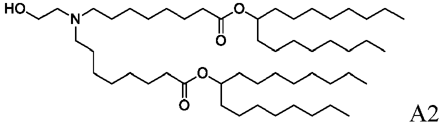

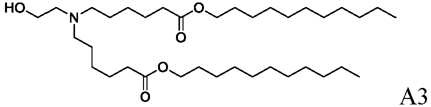

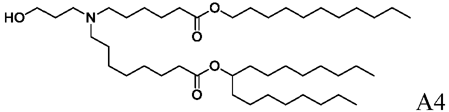

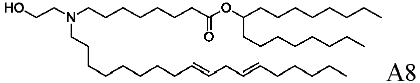

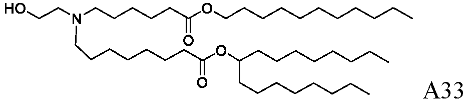

### Example 42 - Production of lipid nanoparticles

Lipid nanoparticles were prepared using the following ingredients: (1) an ionizable lipid compound which were commercially available or synthesized, e.g., MC3 (purchased from Avanti), A1-A33 were synthesized in-house; (2) phospholipid (e.g., DOPE or DSPC, purchased from Avanti); (3) PEG lipid (e.g., PEG-DMG, purchased from Avanti or synthesized in-house); (4) structural lipid (e.g., cholesterol, purchased from Sigma-Aldrich); (5) effective composition/active ingredient (e.g., Luciferase mRNA, siRNA, SARS-CoV-2 S protein mRNA, Cas 9 mRNA, etc.)

Preparation and encapsulation method: (1) ionizable lipid, phospholipid, pegylated lipid and structural lipid were dissolved and mixed in ethanol at 50%, 10%, 1.5%, and 38.5% successively and respectively; (2) a microfluidic chip or T-type mixer were employed to mix lipid mixture and active ingredient (mRNA) evenly at a ratio of 1:3 to obtain lipid nanoparticles.

Encapsulation percentage reflects the encapsulation extent of the encapsulated substance. The higher the encapsulation percentage, the decomposition possibility of the encapsulated substance is less during the delivery in vivo.

**Table 1. Performance of ionizable lipid and its lipid nanoparticle**

| Ionizable lipid | Size (nm) | PDI | Encapsulated percentage (%) |
|---|---|---|---|
| A1 | 127.7 | 0.090 | 88.79 |
| A2 | 145.5 | 0.100 | 87.86 |
| A3 | 140.6 | 0.120 | 81.47 |
| A4 | 131.1 | 0.110 | 79.24 |
| A5 | 135.0 | 0.090 | 90.11 |
| A6 | 171.5 | 0.190 | 87.09 |
| A7 | 105.1 | 0.175 | 94.63 |
| A8 | 142.2 | 0.120 | 76.10 |
| A9 | 129.4 | 0.120 | 93.35 |
| A10 | 121.6 | 0.104 | 82.65 |
| A12 | 119.3 | 0.083 | 86.53 |
| A13 | 96.87 | 0.146 | 95.51 |
| A17 | 109.5 | 0.1037 | 92.99 |
| A18 | 104.8 | 0.0835 | 93.58 |
| A19 | 110.1 | 0.0923 | 88.00 |
| A20 | 77.17 | 0.121 | 97.09 |
| A21 | 78.3 | 0.098 | 97.20 |
| A22 | 86.19 | 0.049 | 96.85 |
| A23 | 74.63 | 0.054 | 97.41 |
| A24 | 78.69 | 0.78 | 96.77 |
| A25 | 105.3 | 0.094 | 83.60 |
| A26 | 120.3 | 0.064 | 83.56 |
| A27 | 94.16 | 0.13 | 96.69 |
| A28 | 91.19 | 0.13 | 89.72 |
| A29 | 126.4 | 0.05 | 89.98 |
| A30 | 110.1 | 0.28 | 98.14 |
| A32 | 128.7 | 0.07 | 94.19 |
| MC3 | 86.7 | 0.10 | 92.00 |

### Example 43 - Experiments for proving transfection efficiency

Encapsulated the nanoparticle of various cationic lipid compounds and luciferase mRNA according to the method in Example 42, tested the fluorescence intensity or total number of photons of luciferase mRNA encapsulated by different LNP.

Experimental animal: SPF grade BALB/c mice, female, 6-8 week old, body weight ranged 18-22 g, were purchased from Beijing Vital River Laboratory Animal Technology Co. Ltd, with production license no.: SXCK (JING) 2016-0006. All animals were kept in adaptive feeding for more than 7 days before experiments, free to uptake food and drink water during experimental period, illumination 12/12 h alternating light and dark, room temperature was 20-26°C, humidity was 40-70%.

Experimental method: female BALB/c mice, encapsulated luciferase mRNA with different LNP by four different administrations which included subcutaneous injection (under the armpit), tail vein injection, intraperitoneal injection, intramuscular injection (the tibialis anterior muscle of mouse hind leg); 3, 6, 24, 48h after administration, applied in vivo fluorescence imaging system (brand: Bruker, model: XTREME) for bioluminescence detection, the specific steps were as follows: substrate preparation: took appropriate amount of substrate Luciferin (brand: Promega) and added saline to make a 10mg/ml solution, avoided light, injected 100µl solution into each mouse intraperitoneally. Mouse moved freely for 5-10 min after being administrated substrate, then put them into anesthesia box and used 2.5% isoflurane for anesthesia. Put the anesthetized mouse into machine, set the bioluminescence setting and took the photos, captured the photograph and then adjusted the upper and lower values of photograph, then collected data on the concentrated distribution of fluorescence (e.g., fluorescence intensity, average photons number, and total photons number) and processed data. Statistical analysis: the in vivo imaging outcome were shown in the fluorescence intensity or average of total photons number of different animal in the same tested group, which were used to judge whether the fluorescence intensity or total photon number of luciferase mRNA encapsulated by different LNP were high or low.

The fluorescence intensity and total photons number reflects the transfection efficiency of LNP, the value is higher, referring to the efficiency of delivering encapsulated substance into cell by LNP is higher.

**Table 2. The expression of induced luciferase of A1-A9 ionizable lipid nanoparticle**

| Cationic lipid | Average fluorescence intensity | | | | | |
|---|---|---|---|---|---|---|
| | 3 h | | 6 h | | 24 h | |
| | Leg (intramuscular injection) | Liver (tail vein injection) | Leg (intramuscular injection) | Liver (tail vein injection) | Leg (intramuscular injection) | Liver (tail vein injection) |
| A1 | 61.3 | 20.4 | 80.6 | 27.80 | 38.9 | 15.9 |
| A2 | 1146 | 5325.2 | 1208.2 | 5934.7 | 377.7 | 162.6 |
| A3 | 60.3 | 12.1 | 69.8 | 11.9 | 35.9 | 13.5 |
| A4 | 1847.5 | 7962.6 | 1956.2 | 7745.9 | 1009.7 | 326.4 |
| A5 | 675.7 | 2231.2 | 1566.4 | 3738.2 | 892.2 | 73.5 |
| A6 | 60.3 | 11.9 | 40.9 | 9.0 | 19.1 | 12.2 |
| A7 | 1683.2 | 21013.1 | 2990.1 | 21315.5 | 1436.4 | 444.2 |
| A8 | 1111 | 6850.3 | 2161.8 | 10174.4 | 1294.9 | 220.8 |
| A9 | 461.4 | 198.9 | 432.0 | 252.7 | 178.3 | 14.6 |
| A33 | 1065.5 | 2841.8 | 1117.9 | 3144.6 | 418.8 | 100.0 |

| | | | | | | |
|---|---|---|---|---|---|---|
| Note: administration route: (1) intramuscular injection; (2) tail vein injection; dose: 10µg/ mice; detection time: 3, 6, and 24 h after administration. | | | | | | |

**Table 3. The expression of induced luciferase of A18-A33 ionizable lipid nanoparticle**

| Cationic lipid | Total photons number | | |
|---|---|---|---|
| | 3 h | 6 h | 24 h |
| | Leg | Leg | Leg |
| A10 | NA | 3.17E+09 | NA |
| A11 | NA | 1.33E+09 | NA |
| A12 | NA | 6.91E+08 | NA |
| A13 | NA | 3.66E+09 | NA |
| A15 | NA | 8.78E+08 | NA |
| A18 | 3.91E+09 | 4.16E+09 | 2.90E+08 |
| A19 | 3.69E+09 | 4.77E+09 | 7.33E+08 |
| A27 | 1.34E+09 | 1.04E+09 | 3.41E+08 |
| A28 | 6.50E+08 | 4.26E+08 | 1.83E+08 |
| A31 | 1.58E+08 | 2.65E+08 | 8.43E+07 |
| A33 | 1.41E+08 | 2.35E+08 | 5.84E+07 |
| MC3 | 3.43E+08 | 7.41E+08 | 2.86E+08 |

| | | | |
|---|---|---|---|
| Note: administration route: intramuscular injection; dose: 1µg/ mice; detection time: 3, 6, and 24 h after administration. | | | |

**Table 4. The expression of induced luciferase of A20-A25, A33 ionizable lipid nanoparticle**

| Cationic lipid | Total photons number | | |
|---|---|---|---|
| | 3 h | 6 h | 24 h |
| | Leg | Leg | Leg |
| A20 | 5.99E+08 | 5.65E+08 | 2.03E+08 |
| A21 | 9.07E+08 | 7.11E+08 | 1.62E+08 |
| A22 | 4.16E+09 | 5.74E+09 | 1.24E+09 |
| A23 | 4.39E+09 | 2.68E+09 | 8.00E+08 |
| A24 | 1.94E+08 | 1.56E+08 | 6.31E+07 |
| A25 | 4.23E+08 | 2.49E+08 | 9.50E+07 |
| A33 | 5.20E+08 | 4.62E+08 | 1.63E+08 |

| | | | |
|---|---|---|---|
| Note: administration route: intramuscular injection; dose: 5µg/mice; detection time: 3, 6, and 24 h after administration. | | | |

### Example 44 - Evaluate the Safety of LNP

Wistar rats including 4 males and 4 females, with a weight difference of no more than 10%, were selected and randomly divided into two groups: solvent control group and tested substance group. A18 was used in the tested substance group, and the LNP preparation's encapsulation condition and size were shown in table 5. The concentration used in the measurement was 2 mg/mL. Each animal was administered 3 times a day with the volume of each injection as 250 µl. The administration interval was 4 hours. Alternate administration to the left and right legs were performed. The total dosage level was 1.50 mg per rat, which was equivalent to 1200 times the maximum unit weight dosage level for human (assuming the maximum dosage level for human is 0.25 mg). Clinical observation was recorded as follows. Within 24 hours after administration, clinical observation was carried out every hour. Within 24-72 hours after administration, clinical observation was carried out once every 6 hours. Within 4-14 days after administration, clinical observation was carried out once a day. The symptoms of toxicity, the time when the symptoms appeared and disappeared, and the time of death (if occurred) were recorded. Body weight was recorded once a day after administration, and food intake was recorded every 2 days after administration. 14 days after the administration, all the remaining animals were weighed, then euthanized, and major organs were obtained by anatomy. Heart, liver, spleen, lung, kidney, thymus, lymph node, were weighted and relative organ weight (the organ weight / the subject weight×100%; also known as organ coefficient) was calculated. After anatomy, the heart, liver, spleen, lung, kidney, intestine, thymus, lymph node, muscle tissue at the injection site and other organs with pathological changes observed were stored in a fixation solution, and the pathology of each organ was examined by H&E staining. Lesion severity scores were given according to the table below.

**Table 5. The LNP preparation's encapsulation condition and size**

| | Ionizable lipid | Size (nm) | PDI |
|---|---|---|---|
| Solvent control group | - | - | - |
| Tested substance group (LNP empty vector control group) | A18 | 147 | 0.088 |

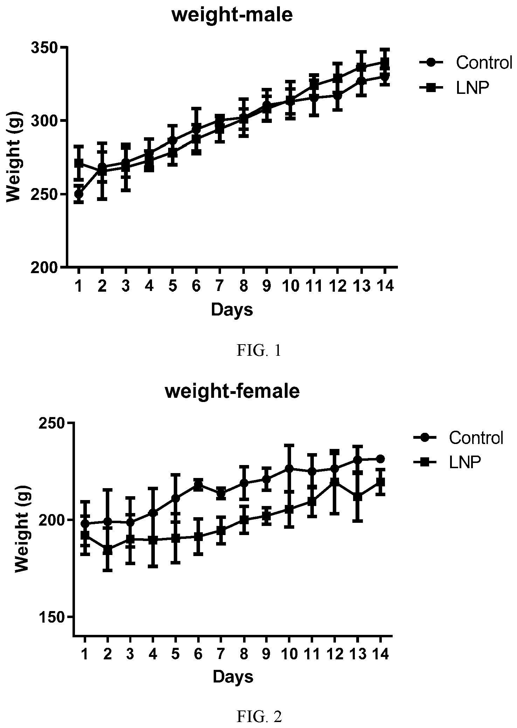

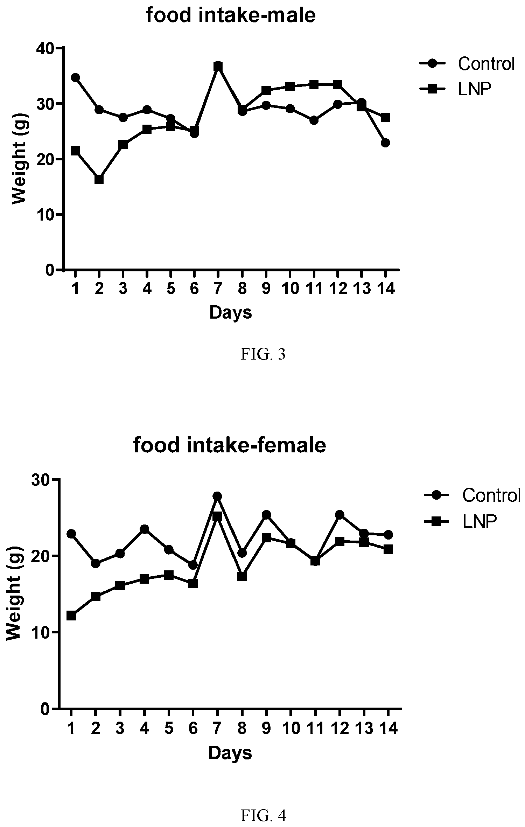

The weight change results are shown in **FIGS. 1-2****.** Specifically, the body weight of male rats in the solvent control group continued to increase. The body weight of rats in the tested substance group dropped initially, then recovered to the pre-administration level on Day 4- Day 6, and continued to increase subsequently. The results of changes in food intake are shown in **FIGS. 3-4****.** The food intake per rat in the solvent control group within 24 hours was stable, within a range of 18-35 grams. The initial food intake of the rats in the tested substance group decreased initially, and returned to normal levels on Day 4- Day 7. The relative organ weight results are shown in **Table 6.** Compared with the solvent control group, the heart, liver, kidney, spleen, thymus and lymph node coefficients of rats in the tested substance group did not show significant difference. The results of histological changes are shown in **Table 8.** Compared with the rats in the solvent control group (one male and one female), the heart, liver, kidney, spleen, thymus, and lymph nodes of the rats in the tested substance group had no pathological changes. For the tested substance group, there were no other abnormal pathological changes except for the proliferation of partial interstitial cells in the lung and a small amount of inflammatory cell infiltration in the muscle tissue. Based on the above results, the rats showed only slight pathological changes in the lungs and legs after the 1200 times higher dosage level of the LNP injection.

**Table 6. Acute toxicity test of LNP in rat (relative organ weight)**

| | | Heart | Liver | Spleen | Lung | Kidney | Kidney | Thymus | Lymph nodes | Brain |
|---|---|---|---|---|---|---|---|---|---|---|
| | | | | | | left | right | | | |
| Solvent control group | Total | 0.302± 0.004 | 4.188± 0.144 | 0.257± 0.045 | 0.376± 0.029 | 0.373±0 .010 | 0.391± 0.006 | 0.186± 0.038 | 0.005± 0.001 | 0.700± 0.127 |
| Tested substance group | Total | 0.308 ± 0.013 | 3.773 ± 0.212 | 0.250 ± 0.029 | 0.413 0.048 | 0.351 ± 0.015 | 0.356 ± 0.025 | 0.149 ± 0.024 | 0.006 ± 0.006 | 0.687 ± 0.142 |

| | | | | | | | | | | |
|---|---|---|---|---|---|---|---|---|---|---|
| Total: average relative organ weight of male, female rat. | | | | | | | | | | |

**Table 7. Lesion severity score standard**

| | |
|---|---|
| Score = 0 (does not exist) | Under the conditions of the experiment, taking into account the age, sex and strain of the animal, it can be considered that the tissue is within the normal range and there is no pathological change. |
| Score = 1 (minimal) | The first (lowest) grade of lesions in the 5 grades of minimal, mild, moderate, severe and serious. |
| Score = 2 (mild) | The second grade of lesion degree in the 5 grades of minimal, mild, moderate, severe and serious. |
| Score = 3 (moderate) | The third grade of lesion degree in the 5 grades of minimal, mild, moderate, severe and serious. |
| Score = 4 (severe) | The fourth grade of lesion degree in the 5 grades of minimal, mild, moderate, severe and serious. |
| Score = 5 (serious) | The fifth (highest) grade of lesion degree in the 5 grades of minimal, mild, moderate, severe and serious. |

**Table 8. Histopathological results of acute toxicity test of LNP in rats**

| Group | Animal number | Heart | Liver | Kidney | Spleen | Thymus | Lymph nodes | Pancreas | Lung | Muscle tissue |
|---|---|---|---|---|---|---|---|---|---|---|
| Solvent control group | 5001 | - | - | - | - | - | - | - | - | - |
| | Score | 0 | 0 | 0 | 0 | 0 | 0 | 0 | 0 | 0 |
| | 5101 | - | - | - | - | - | - | - | - | - |
| | Score | 0 | 0 | 0 | 0 | 0 | 0 | 0 | 0 | 0 |
| Tested substance group | 1001 | - | - | - | - | - | - | - | Partial stromal cell hyperplasia | - |
| | Score | 0 | 0 | 0 | 0 | 0 | 0 | 0 | 1 | 0 |
| | 1101 | - | - | - | - | - | - | - | Partial stromal cell hyperplasia | - |
| | Score | 0 | 0 | 0 | 0 | 0 | 0 | 0 | 1 | 0 |

| | | | | | | | | | | |
|---|---|---|---|---|---|---|---|---|---|---|
| Note: "-" means no abnormality. | | | | | | | | | | |

### Example 45 - Immunity study of mRNA encapsulated by LNP

SARS-CoV-2 S protein mRNA was produced by T7 in vitro transcription method, used ionizable lipid A7, A18 and A33 encapsulated lipid nanoparticle according to synthesis method in Example 42, and their encapsulation condition, encapsulated percentage and size were shown in Table 9.

**Table 9 Encapsulated outcome**

| Ionizable lipid | Encapsulated percentage (%) | Size (nm) | PI |
|---|---|---|---|
| A33 | 95 | 76 | 0.104 |
| A7 | 97 | 79 | 0.060 |
| A18 | 94 | 86 | 0.075 |

### Immunization program:

| Ionizable lipid | Group | Dose | Species | Administration | Numbers of animal | Animal number |
|---|---|---|---|---|---|---|
| A33 | 1 | 4µg | BALB/c | intramuscular | 9 | 8001-8009 |
| | 2 | 50µg | BALB/c | intramuscular | 9 | 9001-9009 |
| A7 | 1 | 4µg | BALB/c | intramuscular | 9 | 16001-16009 |
| | 2 | 50µg | BALB/c | intramuscular | 9 | 17001-17009 |
| A18 | 1 | 5µg | BALB/c | intramuscular | 9 | 3001-3009 |
| | 2 | 20µg | BALB/c | intramuscular | 9 | 4001-4009 |

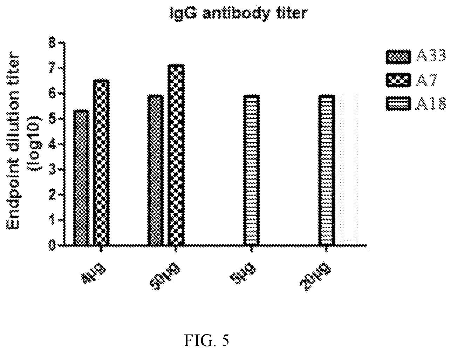

The 3 obtained LNP preparations were used in BALB/c mouse immunization test. The experiment was performed as follows. 6-8 week old female BALB/c mice (9 mice in each group) were administered twice with the LNP preparations on Day 0 and Day 14 by intramuscular injection. The injection volume was 50 µL. After 7 days, the mouse spleens were isolated to separate splenic lymphocytes. The T lymphocytes secreting INFγ were detected by the ELISPOT (Enzyme-linked immune absorbent spot) method and the outcome was shown in table 10, illustrating that mRNA induced stronger cellular immune response in BALB/c mice. 14 days after the second immunization, the S protein specific IgG antibody was detected by indirect ELISA. The IgG antibody EC50 was calculated by fitting the antibody titer curves, as shown in the figure 5. The results showed that A7, A18 and A33 were all induced higher titers of IgG antibodies in BALB/c mice

Specific operation of ELISPOT was carried according to Mouse IFN-γ precoated ELISPOT kit instruction.

**Table 10. Elispot counted T lymphocytes secreting INFγ after administrating different LNP preparations**

| | A33 | | A7 | | A18 | |
|---|---|---|---|---|---|---|
| | 4µg | 50µg | 4µg | 50µg | 5µg | 20µg |
| Blank control | 0 | 2 | 12 | 29 | 1 | 0 |
| S protein (1 µg) | 32 | 310 | 294 | 805 | 161 | 241 |
| | 42 | 338 | 811 | 700 | 153 | 251 |
| Positive control | 679 | 868 | 124 | N/A | 344 | 474 |

Specific operation of indirect ELISA to detect S protein specific IgG antibody titer:
1. coated antigen: S protein was diluted to 2 ng/uL with coating buffer, 100 uL/well, coated overnight at 4 °C;
2. washed plate 3 times with 1X PBST, washed 5 min each time;
3. blocked with 1% BSA blocking solution, 200 uL/well, and left to stand 1 h at 37 °C;
4. washed plate 3 times with 1X PBST, washed 5 min each time;
5. serum to be tested was diluted with dilution buffer by doubling dilution, 100 uL/well, incubated for 1 h at 37 °C, and set negative serum control group and blank control group without serum;
6. washed plate 3 times with 1X PBST, washed 5 min each time;
7. anti IgG secondary antibody was diluted by 1:1000, 100 uL/well, incubated for 1 h at 37 °C;
8. washed plate 3 times with 1X PBST, washed 5 min each time;
9. added fresh TMB chromogen solution, 100 uL/well, incubated for an appropriate time at 37 °C;
10. added 2 mol/L termination solution of sulfuric acid, 50 uL/well.
11. used enzyme-labeled instrument to measure OD450 nm absorbance.

### ELISA to detect antibody

### First immune 14 days antibody detection

1. sample: first immuned 14 days mouse serum of immune group, solvent control group, mixed 6 mouse's serum in each group
2. antigen protein: SARS-CoV-2 (COVID-19) S protein (R683A, R685A), His Tag (SPN-C52H4)
3. coated antigen protein: 2 ng/µL, 100 uL/well
4. second immune: Goat anti-mouse IgG (H+L), HRP conjugate 1:1000 dilution

### Second immune 14 days antibody detection

1. sample: second immuned 14 days mouse serum of immune group, solvent control group, mixed 6 mouse's serum in each group
2. antigen protein: SARS-CoV-2 (COVID-19) S protein (R683A, R685A), His Tag (SPN-C52H4)
3. coated antigen protein: 2 ng/µL,100 uL/well
4. second immune: Goat anti-mouse IgG (H+L), HRP conjugate 1:1000 dilution
5. outcome shown in Figure 5.

## Claims

1. A compound of one of formulae (II), (III), (IV), or (V): 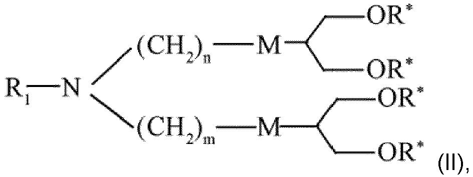 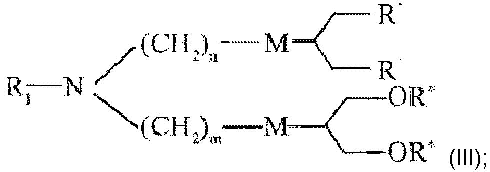 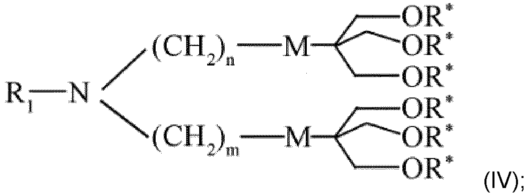 or 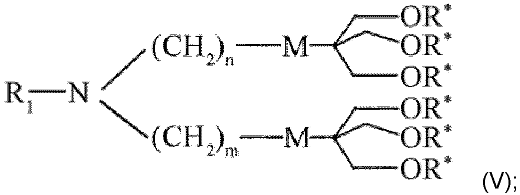
wherein R₁ is selected from -R₁'-X,
R₁' is -(CH₂)₀₋₆-,
X is amino, hydroxyl, ethynyl, cyano, -C(O)(CH₂)₁₋₃NRₐR_{b}, -C(O)O(CH₂)₁₋₃NRₐR_{b}, -OC(O)(CH₂)₁₋₃NRₐR_{b}, -C(O)NH(CH₂)₁₋₃NRₐR_{b}, -NHC(O)(CH₂)₁₋₃NRₐR_{b}, -NHC(O)CH(NRₐR_{b})(CH₂)₁₋₃NRₐR_{b}, C₃₋₇ cycloalkyl, 4-7 membered heterocyclic group, C₆₋₁₀ aryl or 5-10 membered heteroaryl, the said cycloalkyl, heterocyclic group, aryl or heteroaryl are optionally substituted by the following groups: -(CH₂)₁₋₃OH, -(CH₂)₁₋₃NRₐR_{b}, and -(CH₂)₁₋₃C(O)NRₐR_{b}; or
X can also be: 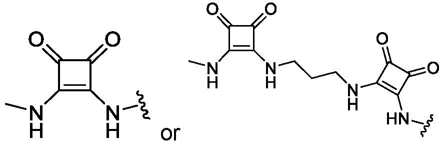
Rₐ, and R_{b} are independently selected from H, C₁₋₃ alkyl, -(CH₂)₁₋₃NH₂, and -(CH₂)₁₋₃NH(CH₂)₁₋₃NH₂; or Rₐ, and R_{b} together with the nitrogen to which they are attached form a 5-10 membered heterocycle including 1-3 heteroatoms selected from N, O or S, by said heterocycle is optionally substituted by one or more of the following groups: C₁₋₆ alkyl, C₁₋₆ alkyl halides, C₁₋₆ alkyl hydroxyl and C₁₋₆ alkyl amino;
each M is independently selected from -CH₂-, -CH=CH-, -NH-, -C(O)-, -O-, -C(O)O-, -OC(O)-, -C(O)NH-, and -NHC(O)- ;
each R' is independently selected from C₁₋₁₀ alkyl ;
each R* is independently selected from C₂₋₁₀ alkyl ;
n, m are independently selected from the integer range from 1-9;
or a salt or an isomer thereof.

2. The compound of claim 1, wherein:
R₁' is -(CH₂)₂₋₃-,
X is hydroxyl, -C(O)(CH₂)₂₋₃NRₐR_{b,} -C(O)O(CH₂)₂₋₃NRₐR_{b}, -C(O)NH(CH₂)₂₋₃NRₐR_{b}, or 5-10 heteroaryl which is optionally substituted by the following groups: -(CH₂)₂₋₃OH, -(CH₂)₂₋₃NRₐR_{b}, -(CH₂)₂₋₃C(O)NRₐR_{b} ; or
X can also be: 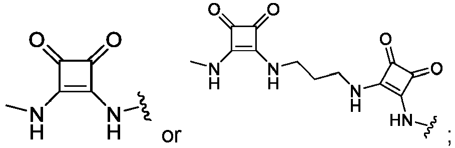
Rₐ, and R_{b} are independently selected from H, C₁₋₃ alkyl, -(CH₂)₂₋₃NH₂, or -(CH₂)₂₋₃NH(CH₂)₂₋₃NH₂; or Rₐ, and R_{b} together with the nitrogen to which they are attached form a 5-10 membered heterocycle including 1-3 heteroatoms selected from N or O, , said heterocycle is optionally substituted by the following groups: C₁₋₆ alkyl, C₁₋₆ alkyl halides, C₁₋₆ alkyl hydroxyl groups and C₁₋₆ alkyl amino groups;
or a salt thereof.

3. The compound of any one of claims 1-2, wherein:
each M is independently selected from -CH₂-, -CH=CH-, -C(O)O-, -OC(O)-, -C(O)NH-, -NHC(O)-;
or a salt thereof.

4. The compound of any one of claims 1-3, wherein the compound has formula (II), and wherein
each R* is independently selected from C₆₋₁₀ alkyl, preferably C₆ alkyl; preferably wherein
each M is independently selected from -C(O)O- or -OC(O)-, further preferably -C(O)O-; and/or
a. R₁ is selected from -R₁'-X, R₁' is -(CH₂)₁₋₆-, and X is hydroxyl; or
b. R₁ is selected from -R₁'-X, R₁' is -(CH₂)₁₋₆-, and X is -C(O)(CH₂)₂₋₃NRₐR_{b}, -C(O)O(CH₂)₂₋₃NRₐR_{b}, -C(O)NH(CH₂)₂₋₃NRₐR_{b},
wherein Rₐ. and R_{b} are independently selected from H, C₁₋₃ alkyl, -(CH₂)₂₋₃NH₂; or 5-10 membered heterocycle containing 1-3 heteroatoms selected from N or O, which is formed by Rₐ, and R_{b} together with the nitrogen atom to which they are attached, preferably morpholinyl or piperidinyl, said heterocycle is -optionally substituted by the following groups: C₁₋₆ alkyl hydroxyl; or
c. R₁ is selected from R₁'-X, R₁' is -(CH₂)₁₋₆-, X is 5-6 membered heteroaromatic group, preferably triazolyl, the said heteroaromatic group is optionally substituted with the following groups: -(CH₂)₂₋₃OH, -(CH₂)₂₋₃NRₐR_{b}, -(CH₂)₂₋₃C(O)NRₐR_{b},
wherein Rₐ, and R_{b} are independently selected from H, C₁₋₃ alkyl, -(CH₂)₂₋₃NH₂, -(CH₂)₂₋₃NH(CH₂)₂₋₃NH₂, or 5-10 membered heterocycle containing 1-3 heteroatoms selected from N or O, which is formed together by Rₐ, R_{b} and their connected nitrogen atom,, preferably morpholinyl, piperazinyl or piperidinyl, the said heterocycle is optionally substituted by the following groups: C₁₋₆ alkyl, or hydroxyl; or
d. R₁ is selected from -R₁'-X, R₁' is -(CH₂)₁₋₆-, and X is 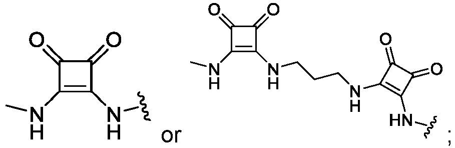 and/or
wherein each n is 7, and m is 7;
or a salt thereof.

5. The compound of any one of claims 1-3, wherein the compound has formula (III), and wherein:
a. each R' is independently selected from C₂₋₈ alkyl; and/or
b. each M is independently selected from -C(O)O- or -OC(O)-, preferably -C(O)O-;
or a salt thereof.

6. The compound of any one of claims 1-3, the compound of the following formula (IV), and wherein
a. each R* is independently selected from C₆₋₁₀ alkyl, preferably C₆ alkyl; and/or
b. each M is independently selected from -C(O)O- or -OC(O)-, preferably -C(O)O-;
or a salt thereof.

7. The compound of any one of claims 1-3, wherein the compound has formula (V), and wherein:
a. each R* is independently selected from C₆₋₁₀ alkyl, preferably C₆ alkyl; and/or
b. each M is independently selected from -CH=CH-, -C(O)O- or -OC(O)-, preferably -CH=CH- or -C(O)O-; and/or
c. each R' is C₁₀ alkyl or C₈ alkenyl;
or a salt thereof.

8. A compound according to claim 1, or salt or isomer thereof, wherein:
the compound is one of 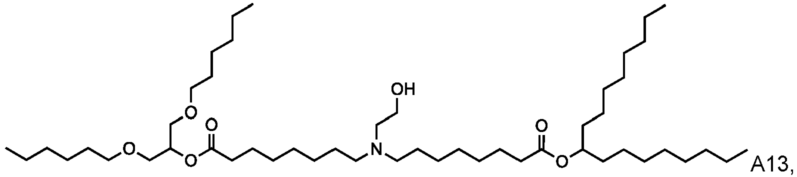 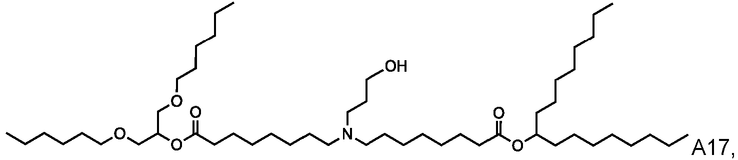 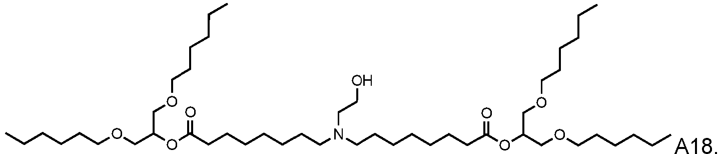 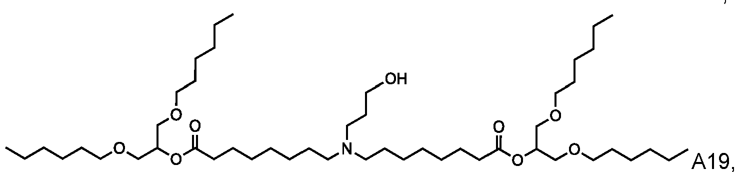 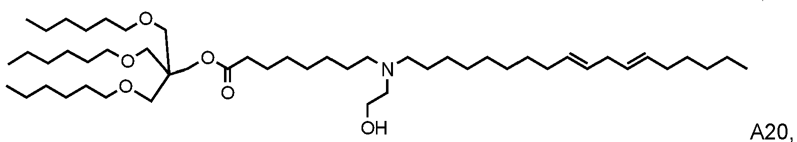 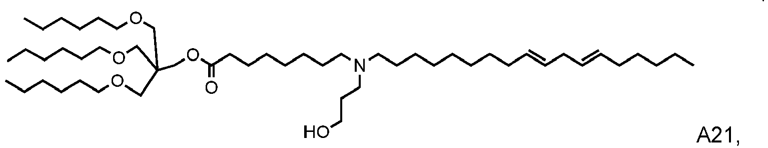 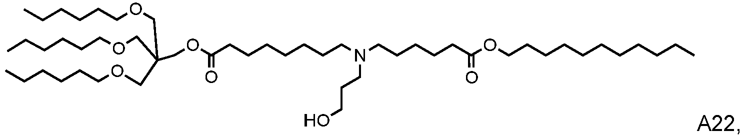 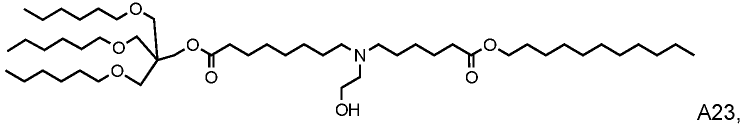 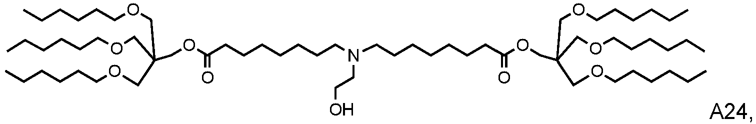 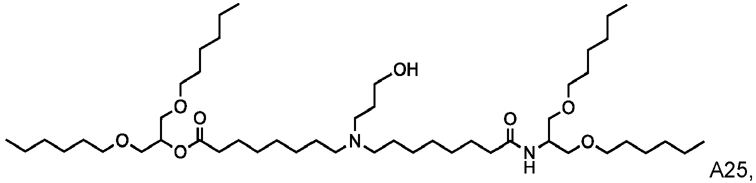 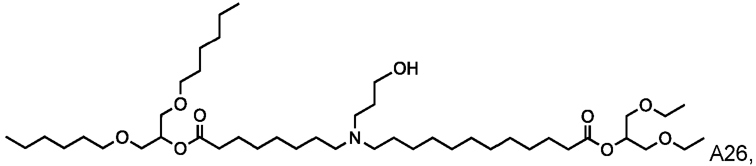 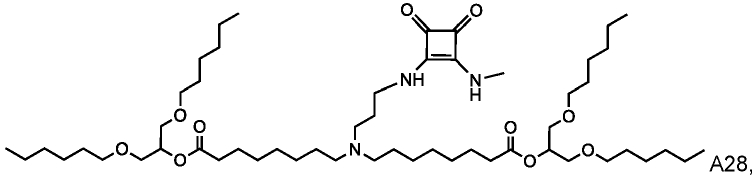 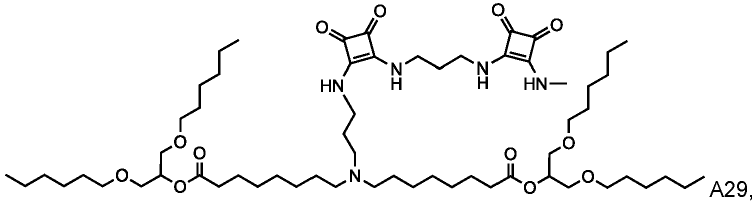 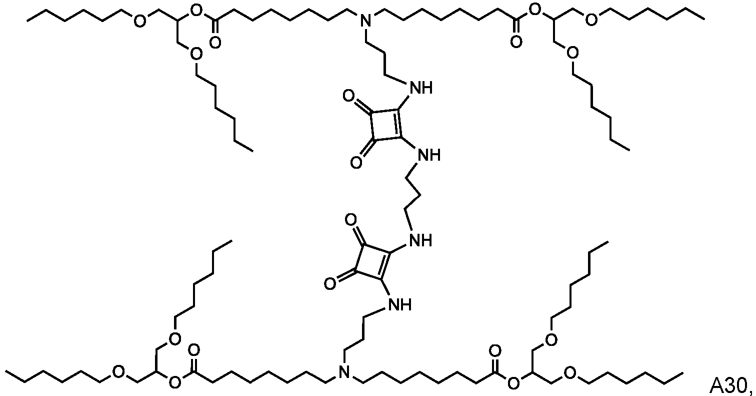 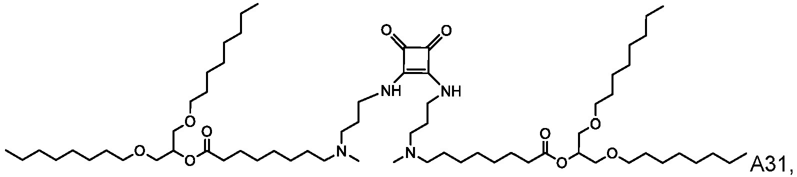 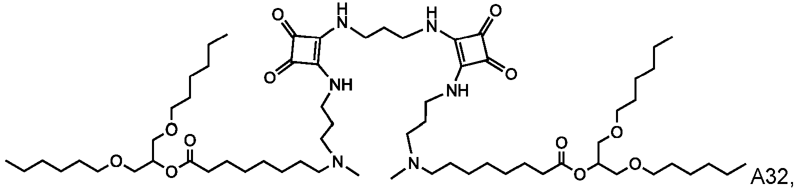 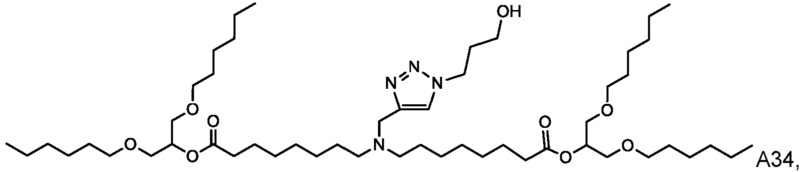 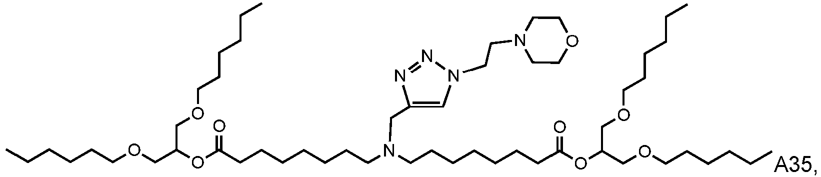 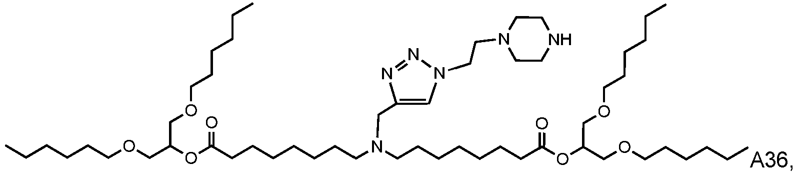 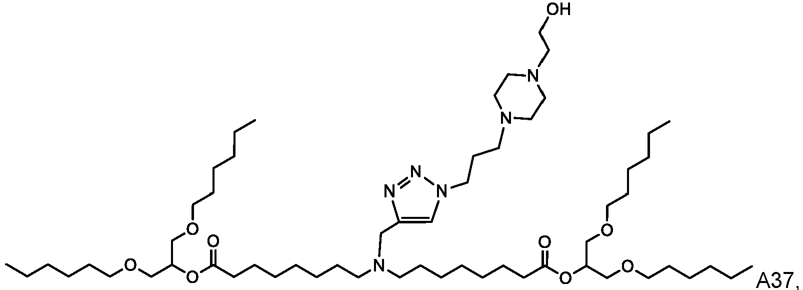 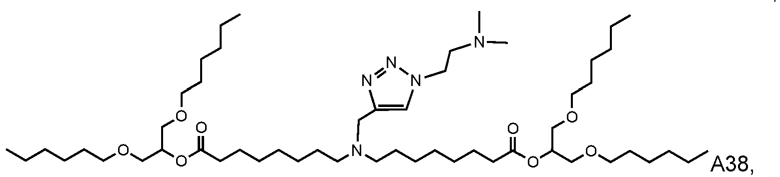 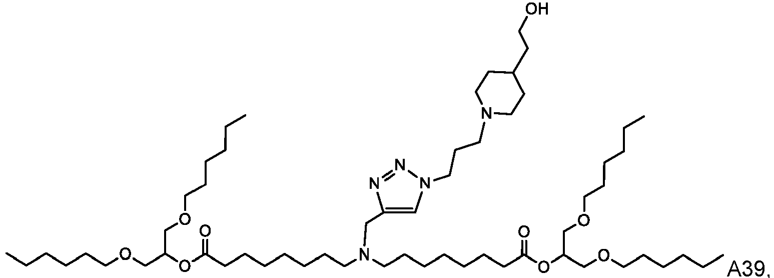 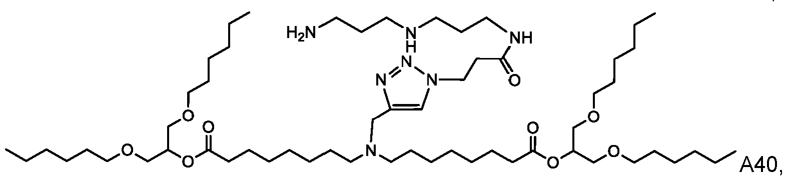 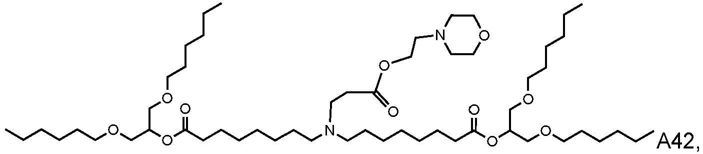 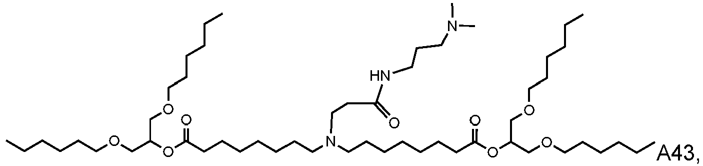 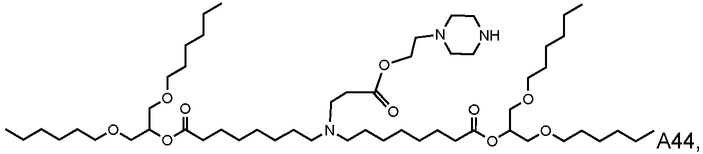 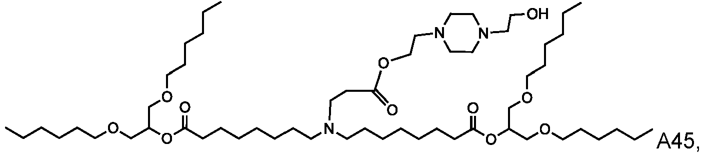 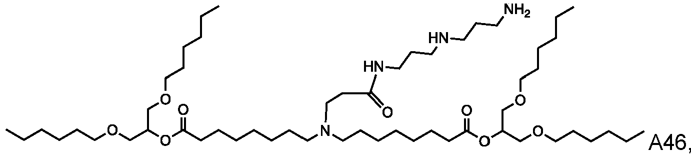 and

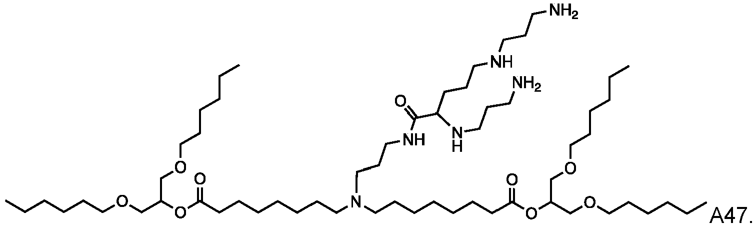

9. A composition comprising a compound according to any one of the claims 1-8 as an ionizable lipid compound, preferably
a. further comprising a phospholipid,
further preferably wherein:
the phospholipid is selected from 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3 -phosphocholine (DSPC), 1,2-diundecanoyl-sn-glycero-phosphocholine (DUPC), I-palmitoyl-2-oleoyl-sn-glycero-3 -phosphocholine (POPC), 1,2-di-O-octadecenyl-5«-glycero-3-phosphocholine (18:0 Diether PC), 1-oleoyl-2-cholesterylhemisuccinoyl-5«-glycero-3-phosphocholine (OChemsPC), 1-hexadecyl-sn-glycero-3-phosphocholine (C16 Lyso PC), 1,2-dilinolenoyl-sn-glycero-3 -phosphocholine, 1,2-diarachidonoyl-sn-glycero-3 -phosphocholine, 1,2-didocosahexaenoyl-sn-glycero-3-phosphocholine, 1,2-dioleoyl-sn-glycero-3-phosphoethanol amine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3 -phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoyl-phosphatidylethanolamine (DSPE), dipalmitoyl phosphatidyl ethanolamine (DPPE), dimyristoylphosphoethanolamine (DMPE), 1-stearoyl-2-oleoyl-phosphatidyethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine (LPE), or a combination thereof; and/or
b. further comprising a PEG lipid,
further preferably wherein:
the PEG lipid is selected from PEG-modified phosphatidylethanolamine, PEG- modified phosphatidic acid, PEG-modified ceramide, PEG-modified dialkylamine, PEG- modified diacylglycerol, PEG-modified dialkylglycerol, or a combination thereof; and/or
c. further comprising a structural lipid,
further preferably wherein:
the structural lipid is selected from: cholesterol, fecosterol, sitosterol, ergosterol, campesterol, stigmasterol, brassicasterol, tomatidine, ursolic acid, alpha-tocopherol, or a combination thereof; and/or
d. further comprising an active ingredient, which is selected from at least any one of: DNA, RNA, protein, or an active pharmaceutical molecule,
further preferably:
i) wherein the active agent is RNA and the RNA is selected from at least any one of: mRNA, siRNA, aiRNA, miRNA, dsRNA, aRNA, or IncRNA; or
ii) wherein the active agent is a protein which is is selected from at least any one of: antibody, enzyme, recombinant protein, polypeptide and short chain polypeptide.

10. The composition of claim 9, wherein:
the composition is in the form of a lipid nanoparticle.

11. A method of producing lipid nanoparticle, comprising:
mixing an ionizable compound of claim 1 with a PEG lipid, a structural lipid and a phospholipid to form a lipid mixture, preferably
further comprising:
mixing an active ingredient with the lipid mixture to form lipid nanoparticle by mixer.

12. The compound of any one of claims 1-8, for use in the production of lipid nanoparticle, preferably wherein
the said lipid nanoparticle is neutral and uncharged in a neutral medium, and is positively charged after being protonated in an acidic medium; and
the said lipid nanoparticle is as defined in claim 9.

13. The method of claim 11, wherein:
the compound is dissolved and mixed with a PEG lipid, a structural lipid and a phospholipid to form a lipid mixture, then mixing an active ingredient with the lipid mixture by mixer to form a lipid nanoparticle.

14. A pharmaceutical composition comprising the lipid nanoparticle of claim 10, and a pharmaceutically acceptable carrier.

15. The lipid nanoparticle composition of claim 10, or the pharmaceutical composition of claim 14, for use as a medicament, preferably
i) further comprising an active ingredient, the active ingredient selected from at least any one of DNA, RNA, protein, or an active pharmaceutical molecule, preferably
a. wherein the active ingredient is RNA that is selected from at least any one of: mRNA, siRNA, aiRNA, miRNA, dsRNA, aRNA, or IncRNA, or
b. wherein the active ingredient is a protein is selected from at least any one of: antibody, enzyme, recombinant protein, polypeptide and short chain polypeptide; and/or
ii) wherein the said medicament can be administered to a human by intravenous injection, intramuscular injection, subcutaneous injection, microneedle patch, oral administration, oral and nasal spray, or painting.

## Patentansprüche

1. Eine Verbindung einer der Formeln (II), (III), (IV) oder (V): 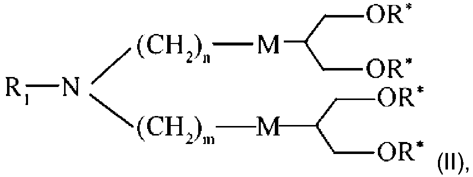 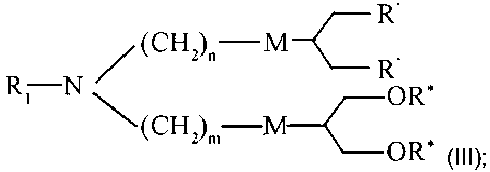 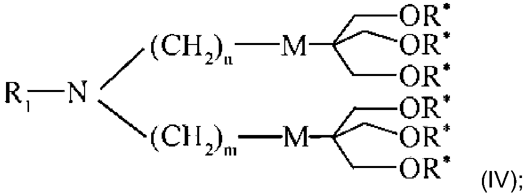 oder 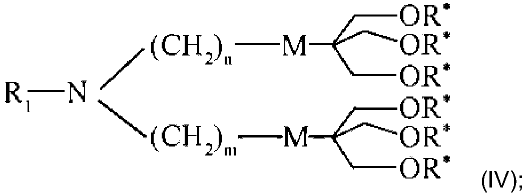
wobei R₁ aus -R₁'-X,
R₁' -(CH₂)₀₋₆- ist,
X für Amino, Hydroxyl, Ethinyl, Cyano, -C(O)(CH₂)₁₋₃NRₐ R_{b} , -C(O)O(CH₂)₁₋₃NRₐR_{b}, -OC(O)(CH₂)₁₋₃NRₐR_{b}, -C(O)NH(CH₂)₁₋₃NRₐR_{b}, -NHC(O)(CH₂)₁₋₃NRₐR_{b}, -NHC(O)CH(NRₐR_{b})(CH₂)₁₋₃NRₐR_{b}, C₃₋₇ Cycloalkyl, eine 4-7-gliedrige heterocyclische Gruppe, C₆₋₁₀-Aryl oder 5-10-gliedriges Heteroaryl steht, wobei das Cycloalkyl, die heterocyclische Gruppe, das Aryl oder das Heteroaryl gegebenenfalls durch die folgenden Gruppen substituiert sind: (CH₂)₁₋₃OH, -(CH₂)₁₋₃NRₐR_{b} und -(CH₂)₁₋₃C(O)NRₐR_{b}; oder
X kann auch sein: 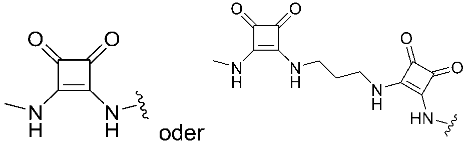
Rₐ und R_{b} sind unabhängig voneinander ausgewählt aus H, C₁₋₃-Alkyl, -(CH₂)₁₋₃NH₂ und -(CH₂)₁₋₃NH(CH₂)₁₋₃NH₂; oder Rₐ und R_{b} bilden zusammen mit dem Stickstoffatom, an das sie gebunden sind, einen 5- bis 10-gliedrigen Heterocyclus, der 1 bis 3 Heteroatome enthält, ausgewählt aus N, O oder S, wobei der Heterocyclus gegebenenfalls durch eine oder mehrere der folgenden Gruppen substituiert ist: C₁₋₆-Alkyl, C₁₋₆-Alkylhalogenide, C₁₋₆-Alkylhydroxyl und C₁₋₆-Alkylamino;
jedes M unabhängig aus -CH₂-, -CH=CH-, -NH-, -C(O)-, -O-, -C(O)O-, -OC(O)-, -C(O)NH- und -NHC(O)- ausgewählt ist;
jedes R' unabhängig aus C₁₋₁₀-Alkyl ausgewählt ist;
jedes R* unabhängig aus C₂₋₁₀-Alkyl ausgewählt ist;
n und m sind unabhängig voneinander aus dem ganzzahligen Bereich von 1 bis 9 ausgewählt;
oder ein Salz oder ein Isomer davon.

2. Verbindung nach Anspruch 1, wobei:
R₁' ist -(CH₂)₂₋₃-,
X ist Hydroxyl, -C(O)(CH₂)₂₋₃NRₐR_{b}, -C(O)O(CH₂)₂₋₃NRₐR_{b}, -C(O)NH(CH₂ )₂₋₃NRₐR_{b} oder 5-10-Heteroaryl, das gegebenenfalls durch die folgenden Gruppen substituiert ist: -(CH₂)₂₋₃OH, -(CH₂)₂₋₃NRₐR_{b}, -(CH₂)₂₋₃C(O)NRₐR_{b}; oder
X kann auch sein: 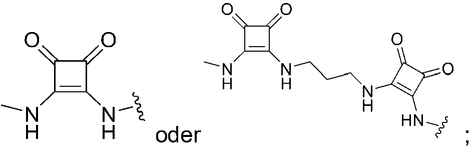
Rₐ und R_{b} sind unabhängig voneinander ausgewählt aus H, C₁₋₃-Alkyl, -(CH₂)₂₋₃NH₂ oder -(CH₂)₂₋₃NH(CH₂)₂₋₃NH₂; oder Rₐ und R_{b} bilden zusammen mit dem Stickstoffatom, an das sie gebunden sind, einen 5- bis 10-gliedrigen Heterocyclus, der 1 bis 3 Heteroatome enthält, ausgewählt aus N oder O, wobei der Heterocyclus gegebenenfalls durch die folgenden Gruppen substituiert ist: C₁₋₆-Alkyl, C₁₋₆-Alkylhalogenide, C₁₋₆-Alkylhydroxylgruppen und C₁₋₆-Alkylaminogruppen;
oder ein Salz davon.

3. Die Verbindung nach einem der Ansprüche 1-2, wobei:
jedes M unabhängig voneinander aus -CH₂-, -CH=CH-, -C(O)O-, -OC(O)-, -C(O)NH- und -NHC(O)- ausgewählt ist;
oder ein Salz davon.

4. Die Verbindung nach einem der Ansprüche 1-3, wobei die Verbindung die Formel (II) aufweist und wobei
jedes R* unabhängig aus C₆₋₁₀-Alkyl, vorzugsweise C₆-Alkyl, ausgewählt ist; wobei vorzugsweise
jedes M unabhängig aus -C(O)O- oder -OC(O)- ausgewählt ist, weiter bevorzugt aus -C(O)O-; und/oder
a. R₁ aus -R₁'-X ausgewählt ist, wobei R₁' -(CH₂)₁₋₆- ist und X Hydroxyl ist; oder
b. R₁ ausgewählt wird aus -R₁'-X, wobei R₁' -(CH₂ )₁₋₆ - ist und X -C(O)(CH₂)₂₋₃NRₐR_{b}, -C(O)O(CH₂)₂₋₃NRₐR_{b}, -C(O)NH(CH₂)₂₋₃NRₐR_{b},
wobei Rₐ und R_{b} unabhängig voneinander ausgewählt sind aus H, C₁₋₃-Alkyl, -(CH₂)₂₋₃NH₂; oder einem 5- bis 10-gliedrigen Heterocyclus, der 1 bis 3 Heteroatome enthält, die aus N oder O ausgewählt sind, und der durch Rₐ und R_{b} zusammen mit dem Stickstoffatom, an das sie gebunden sind, gebildet wird, vorzugsweise Morpholinyl oder Piperidinyl, wobei der Heterocyclus gegebenenfalls durch die folgenden Gruppen substituiert ist: C₁₋₆-Alkyl, Hydroxyl; oder
c. R₁ ausgewählt wird aus R₁'-X, wobei R₁' -(CH₂)₁₋₆- ist, X eine 5- bis 6-gliedrige heteroaromatische Gruppe, vorzugsweise Triazolyl, ist, wobei die heteroaromatische Gruppe gegebenenfalls mit den folgenden Gruppen substituiert ist: -(CH₂)₂₋₃OH, -(CH₂)₂₋₃NRₐR_{b}, -(CH₂)₂₋₃C(O)NRₐR_{b},
wobei Rₐ und R_{b} unabhängig voneinander aus H, C₁₋₃-Alkyl, -(CH₂)₂₋₃NH₂, -(CH₂)₂₋₃NH(CH₂)₂₋₃NH₂ oder einem 5- bis 10-gliedrigen Heterocyclus, der 1 bis 3 Heteroatome enthält, die aus N oder O ausgewählt sind, und der gemeinsam durch Rₐ, R_{b} und ihr verbundenes Stickstoffatom gebildet wird, vorzugsweise Morpholinyl, Piperazinyl oder Piperidinyl, wobei der genannte Heterocyclus gegebenenfalls durch die folgenden Gruppen substituiert ist: C₁₋₆-Alkyl oder Hydroxyl; oder
d. R₁ ausgewählt wird aus -R₁'-X, wobei R₁' -(CH₂)₁₋₆- ist und X ist 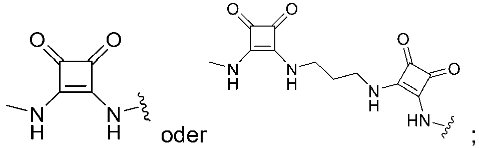 und/oder
wobei jedes n gleich 7 und m gleich 7 ist;
oder ein Salz davon.

5. Die Verbindung nach einem der Ansprüche 1-3, wobei die Verbindung die Formel (III) aufweist und wobei:
a. jedes R' unabhängig voneinander aus C₂₋₈-Alkyl ausgewählt ist; und/oder
b. jedes M unabhängig aus -C(O)O- oder -OC(O)- ausgewählt ist, vorzugsweise -C(O)O- ist;
oder ein Salz davon.

6. Die Verbindung nach einem der Ansprüche 1 bis 3, wobei die Verbindung die folgende Formel (IV) aufweist und wobei
a. jedes R* ist unabhängig ausgewählt aus C₆₋₁₀-Alkyl, vorzugsweise C₆-Alkyl ist; und/oder
b. jedes M unabhängig voneinander aus -C(O)O- oder -OC(O)-, vorzugsweise aus -C(O)O-, ausgewählt ist;
oder ein Salz davon.

7. Verbindung nach einem der Ansprüche 1 bis 3, wobei die Verbindung die Formel (V) aufweist und wobei:
a. jedes R* unabhängig voneinander aus C₆₋₁₀-Alkyl, vorzugsweise C₆-Alkyl, ausgewählt ist; und/oder
b. jedes M unabhängig aus -CH=CH-, -C(O)O- oder -OC(O)-, vorzugsweise aus -CH=CH- oder -C(O)O-, ausgewählt ist; und/oder
c. jedes R' C₁₀-Alkyl oder C₈-Alkenyl ist;
oder ein Salz davon.

8. Verbindung nach Anspruch 1 oder ein Salz oder Isomer davon, wobei:
die Verbindung eine der folgenden ist:

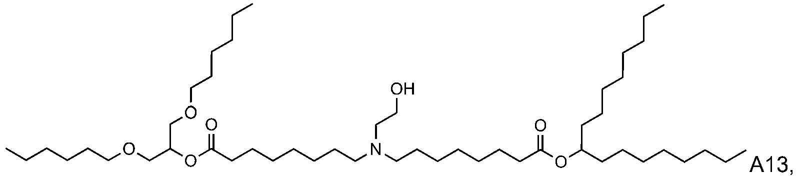

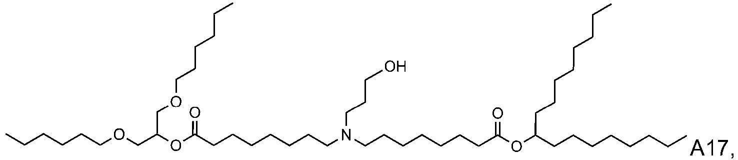

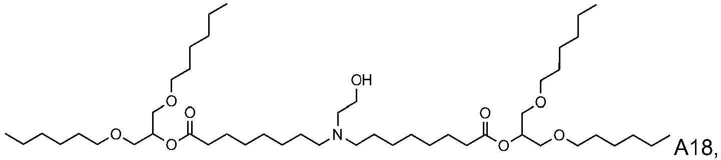

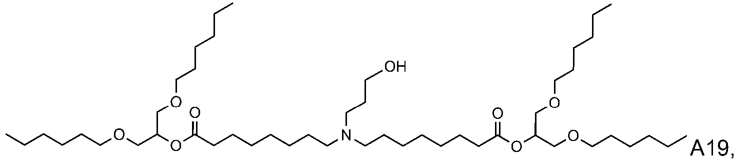

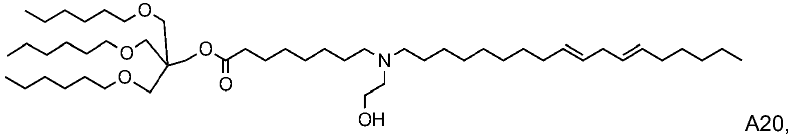

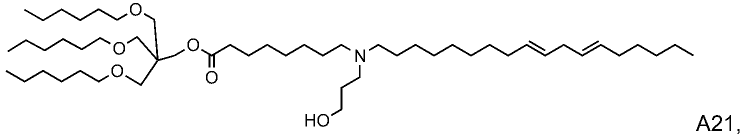

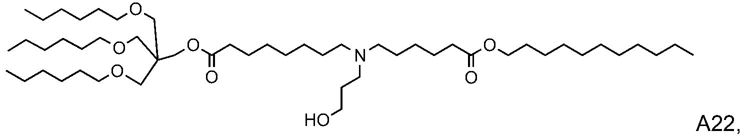

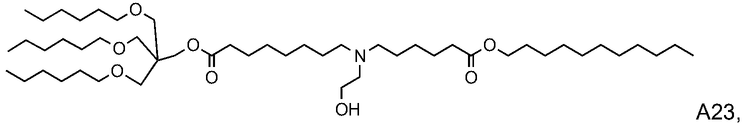

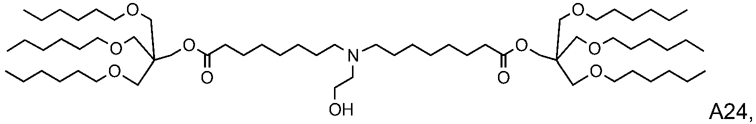

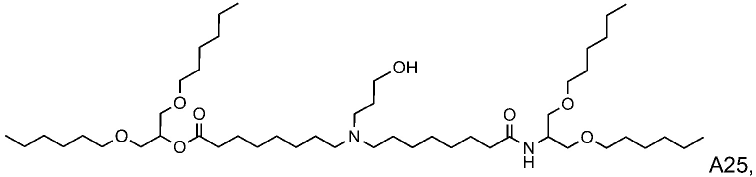

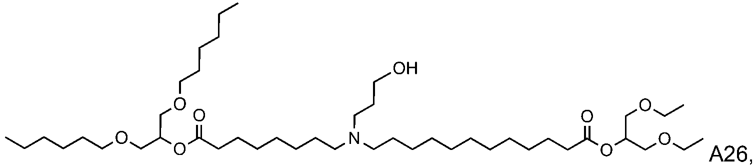

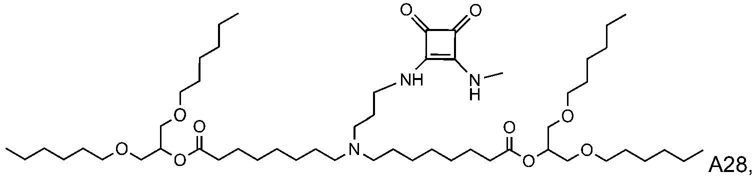

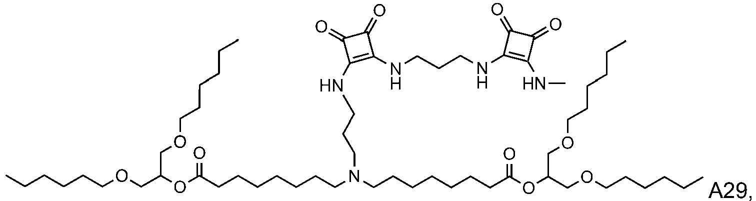

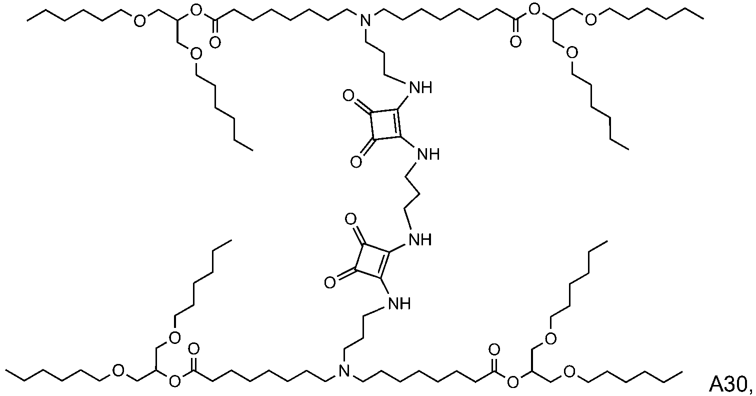

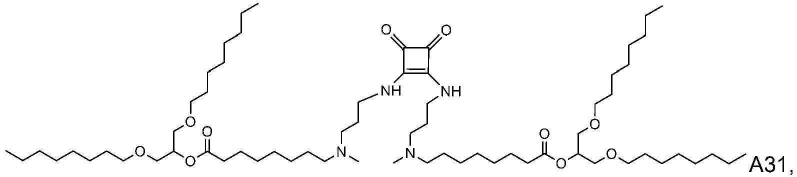

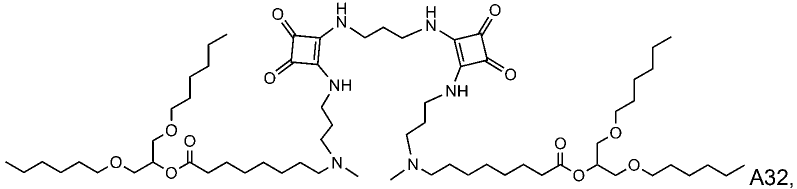

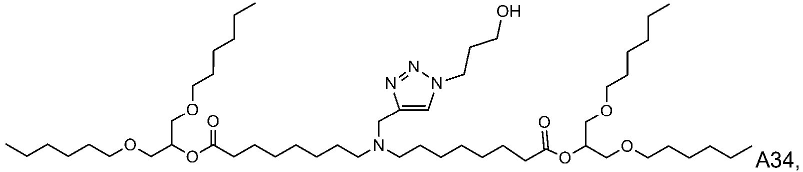

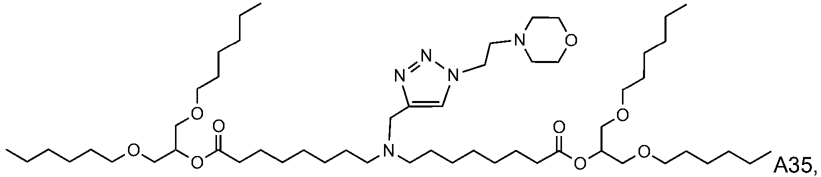

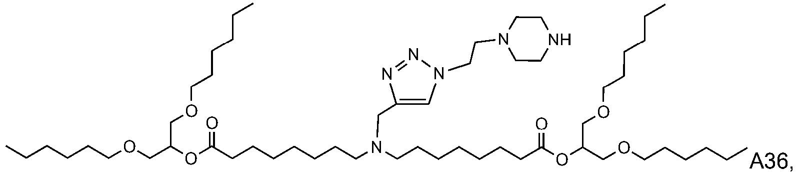

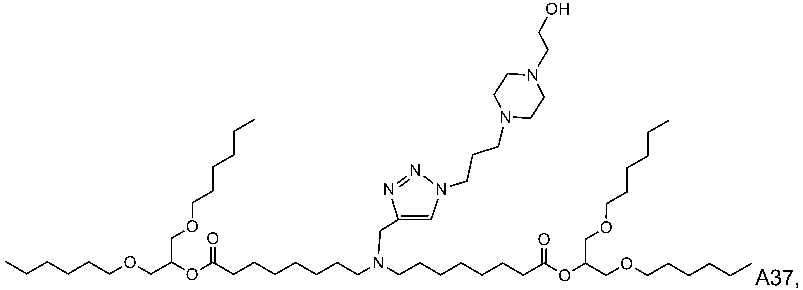

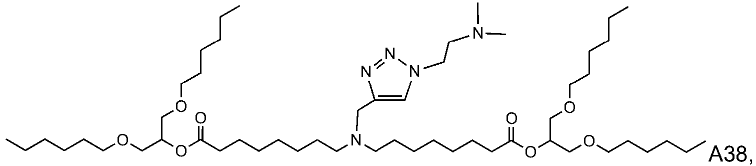

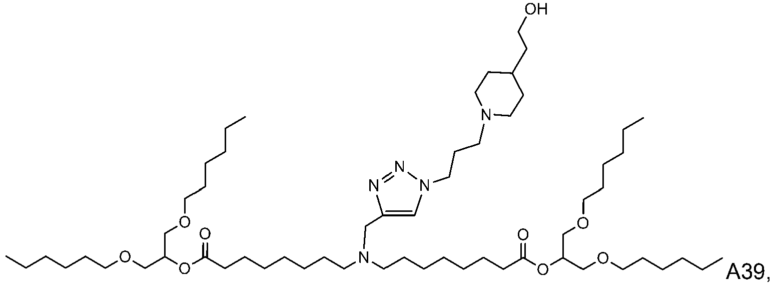

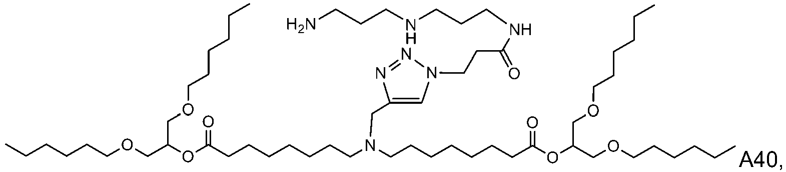

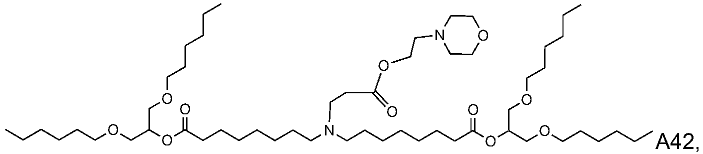

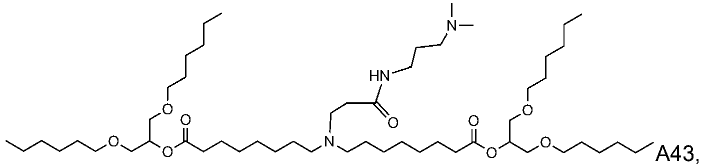

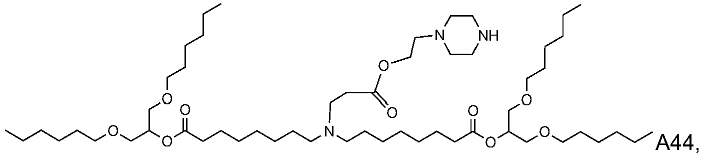

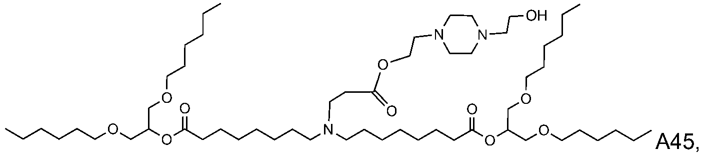

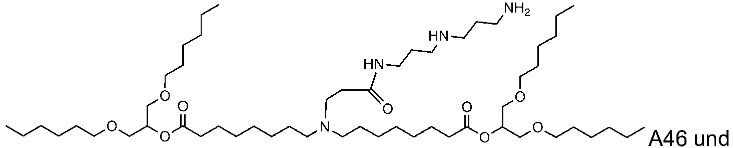

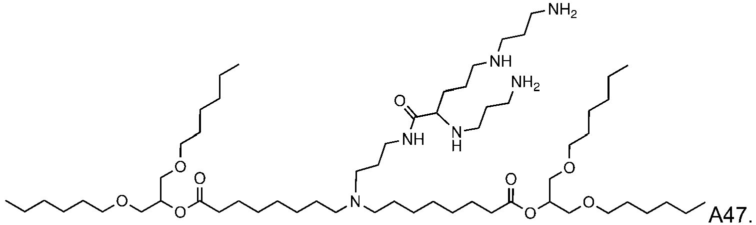

9. Zusammensetzung, die eine Verbindung gemäß einem der Ansprüche 1-8 als ionisierbare Lipidverbindung enthält, vorzugsweise
a. die ferner ein Phospholipid enthält,
wobei weiter bevorzugt gilt:
das Phospholipid wird ausgewählt aus 1,2-Dilinoleoyl-sn-glycero-3-phosphocholin (DLPC), 1,2-Dimyristoyl-sn-glycero-phosphocholin (DMPC), 1,2-Dioleoyl-sn-glycero-3-phosphocholin (DOPC), 1,2-Dipalmitoyl-sn-glycero-3-phosphocholin (DPPC), 1,2-Distearoyl-sn-glycero-3-phosphocholin (DSPC), 1,2-Diundecanoyl-sn-glycero-phosphocholin (DUPC), I-Palmitoyl-2-oleoyl-sn-glycero-3-phosphocholin (POPC), 1,2-Di-O-octadecenyl-5«-glycero-3-phosphocholin (18:0-Diether-PC), 1-Oleoyl-2-cholesterylhemisuccinoyl-5«-glycero-3-phosphocholin (OChemsPC), 1-Hexadecyl-sn-glycero-3-phosphocholin (C16 Lyso PC), 1,2-Dilinolenoyl-sn-glycero-3-phosphocholin, 1,2-Diarachidonoyl-sn-glycero-3-phosphocholin, 1,2-Didocosahexaenoyl-sn-glycero-3-phosphocholin, 1,2-Dioleoyl-sn-glycero-3-Phosphoethanolamin (DOPE), 1,2-Diphytanoyl-sn-glycero-3-phosphoethanolamin (ME 16,0 PE), 1,2-Distearoyl-sn-glycero-3-phosphoethanolamin, 1,2-Dilinoleoyl-sn-glycero-3-phosphoethanolamin, 1,2-Dilinolenoyl-sn-glycero-3-phosphoethanolamin, 1,2-Diarachidonoyl-sn-glycero-3-phosphoethanolamin, 1,2-Didocosahexaenoyl-sn-glycero-3-phosphoethanolamin, 1,2-Dioleoyl-sn-glycero-3-phospho-rac-(1-glycerin)-natriumsalz (DOPG), Dipalmitoylphosphatidylglycerin (DPPG), Palmitoyloleoylphosphatidylethanolamin (POPE), Distearoyl-phosphatidyl-ethanolamin (DSPE), Dipalmitoylphosphatidylethanolamin (DPPE), Dimyristoylphosphoethanolamin (DMPE), 1-Stearoyl-2-oleoyl-phosphatidylethanolamin (SOPE), 1-Stearoyl-2-oleoyl-phosphatidylcholin (SOPC), Sphingomyelin, Phosphatidylcholin, Phosphatidylethanolamin, Phosphatidylserin, Phosphatidylinositol, Phosphatidsäure, Palmitoyloleoyl-Phosphatidylcholin, Lysophosphatidylcholin, Lysophosphatidylethanolamin (LPE) oder eine Kombination davon; und/oder
b. wobei es ferner ein PEG-Lipid umfasst,
wobei ferner bevorzugt gilt:
das PEG-Lipid ausgewählt ist aus PEG-modifiziertem Phosphatidylethanolamin, PEG-modifizierter Phosphatidsäure, PEG-modifiziertem Ceramid, PEG-modifiziertem Dialkylamin, PEG-modifiziertem Diacylglycerin, PEG-modifiziertem Dialkylglycerin oder einer Kombination davon; und/oder
c. ferner ein Strukturlipid enthält,
wobei ferner bevorzugt gilt, dass:
das Strukturlipid ausgewählt ist aus: Cholesterin, Fecosterol, Sitosterol, Ergosterol, Campesterol, Stigmasterol, Brassicasterol, Tomatidin, Ursolsäure, Alpha-Tocopherol oder einer Kombination davon; und/oder
d. ferner einen Wirkstoff enthält, der aus mindestens einem der folgenden ausgewählt ist: DNA, RNA, Protein oder einem pharmazeutisch wirksamen Molekül, weiterhin vorzugsweise:
i) wobei der Wirkstoff RNA ist und die RNA aus mindestens einem der folgenden ausgewählt ist: mRNA, siRNA, aiRNA, miRNA, dsRNA, aRNA oder IncRNA; oder
ii) wobei der Wirkstoff ein Protein ist, das aus mindestens einer der folgenden Gruppen ausgewählt ist: Antikörper, Enzym, rekombinantes Protein, Polypeptid und kurzkettiges Polypeptid.

10. Die Zusammensetzung nach Anspruch9 , wobei:
die Zusammensetzung in Form eines Lipid-Nanopartikels vorliegt.

11. Verfahren zur Herstellung von Lipidnanopartikeln, umfassend:
das Vermischen einer ionisierbaren Verbindung nach Anspruch 1 mit einem PEG-Lipid, einem Strukturlipid und einem Phospholipid zur Bildung einer Lipidmischung, vorzugsweise
ferner umfassend:
das Mischen eines Wirkstoffs mit der Lipidmischung unter Verwendung eines Mischers zur Bildung von Lipidnanopartikeln.

12. Die Verbindung nach einem der Ansprüche 1-8 zur Verwendung bei der Herstellung von Lipidnanopartikeln, vorzugsweise wobei
die Lipidnanopartikel in einem neutralen Medium neutral und ungeladen sind und nach Protonierung in einem sauren Medium positiv geladen sind; und
das Lipidnanopartikel wie in Anspruch 9 definiert ist.

13. Verfahren nach Anspruch11, wobei:
die Verbindung gelöst und mit einem PEG-Lipid, einem Strukturlipid und einem Phospholipid gemischt wird, um eine Lipidmischung zu bilden, und anschließend ein Wirkstoff mit der Lipidmischung mittels eines Mischers vermischt wird, um ein Lipidnanopartikel zu bilden.

14. Pharmazeutische Zusammensetzung, umfassend das Lipidnanopartikel nach Anspruch 10 sowie einen pharmazeutisch verträglichen Träger.

15. Die Lipidnanopartikel-Zusammensetzung nach Anspruch 10 oder die pharmazeutische Zusammensetzung nach Anspruch 14 zur Verwendung als Arzneimittel, vorzugsweise
i) die ferner einen Wirkstoff enthält, wobei der Wirkstoff aus mindestens einem der folgenden ausgewählt ist: DNA, RNA, Protein oder ein pharmazeutisch wirksames Molekül, vorzugsweise
a. wobei der Wirkstoff RNA ist, die aus mindestens einem der folgenden ausgewählt ist: mRNA, siRNA, aiRNA, miRNA, dsRNA, aRNA oder IncRNA, oder
b. wobei der Wirkstoff ein Protein ist, das aus mindestens einem der folgenden ausgewählt ist: Antikörper, Enzym, rekombinantes Protein, Polypeptid und kurzkettiges Polypeptid; und/oder
ii) wobei das genannte Arzneimittel einem Menschen durch intravenöse Injektion, intramuskuläre Injektion, subkutane Injektion, Mikronadelpflaster, orale Verabreichung, Mund- und Nasenspray oder Auftragen verabreicht werden kann.

## Revendications

1. Composé répondant à l'une des formules (II), (III), (IV) ou (V) : 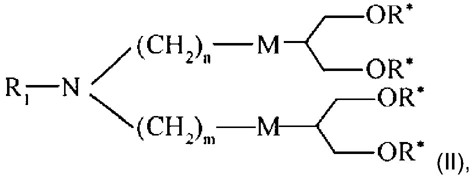 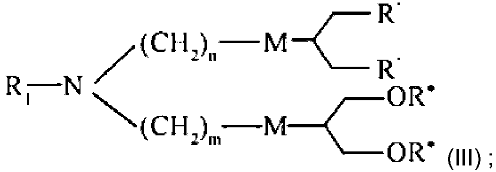 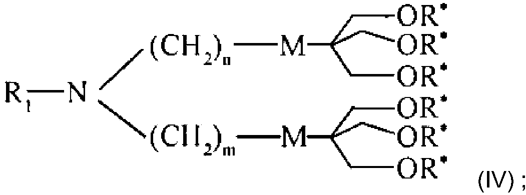 ou 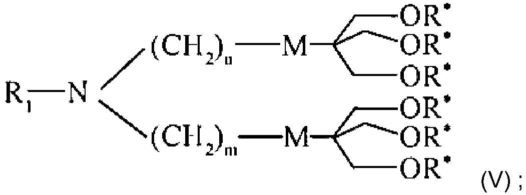
dans lesquelles R₁ est choisi parmi -R₁ '-X,
R₁ ' est -(CH₂ )₀₋₆-,
X est un groupe amino, hydroxyle, éthynyle, cyano, -C(O)(CH₂)₁₋₃NRₐR_{b}, -C(O)O(CH₂)_{1 -3}NRₐR_{b} , -OC(O)(CH₂)₁₋₃NRₐR_{b} , -C(O)NH(CH₂)₁₋₃NRₐR_{b}, -NHC(O)(CH₂)₁₋₃NRₐR_{b} , -NHC(O)CH(NRₐR_{b})(CH₂)₁₋₃NRₐR_{b}, un groupe cycloalkyle en C₃₋₇, un groupe hétérocyclique à 4-7 chaînons, un groupe aryle en C₆₋₁₀ ou un groupe hétéroaryle à 5-10 chaînons, lesdits groupes cycloalkyle, hétérocyclique, aryle ou hétéroaryle étant éventuellement substitués par les groupes suivants : -(CH₂)₁₋₃OH, -(CH₂)₁₋₃NRₐR_{b} , et -(CH₂)₁₋₃C(O)NRₐR_{b} ; ou
X peut également être : 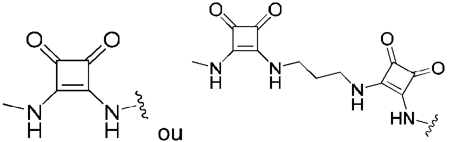
Rₐ et R_{b} sont choisis indépendamment parmi H, un groupe alkyle en C₁₋₃, -(CH₂)₁₋₃NH₂ et -(CH₂)₁₋₃NH(CH₂)₁₋₃NH₂; ou Rₐ et R_{b} forment, conjointement avec l'atome d'azote auquel ils sont liés, un hétérocycle de 5 à 10 chaînons comprenant 1 à 3 hétéroatomes choisis parmi N, O ou S, ledit hétérocycle étant éventuellement substitué par un ou plusieurs des groupes suivants : alkyle en C₁₋₆, halogénure d'alkyle en C₁₋₆, hydroxyle d'alkyle en C₁₋₆ et aminoalkyle en C₁₋₆;
chaque M est choisi indépendamment parmi -CH₂-, -CH=CH-, -NH-, -C(O)-, -O-,- C(O)O-, -OC(O)-, -C(O)NH- et -NHC(O)- ;
chaque R' est choisi indépendamment parmi les groupes alkyle en C₁₋₁₀;
chaque R* est choisi indépendamment parmi les groupes alkyle en C₂₋₁₀;
n et m sont choisis indépendamment parmi les nombres entiers compris entre 1 et 9 ;
ou un sel ou un isomère de celui-ci.

2. Composé selon la revendication 1, dans lequel :
R₁ est -(CH₂)₂₋₃-,
X est un groupe hydroxyle, -C(O)(CH₂)₂₋₃NRₐR_{b} , -C(O)O(CH₂)₂₋₃NRₐR_{b}, -C(O)NH(CH₂)₂₋₃NRₐR_{b}, ou un hétéroaryle en C₅₋₁₀ éventuellement substitué par les groupes suivants : -(CH₂)₂₋₃ OH, -(CH₂)₂₋₃NRₐR_{b} , -(CH₂)₂₋₃C(O)NRₐR_{b} ; ou
X peut également être 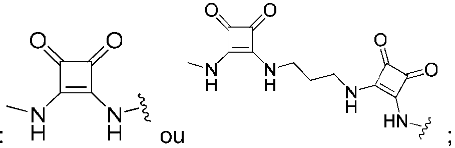
Rₐ et R_{b} sont choisis indépendamment parmi H, un groupe alkyle en C₁₋₃, -(CH₂)₂₋₃NH₂, ou- (CH₂)₂₋₃NH(CH₂)₂₋₃NH₂ ; ou Rₐ et R_{b} forment, conjointement avec l'atome d'azote auquel ils sont liés, un hétérocycle de 5 à 10 chaînons comprenant 1 à 3 hétéroatomes choisis parmi N ou O, ledit hétérocycle étant éventuellement substitué par les groupes suivants : alkyle en C₁₋₆, halogénure d'alkyle en C₁₋₆, hydroxyle d'alkyle en C₁₋₆ et aminoalkyle en C₁₋₆;
ou un sel de celui-ci.

3. Composé selon l'une quelconque des revendications 1 à 2, dans lequel :
chaque M est choisi indépendamment parmi -CH₂ -, -CH=CH-, -C(O)O-, -OC(O)-,- C(O)NH-, -NHC(O)- ;
ou un sel de celui-ci.

4. Composé selon l'une quelconque des revendications 1 à 3, dans lequel le composé répond à la formule (II), et dans lequel
chaque R* est choisi indépendamment parmi un groupe alkyle en C₆₋₁₀, de préférence un groupe alkyle en C₆; de préférence, dans lequel
chaque M est choisi indépendamment parmi -C(O)O- ou -OC(O)-, de préférence encore -C(O)O- ; et/ou
a. R₁ est choisi parmi -R₁ '-X, R₁ ' est -(CH₂ )₁₋₆-, et X est un groupe hydroxyle ; ou
b. R₁ est choisi parmi -R₁ '-X, R₁ 'est -(CH₂ )₁₋₆-, et X est -C(O)(CH₍₂₎)₂₋₃NRₐR_{b}, - C(O)O(CH₂)₂₋₃NRₐR_{b}, -C(O)NH(CH₂)₂₋₃NRₐR_{b},
dans lequel Rₐ et R_{b} sont choisis indépendamment parmi H, un groupe alkyle en C₁₋₃, un groupe -(CH₂)₂₋₃NH₂ ; ou un hétérocycle de 5 à 10 chaînons contenant 1 à 3 hétéroatomes choisis parmi N ou O, qui est formé par Rₐ et R_{b} conjointement avec l'atome d'azote auquel ils sont liés, de préférence un groupe morpholinyle ou pipéridinyle, ledit hétérocycle étant éventuellement substitué par les groupes suivants : hydroxyle alkyle en C₁₋₆; ou
c. R₁ est choisi parmi R₁ '-X, R₁' est -(CH₂)₁₋₆-, X est un groupe hétéroaromatique à 5 ou 6 chaînons, de préférence un groupe triazolyle, ledit groupe hétéroaromatique étant éventuellement substitué par les groupes suivants : -(CH₂)₂₋₃OH, -(CH₂)₂₋₃NRₐR_{b}, -(CH₂)₂₋₃C(O)NRₐR_{b}, où Rₐ et R_{b} sont choisis indépendamment parmi H, un groupe alkyle en
C₁₋₃, -(CH₂)₂₋₃NH₂, -(CH₂)₂₋₃NH(CH₂)₂₋₃NH₂, ou un hétérocycle de 5 à 10 chaînons contenant 1 à 3 hétéroatomes choisis parmi N ou O, qui est formé conjointement par Rₐ, R_{b} et leur atome d'azote de liaison, de préférence un groupe morpholinyle, pipérazinyle ou pipéridinyle, ledit hétérocycle étant éventuellement substitué par les groupes suivants : un groupe alkyle en C₁₋₆ ou un groupe hydroxyle ; ou
d. R₁ est choisi parmi -R₁'-X, R₁' est -(CH₂)₁₋₆ -, et X est 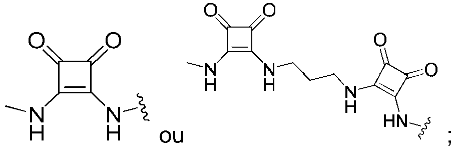 et/ou
où chaque n vaut 7, et m vaut 7 ;
ou un sel de celui-ci.

5. Composé selon l'une quelconque des revendications 1 à 3, dans lequel le composé répond à la formule (III), et dans lequel :
a. chaque R' est choisi indépendamment parmi un groupe alkyle en C₂₋₈; et/ou
b. chaque M est choisi indépendamment parmi -C(O)O- ou -OC(O)-, de préférence -C(O)O- ;
ou un sel de celui-ci.

6. Composé selon l'une quelconque des revendications 1 à 3, le composé répondant à la formule (IV) suivante, et dans lequel
a. chaque R* est choisi indépendamment parmi un groupe alkyle en C₆₋₁₀, de préférence un groupe alkyle en C₆; et/ou
b. chaque M est choisi indépendamment parmi -C(O)O- ou -OC(O)-, de préférence -C(O)O- ;
ou un sel de celui-ci.

7. Composé selon l'une quelconque des revendications 1 à 3, dans lequel le composé répond à la formule (V), et dans lequel :
a. chaque R* est choisi indépendamment parmi un groupe alkyle en C₆₋₁₀, de préférence un groupe alkyle en C₆; et/ou
b. chaque M est choisi indépendamment parmi -CH=CH-, -C(O)O- ou -OC(O), de préférence -CH=CH- ou -C(O)O- ; et/ou
c. chaque R' est un groupe alkyle en C₁₀ ou alcényle en C₈;
ou un sel de celui-ci.

8. Composé selon la revendication 1, ou un sel ou un isomère de celui-ci, dans lequel :
le composé est l'un des composés 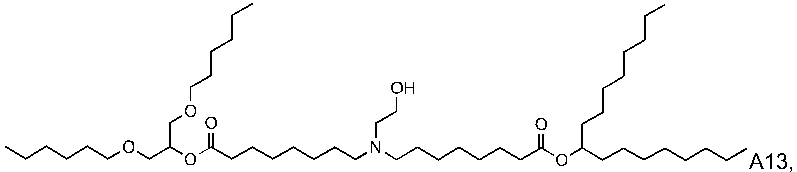 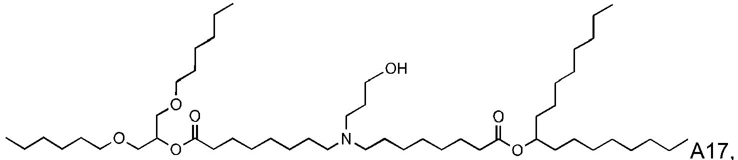 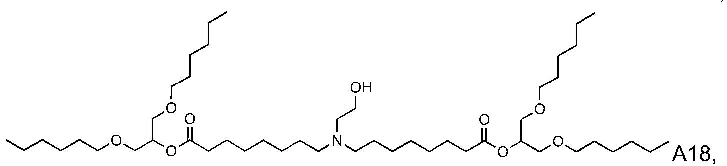 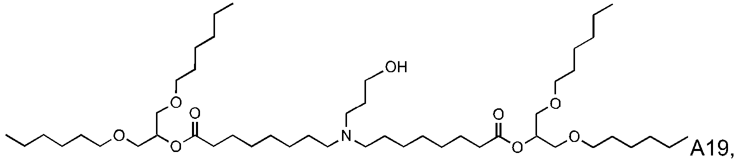 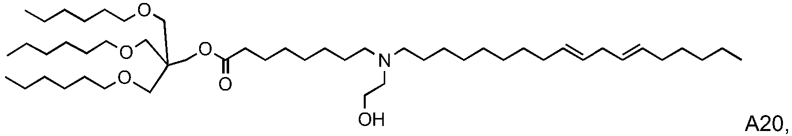 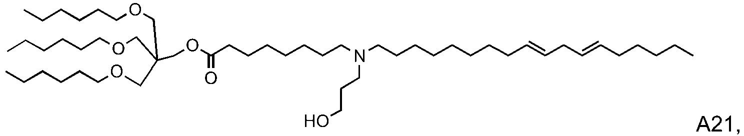 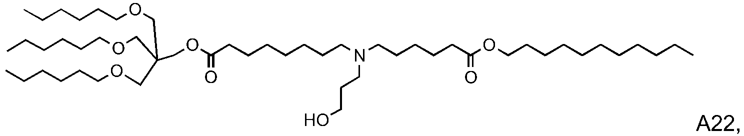 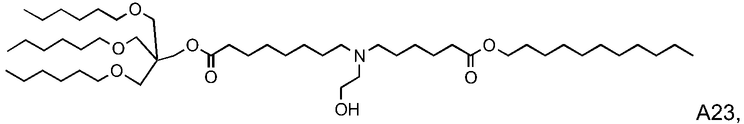 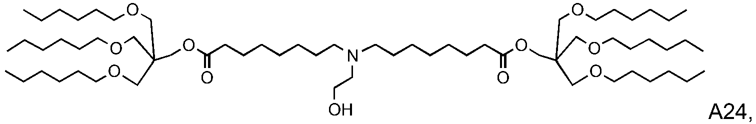 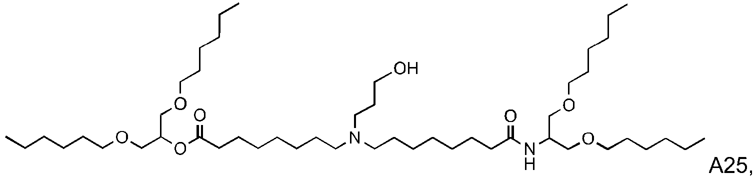 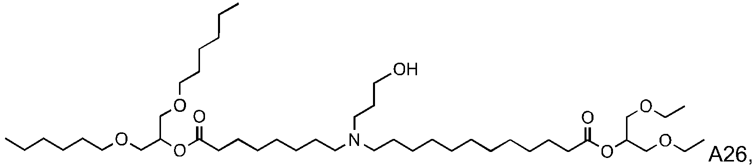 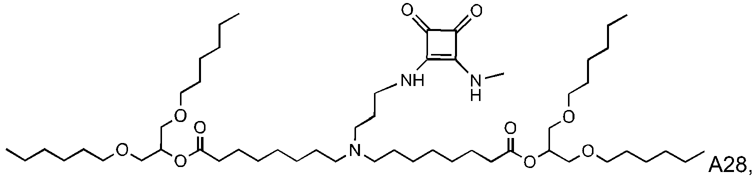 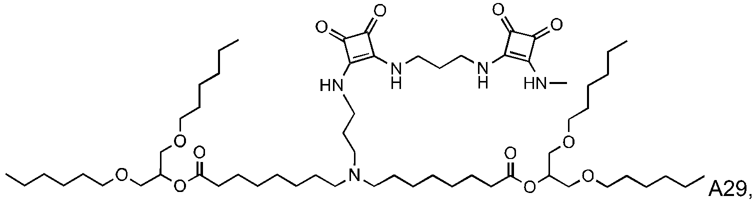 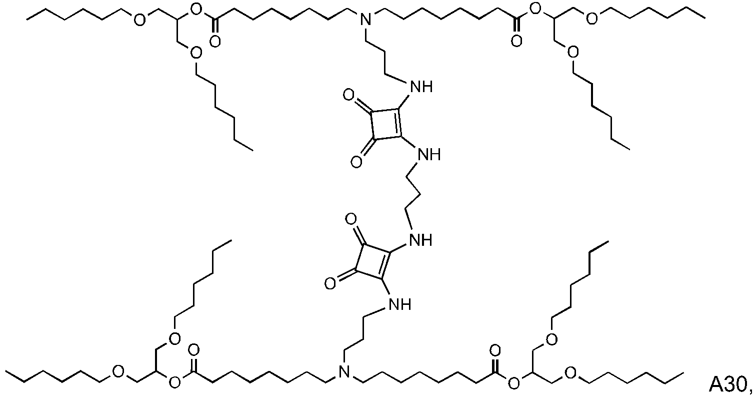 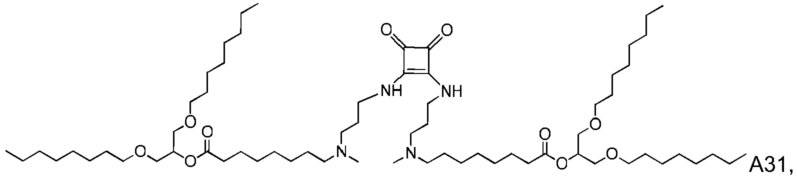 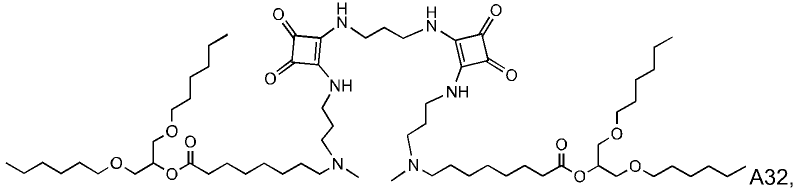 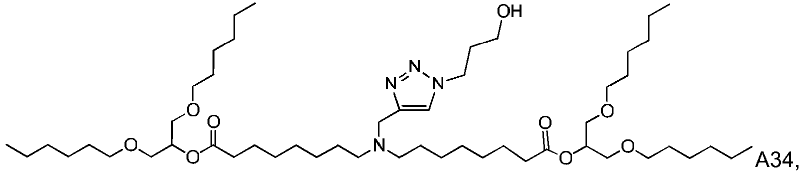 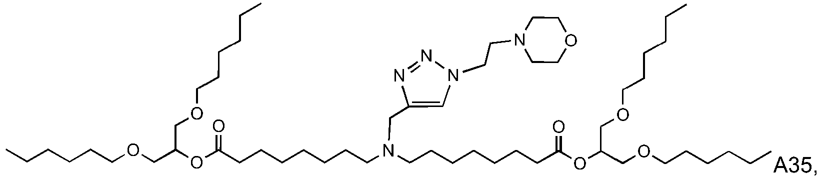 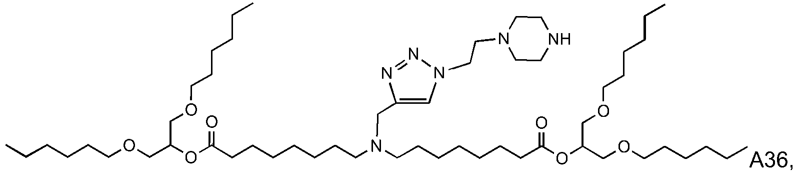 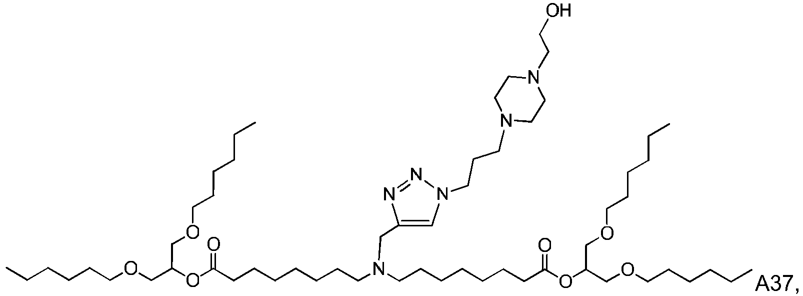 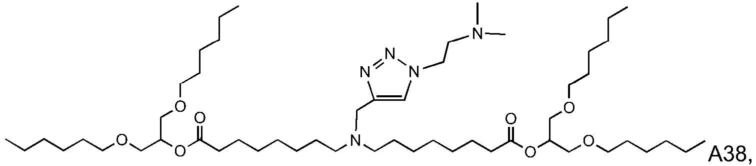 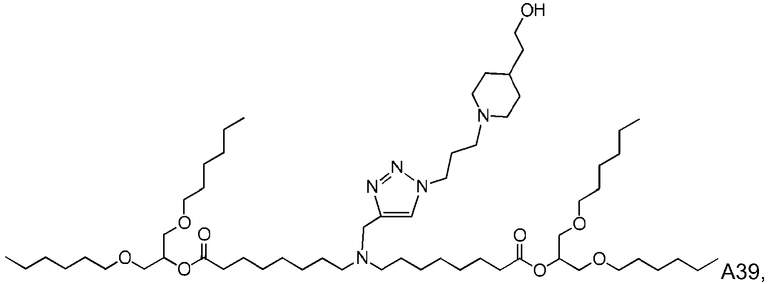 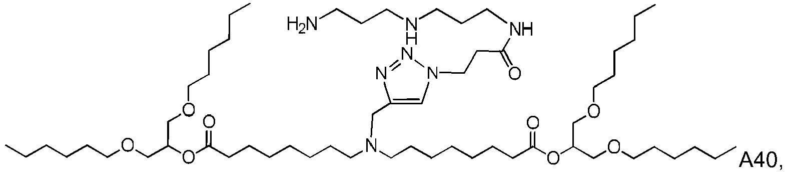 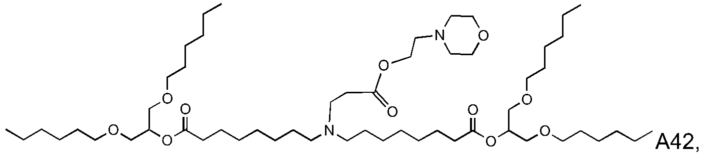 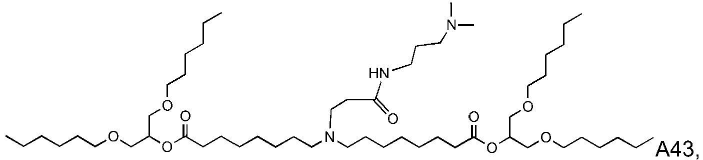 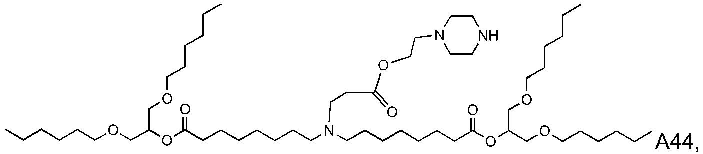 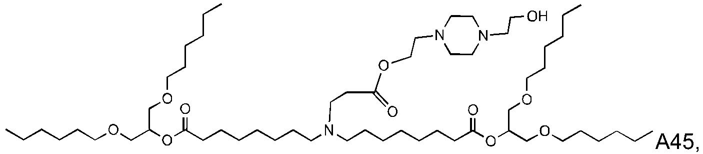 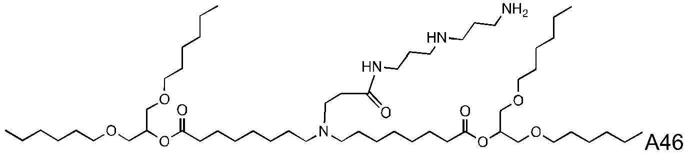 et

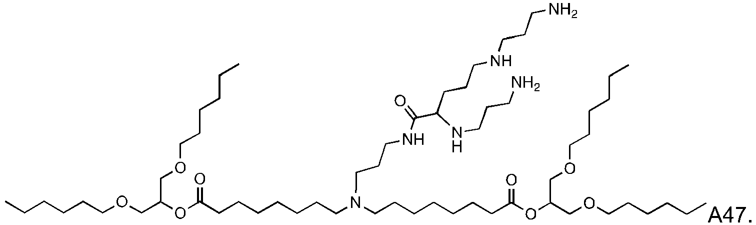

9. Composition comprenant un composé selon l'une quelconque des revendications 1 à 8 en tant que composé lipidique ionisable, de préférence
a. comprenant en outre un phospholipide,
de préférence en outre dans lequel :
le phospholipide est choisi parmi la 1,2-dilinoleoyl-sn-glycéro-3-phosphocholine (DLPC), la 1,2-dimyristoyl-sn-glycéro-3-phosphocholine (DMPC), la 1,2-dioleoyl-sn-glycéro-3-phosphocholine (DOPC), la 1,2-dipalmitoyl-sn-glycéro-3-phosphocholine (DPPC), la 1,2-distéaroyl-sn-glycéro-3-phosphocholine (DSPC), la 1,2-diundécanoyl-sn-glycéro-phosphocholine (DUPC), la I-palmitoyl-2-oléoyl-sn-glycéro-3-phosphocholine (POPC), la 1,2-di-O-octadécényl-5«-glycéro-3-phosphocholine (18:0 Diether PC), la 1-oléoyl-2-cholestéryle-hémisuccinoyl-5«-glycéro-3-phosphocholine (OChemsPC), la 1-hexadécyl-sn-glycéro-3-phosphocholine (C16 Lyso PC), la 1,2-dilinolénoïl-sn-glycéro-3 -phosphocholine, la 1,2-diarachidonoyl-sn-glycéro-3 -phosphocholine, la 1,2-didocosahexaénoyl-sn-glycéro-3-phosphocholine, la 1,2-dioleoyl-sn-glycéro-3-phosphoéthanolamine (DOPE), la 1,2-diphytanoyl-sn-glycéro-3-phosphoéthanolamine (ME 16,0 PE), la 1,2-distéaroyl-sn-glycéro-3-phosphoéthanolamine, I 1,2-dilinoleoyl-sn-glycéro-3-phosphoéthanolamine, la 1,2-dilinolénoïl-sn-glycéro-3-phosphoéthanolamine, la 1,2-diarachidonoyl-sn-glycéro-3 -phosphoéthanolamine, la 1,2-didocosahexaénoyl-sn-glycéro-3-phosphoéthanolamine, le sel de sodium de 1,2-dioleoyl-sn-glycéro-3-phospho-rac-(1-glycérol) (DOPG), le dipalmitoylphosphatidylglycérol (DPPG), la palmitoyloleoylphosphatidyléthanolamine (POPE), la distéaroyl-phosphatidyléthanolamine (DSPE), la dipalmitoyl phosphatidyléthanolamine (DPPE), la dimyristoylphosphoéthanolamine (DMPE), la 1-stéaroyl-2-oléoyl-phosphatidyléthanolamine (SOPE), la 1-stéaroyl-2-oléoyl-phosphatidylcholine (SOPC), la sphingomyéline, la phosphatidylcholine, la phosphatidyléthanolamine, la phosphatidylsérine, le phosphatidylinositol, l'acide phosphatidique, la palmitoyloleoyl phosphatidylcholine, la lysophosphatidylcholine, la lysophosphatidyléthanolamine (LPE), ou une combinaison de ceux-ci ; et/ou
b. comprenant en outre un lipide PEG,
de préférence en outre dans lequel :
le lipide PEG est choisi parmi la phosphatidyléthanolamine modifiée par du PEG, l'acide phosphatidique modifié par du PEG, la céramide modifiée par du PEG, la dialkylamine modifiée par du PEG, le diacylglycérol modifié par du PEG, le dialkylglycérol modifié par du PEG, ou une combinaison de ceux-ci ; et/ou
c. comprenant en outre un lipide structurel,
de préférence en outre en ce que :
le lipide structurel est choisi parmi : le cholestérol, le fécostérol, le sitostérol, l'ergostérol, le campestérol, le stigmastérol, le brassicastérol, la tomatidine, l'acide ursolique, l'alpha-tocophérol, ou une combinaison de ceux-ci; et/ou
d. comprenant en outre un ingrédient actif, qui est choisi parmi au moins l'un des suivants : ADN, ARN, protéine ou une molécule pharmaceutique active,
de préférence en outre :
i) dans lequel l'agent actif est un ARN et l'ARN est choisi parmi au moins l'un des suivants : ARNm, ARNsi, ARNai, ARNmi, ARNds, ARNa ou ARNInc ; ou
ii) dans lequel l'agent actif est une protéine choisie parmi au moins l'un des éléments suivants : anticorps, enzyme, protéine recombinante, polypeptide et polypeptide à chaîne courte.

10. Composition selon la revendication 9, dans laquelle :
la composition se présente sous la forme d'une nanoparticule lipidique.

11. Procédé de production d'une nanoparticule lipidique, comprenant :
le mélange d'un composé ionisable selon la revendication 1 avec un lipide PEG, un lipide structurel et un phospholipide pour former un mélange lipidique, de préférence
comprenant en outre :
le mélange d'un ingrédient actif avec le mélange lipidique pour former une nanoparticule lipidique à l'aide d'un mélangeur.

12. Composé selon l'une quelconque des revendications 1 à 8 , pour utilisation dans la production de nanoparticules lipidiques, de préférence dans lequel
ladite nanoparticule lipidique est neutre et non chargée dans un milieu neutre, et est chargée positivement après avoir été protonée dans un milieu acide ; et
ladite nanoparticule lipidique est telle que définie dans la revendication 9 .

13. Procédé selon la revendication11, dans lequel :
le composé est dissous et mélangé avec un lipide PEG, un lipide structurel et un phospholipide pour former un mélange lipidique, puis un principe actif est mélangé avec le mélange lipidique à l'aide d'un mélangeur pour former une nanoparticule lipidique.

14. Composition pharmaceutique comprenant la nanoparticule lipidique de la revendication 10, et un excipient pharmaceutiquement acceptable.

15. La composition de nanoparticules lipidiques selon la revendication 10, ou la composition pharmaceutique selon la revendication 14, pour utilisation comme médicament, de préférence
i) comprenant en outre un ingrédient actif, l'ingrédient actif étant choisi parmi au moins l'un des éléments suivants : ADN, ARN, protéine ou une molécule pharmaceutique active, de préférence
a. dans laquelle l'ingrédient actif est un ARN choisi parmi au moins l'un des suivants : ARNm, ARNsi, ARNai, ARNmi, ARNds, ARNa ou ARNInc, ou
b. dans laquelle l'ingrédient actif est une protéine choisie parmi au moins l'un des éléments suivants : anticorps, enzyme, protéine recombinante, polypeptide et polypeptide à chaîne courte ; et/ou
ii) dans laquelle ledit médicament peut être administré à un être humain par injection intraveineuse, injection intramusculaire, injection sous-cutanée, patch à micro-aiguilles, administration orale, spray oral et nasal, ou application topique.
